(19) **Europäisches Patentamt**
**European Patent Office**
**Office européen des brevets**

(11) **EP 2 238 156 B1**

(12) **EUROPEAN PATENT SPECIFICATION**

(45) Date of publication and mention
of the grant of the patent:
**01.10.2014 Bulletin 2014/40**

(51) Int Cl.:
***C07K 14/00*** *(2006.01)*  ***C07K 16/00*** *(2006.01)*

(21) Application number: **09705250.0**

(22) Date of filing: **29.01.2009**

(86) International application number:
**PCT/EP2009/000573**

(87) International publication number:
**WO 2009/095235 (06.08.2009 Gazette 2009/32)**

(54) **METHODS TO STABILIZE PROTEINS AND POLYPEPTIDES**

VERFAHREN ZUR STABILISIERUNG VON PROTEINEN UND POLYPEPTIDEN

PROCÉDÉS DE STABILISATION DE PROTÉINES ET DE POLYPEPTIDES

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **29.01.2008 US 62877
01.02.2008 US 63206**

(43) Date of publication of application:
**13.10.2010 Bulletin 2010/41**

(73) Proprietor: **Ablynx N.V.
9052 Ghent-Zwijnaarde (BE)**

(72) Inventors:
• **JONNIAUX, Jean-Luc
B-3300 Tienen (BE)**
• **LAUWEREYS, Marc, Jozef
B-9450 Haaltert (BE)**
• **STANSSENS, Patrick
B-9810 Nazareth (BE)**

(74) Representative: **HOFFMANN EITLE
Patent- und Rechtsanwälte
Arabellastrasse 4
81925 München (DE)**

(56) References cited:
**EP-A- 0 200 280**    **WO-A-99/01556**
**WO-A-2006/122825**

• **CACIA J ET AL: "Isomerization of an aspartic acid
residue in the complementarity-determining
regions of a recombinant antibody to human IgE:
Identification and effect on binding affinity"
BIOCHEMISTRY, AMERICAN CHEMICAL
SOCIETY, EASTON, PA.; US, vol. 35, no. 6, 13
February 1996 (1996-02-13), pages 1897-1903,
XP002094430 ISSN: 0006-2960**
• **LIU H ET AL: "Characterization of the stability of
a fully human monoclonal IgG after prolonged
incubation at elevated temperature" JOURNAL
OF CHROMATOGRAPHY B: BIOMEDICAL
SCIENCES & APPLICATIONS, ELSEVIER,
AMSTERDAM, NL, vol. 837, no. 1-2, 6 June 2006
(2006-06-06), pages 35-43, XP025122477 ISSN:
1570-0232 [retrieved on 2006-06-06]**
• **NIGEL JENKINS: "Modifications of therapeutic
proteins: challenges and prospects"
CYTOTECHNOLOGY, KLUWER ACADEMIC
PUBLISHERS, DO, vol. 53, no. 1-3, 25 May 2007
(2007-05-25), pages 121-125, XP019499837 ISSN:
1573-0778**

**Description**

Methods of modifying and in particular stabilizing proteins and polypeptides by which a predetermined amino acid is introduced into selected positions of said protein or polypeptide to produce a small group of mutants. The methods are based on the premise that certain amino acids play a crucial role in the stability of proteins or polypeptides. Generated mutants can then be further analysed for stability and/or function, e.g. affinity. Furthermore, appropriate mutants may be combined to result in further optimized proteins or polypeptides. In addition, stabilized example polypeptides and suitable methods to identify and/or analyse de-stabilized or stabilized proteins or polypeptides are provided. The methods can be used to study the role of specific amino acids in protein structure and function and to develop new or improved, e.g. stabilized proteins and polypeptides such as antibodies and single variable domains.

Randomized mutagenesis or other strategies for protein stabilizations is beset by several limitations. Among these are the large number of mutants that can be generated and the practical inability to select from these, the mutants that will be informative or have a desired property. For instance, there is no reliable way to predict whether the substitution, deletion or insertion of a particular amino acid in a protein or polypeptide will have a local or global effect on the protein/polypeptide, and therefore, whether it will be likely to yield useful information or function. Even if mutations are restricted to certain areas of a protein, such as regions at or around the active or binding site of a protein, the number of potential mutations can be extremely large, making it difficult or impossible to identify and evaluate those produced in a sensible manner. For example, substitution of a single amino acid position with all the other naturally occurring amino acids yields 19 different variants of a protein. If several positions are substituted at once, the number of variants increases exponentially. For substitution with all amino acids at 10 amino acid positions of a protein 19 x 19 x 19 x 19 x 19 x 19 x 19 x 19 x 19 x 19 or $6.1 \times 10^{12}$ variants of the protein are generated, from which useful mutants must be selected. It follows that for an effective use of mutagenesis in the stabilization of proteins, the type and number of mutations must be subjected to some restrictive criteria which keep the number of mutant proteins generated to a number suitable for analysing.

This invention pertains to a method of selection or mutagenesis for the generation of novel or stabilized proteins (or polypeptides) and to libraries of mutant proteins and specific mutant proteins generated by the method. The protein, peptide or polypeptide targeted for mutagenesis can be natural, synthetic or engineered proteins, peptides or polypeptides, e.g. antibodies, single variable domains such as Nanobodies or dAbs, a polypeptide comprising one or more single variable domains or a variant (e.g., a mutant). A protein library may be generated which contains mutant proteins having the predetermined amino acid in one or more positions of the predefmed amino acid position and, collectively, in every position. The method allows for a systematic evaluation of the role of a specific amino acid in the stabilization of a protein, e.g. an antibody or a single variable domain.

This invention identifies that some of the major variants of proteins, e.g. of antibodies, or single variable domains, generated during storage are the result of:

i.oxidation event(s) (if only one oxidation event +16Da variant), occurring in typically only the "accessible" methionines wherein oxidation increases during storage in parallel with incubation temperature and time,
ii.cyclization of the E1 residue, if present, resulting in formation of pyroglutamate, and
iii.isomerization or deamidation of aspartic acids or asparagines, e.g. in DG, DS, NG or NS motif wherein isomerization increases during storage in parallel with incubation temperature and time.

The invention makes use of this observation and provides a convenient method to focus on those possible "sources" of instability and use this information to select lead candidates or generate a library of mutant proteins, antibodies or single variable domains that can be generated by syrithesizing a single mixture of oligonucleotides which encodes all of the designed variations of the amino acid sequence for the region containing the predetermined amino acid. In an embodiment of the invention, this mixture of oligonucleotides is synthesized by incorporating in each condensation step of the synthesis both the nucleotide of the sequence to be mutagenized (for example, the wild type sequence) and the nucleotide required for the codon of the predetermined amino acid. Where a nucleotide of the sequence to be mutagenized is the same as a nucleotide for the predetermined amino acid, no additional nucleotide is added (see also e.g. WO9115581).

There are provided predefined amino acids to replace said identified "sources" of instability of proteins, polypeptides, antibodies, or single variable domains, e.g. by selective mutagenesis, and subsequent analysis of e.g. functional, e.g. binding property of said proteins, polypeptides, antibodies, or single variable domains.

This method of mutagenesis can be used to generate small groups of mutant proteins or libraries which are of a practical size for screening, e.g. for binding affinity. The method can be used to study the role of specific amino acids in protein stability and function and to develop new or stabilized proteins and polypeptides such as enzymes, antibodies, binding fragments or analogues thereof, single chain antibodies, single variable domains, Nanobodies®, dAbs, single domain antibodies and catalytic antibodies.

DETAILED DESCRIPTION OF THE INVENTION

The study of proteins has revealed that certain amino acids play a crucial role in their stability and function. For example, it appears that only a discrete number of amino acids participate in the catalytic event of an enzyme or the binding of an antibody.

Though it is clear that certain amino acids are particularly prone to destabilize, it is difficult, if not impossible, to predict with certainty which position (or positions) an amino acid must occupy to have such an effect. Unfortunately, the complex spatial configuration of amino acid side chains in proteins and the interrelationship of different side chains in the framework or CDR regions of e.g. antibodies are insufficiently understood to allow for such predictions. As pointed out above, randomized mutagenesis are of limited utility for the study of protein stability and function in view of the enormous number of possible variations in even small proteins, peptides or polypeptides.

The method of this invention provides a systematic and practical approach for evaluating the importance of particular amino acids, and their position within a defined region of a protein, to the stability and function of a protein and for producing useful, e.g. stabilized, proteins. The method begins with the assumption that a certain, predetermined amino acid is important to a particular stability of a protein, e.g. an antibody (see e.g. A A Wakankar et al., 2007, Biochemistry, 46, 1534-1544 for the study of isomerization of Aspartate in CDRs of antibodies).

With selection of the predetermined amino acid, a library of mutants of the protein to be studied is generated by incorporating the predetermined amino acid into each selected amino acid positions of the protein. As e.g. listed in Table B-2, different mutants are generated according to the proposed rules of the invention.

The skilled person understands it is a general aspect of all embodiments described herein, that in the selection of an appropriate amino acid to replace a selected residue, additional considerations can be applied, e.g. lowering the immunogenicity of the polypeptide, comprising Nanobodies or Dabs. For example in the context of a nanobody it may be advantageous to choose an amino acid for replacement of M (or any other residue or motif as discussed herein) which is present in the corresponding position of a human framework region. For additional reference the skilled person can also consider the teaching of WO 2009/004065 and/or WO 2009/004066.

The library of mutant proteins contains individual proteins which have the predetermined amino acid in each selected amino acid to be designed for replacement. The protein library will have a much higher probability of containing mutants that have improved stability and retain functional activity relative to a library of mutants that would be e.g. generated by completely random mutation or e.g. "walk through" mutation. Thus, the desired types of mutants are concentrated in the library. This is important because it allows faster and more detailed analysis of the generated mutants in an appropriate timeframe.

In another embodiment, a predetermined amino acid (e.g. one of the other naturally occurring 19 amino acids replacing the identified amino acid of possible instability) replaces an identified amino acid in a DG, DS, NG or NS motif of a protein wherein said motif is exposed to the solvent, e.g. such an identified amino acid motif may be found in a CDR of an antibody. Preferably the predetermined amino acid is selected from the group of Q, E, preferably E if the D or N is to be replaced or T or A if the S or G is to be replaced. In a further embodiment, a predetermined to be replaced amino acid, replaces an identified methionine, preferably said methionine is sensitive to forced oxidation, and said predetermined amino acid amino is selected from the group of amino acids consisting of A and T. In a further embodiment, a predetermined amino acid, D, replaces the terminal E if present. Additional considerations, based on structural information or modelling of the molecule mutagenized and/or the desired structure, can be further used to streamline or narrow down the subset of candidate positions for mutagenesis. Furthermore, an alanine screen through certain identified positions can quickly identify amino acids that are crucial for the functional properties of the protein which is to be stabilized. In a further embodiment, the learning from the different mutants can be combined and mutants with more than one mutagenized amino acid (compared to the original protein with which the method started with) can be generated.

The size of a library will vary depending upon the number of amino acids that are mutagenized. Preferably, the library will be designed to contain less than 50 mutants, and more preferably less than 40, more preferably less than 30, more preferably less than 20, more preferably less than 10 mutants.

In another approach, the gene of interest is present on a single stranded plasmid. For example, the gene can be cloned into an M13 phage vector or a vector with a filamentous phage origin of replication which allows propagation of single-stranded molecules with the use of a helper phage. The single-stranded template can be annealed with a set of degenerate probes. The probes can be elongated and ligated, thus incorporating each variant strand into a population of molecules which can be introduced into an appropriate host (Sayers, J. R. et al., Nucleic Acids Res. 16: 791-802 (1988)). This approach can circumvent multiple cloning steps where multiple domains are selected for mutagenesis.

Polymerase chain reaction (PCR) methodology can also be used to incorporate degenerate oligonucleotides into a gene. For example, the degenerate oligonucleotides themselves can be used as primers for extension.

In this embodiment, A and B are populations of degenerate oligonucleotides encoding the mutagenic cassettes or "windows", and the windows are complementary to each other (the zig-zag portion of the oligos represents the degenerate portion). A and B also contain wild type sequences complementary to the template on the 3' end for amplification and are thus primers for amplification capable of generating fragments incorporating a window. C and D are oligonucleotides which can amplify the entire gene or region of interest, including those with mutagenic windows incorporated (Steffan, N. H. et al., Gene 77: 51-59 (1989)). The extension products primed from A and B can hybridize through their complementary windows and provide a template for production of full-length molecules using C and D as primers. C and D can be designed to contain convenient sites for cloning. The amplified fragments can then be cloned.

Libraries of mutants generated by any of the above techniques or other suitable techniques can be screened to identify mutants of desired stability and activity. The screening can be done by any appropriate means. For example, binding affinity can be ascertained by suitable assays, e.g. BiaCore measurements, standard immunoassay and/or affinity chromatography.

The method of this invention can be used to stabilize proteins that were identified according to the invention to have possible sources of instability. The description heretofore has centered around proteins, but it should be understood that the method applies to, polypeptides comprising single variable domains such as Nanobodies and dAbs and multi-subunit proteins as well. The amino acids to be proposed to be mutagenized in the wild type protein by the method of this invention can be more than one and preferably do not influence other properties of the wild type protein.

Usually, the region studied will be a functional domain of the protein such as a binding or catalytic domain. For example, the region can be the hypervariable region (complementarity-determining region or CDR) of an immunoglobulin, the catalytic site of an enzyme, or a binding domain.

Importantly, several different amino acids of a protein can be mutagenized simultaneously or sequentially. The same or a different amino acid can be "walked-through" in each identified amino acid position of possible source of instability and checked for their retained or not retained function, e.g. binding property.

The method of this invention opens up new possibilities for the design of different types of stabilized proteins. The new structures can be built on the natural "scaffold" of an existing protein by mutating only relevant amino acids by the method of this invention.

The method of this invention is useful for modifying single variable domains such as Nanobodies and dAbs. Alterations as proposed by the method of the invention can be introduced into the variable region and/or into the framework (constant) region of the antibody. Modification of the variable region can produce antibodies with better stability properties but also with better antigen binding properties, and catalytic properties.

The method of this invention is particularly suited to the design of stabilized polypeptides comprising single variable domains and new uses of said polypeptides comprising stabilized single variable domains such as a Nanobody®, a domain antibody, a single domain antibody, a "dAb" or formatted version thereof, e.g. polypeptides comprising Nanobodies and/or dAbs having multivalent or multimeric binding properties as diagnostics and/or therapeutics.

The use of polypeptides comprising single variable domains as diagnostics and therapeutics is a rapidly expanding field and research concerning said polypeptides comprising single variable domains is looking among others into the extension of half-life in vivo and the possibility to obtain a regulatory approvable long term storage property at elevated temperature, e.g. room temperature or higher, of the drug.

Natural single variable domains such as e.g. Nanobodies derived from Llamas, or genetically engineered camelized dAbs, are not optimized for long term stability in storage and/or for long term efficacious action in vivo and thus at least some of them may, although considered to be generally more stable than conventional antibodies, may still be destabilized, i.e. may not be stable enough to accommodate for the required regulatory storage time at certain temperature or for the extended half life in vivo at body temperature.

Thus there is a need in the art to identify possible sources of instability in polypeptides comprising such single variable domains and to find methods to modify, e.g. stabilize or destabilize, said polypeptides. This invention in particular focuses on methods to modify, e.g. stabilize or destabilize, said polypeptides comprising single variable domains by specific mutations of the amino acid sequence.

Analogous to the general principle as discussed above, the method of the invention provides one or more of the following main strategies to achieve a modified, e.g. improved or decreased, stability profile for the polypeptides comprising at least one single variable domain: a) avoid isomerization of Asp (D) and Asn (N), i.e. inspect sequences for the presence of Asp (D) and Asn (N), in particular for Asp-Gly (DG), Asp-Ser (DS), Asn-Gly (NG) and Asn-Ser (NS) in the CDRs of the single variable domain and replace Asp and/or Asn with Glu (E) or Gln (Q) so that at least one bioactivity, e.g. binding affinity, is preserved, ; b) avoid oxidation of Met, e.g. check for Met which are susceptible to oxidation, in particular forced oxidation, and if not resistant to oxidation or forced oxidation replace Met with Ala or Thr other so that at least one bioactivity, e.g. binding affinity, is preserved, , and c) avoid or replace N-terminal Glu by an alternative N-terminus, Asp.

In another embodiment of the invention, there is provided a method for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domain of the polypeptides is inspected for nucleotide sequences coding for NG, NS, DG or DS in the CDR loops, preferably in the CDR2 and/or CDR3 loops, more preferably CDR3; and

b) if at least one nucleotide sequence coding for said di-peptide sequence is present, generate a library of mutants comprising (or essentially consisting of) polypeptide derivatives wherein one or more of said identified nucleotide sequences in a) is replaced with nucleotide sequences coding for EG, QG, ES, QS, NA, NT, DA or DT, preferably EG or QG in case NG or DG is found in the polypeptides to be stabilized or ES or QS in case NS or DS is found in the polypeptides to be stabilized; and

c) the suitable, e.g. prokaryotic or eukaryotic, organism is transformed with the gene modified in this manner and the polypeptides, the fragment or derivative with the desired activity is expressed.

In another embodiment of the invention, there is provided a method for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domain of the polypeptides is inspected for nucleotide sequences coding for a NG, NS, DG or DS motif wherein said amino acid or motif is surface exposed and wherein H-bond donating residues are in close proximity to the labile N or D; and

b) if nucleotide sequences in a) are identified, generate a library of mutants comprising (or essentially consisting of) polypeptide derivatives wherein one or more of said identified nucleotide sequences in a) is replaced with nucleotide sequences coding for EG, QG, ES, QS, NA, NT, DA or DT, preferably EG or QG in case NG or DG is found in the polypeptides to be stabilized or ES or QS in case NS or DS is found in the polypeptides to be stabilized; and

c) the suitable, e.g. prokaryotic or eukaryotic, organism is transformed with the gene modified in this manner and the polypeptides, the fragments or derivatives with the desired activity is expressed.

In another embodiment of the invention, there is provided a method/process for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domains of the polypeptides is inspected for nucleotide sequences coding for a NG, NS, DG or DS in the CDR loops, preferably in the CDR2 and/or CDR3 loops, more preferably CDR3; and

b) check whether isomerization of the identified sequences is taking place (e.g. by pro-longed storage at elevated temperature and subsequent observation of a pre-peak in the RPC profile - see experimental part) and optionally is responsible for the loss of at least one activity of said polypeptides, preferably all activities; and

c) whenever isomerization is observed, generate a library of mutants comprising (or essentially consisting of) polypeptide derivatives wherein one or more of said identified nucleotide sequences in a) is replaced with nucleotide sequences coding for EG, QG, ES, QS, NA, NT, DA or DT, preferably EG or QG in case NG or DG is found in the polypeptides to be stabilized or ES or QS in case NS or DS is found in the polypeptides to be stabilized; and

d) the prokaryotic or eukaryotic organism is transformed with the gene modified in this manner and the polypeptide, the fragment or derivative with the desired activity is expressed.

In another embodiment of the invention, there is provided a method/process for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domain of the polypeptides is inspected for nucleotide sequences coding for NG or DG in the CDR loops, preferably in the CDR2 and/or CDR3 loops, more preferably CDR3; and

b) check whether isomerization of the identified sequences is taking place (e.g. by pro-longed storage at elevated temperature and subsequent observation of a pre-peak in the RPC profile - see experimental part) and optionally is responsible for the loss of at least one activity of said polypeptides, preferably all activities; and

c) whenever isomerization is observed, generate a library of mutants comprising (or essentially consisting of) polypeptide derivatives wherein one or more of said identified nucleotide sequences in a) is replaced with nucleotide sequences coding for EG, QG, NA, NT, DA or DT, preferably EG or QG in case NG or DG is found in the polypeptides to be stabilized; and

d) the prokaryotic or eukaryotic organism is transformed with the gene modified in this manner and the polypeptide, the fragment or derivative with the desired activity is expressed.

In another embodiment of the invention, there is provided a method/process for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domains of the polypeptides is inspected for nucleotide sequences coding for NS or DS in the CDR loops, preferably in the CDR2 and/or CDR3 loops, more preferably CDR3; and

b) check whether isomerization of the identified sequences is taking place (e.g. by pro-longed storage at elevated temperature and subsequent observation of a pre-peak in the RPC profile - see experimental part) and optionally is responsible for the loss of at least one activity of said polypeptides, preferably all activities; and

c) whenever isomerization is observed, generate a library of mutants comprising (or essentially consisting of) polypeptide derivatives wherein one or more of said identified nucleotide sequences in a) is replaced with nucleotide sequences coding for ES, QS, NA, NT, DA or DT, preferably ES or QS in case NS or DS is found in the polypeptides to be stabilized; and

d) the prokaryotic or eukaryotic organism is transformed with the gene modified in this manner and the polypeptide, the fragment or derivative with the desired activity is expressed.

In another embodiment of the invention, there is provided a method/process for the production of polypeptides comprising at least one single variable domain, e.g. Nanobodies or dAbs, preferably Nanobodies, functional derivatives or fragments thereof with an improved stability, in a suitable, e.g. eukaryotic or prokaryotic, organism by transformation with an expression vector which contains a recombinant gene which codes for said polypeptides, derivative or fragment comprising the steps:

a) the gene coding for at least one of the variable domains of the polypeptides is inspected for nucleotide sequences coding for NG, DG, NS or DS in the CDR loops, preferably in the CDR2 and/or CDR3 loops, more preferably CDR3; and

b) generate a library of mutants comprising (or essentially consisting of) polypeptide derivatives wherein one or more of said identified nucleotide sequences in a) is replaced with nucleotide sequences coding for EG, QG, ES, QS, NA, NT, DA or DT, preferably ES or QS in case NS or DS is found in the polypeptides to be stabilized or preferably EG or QG in case NG or DG is found in the polypeptides to be stabilized; and

c) the prokaryotic or eukaryotic organism is transformed with the gene modified in this manner and the polypeptide, the fragment or derivative with the desired activity is expressed.

In a preferred embodiment of the invention the method is carried out for polypeptides comprising at least one single variable domain, e.g. Nanobodies or dAbs, preferably Nanobodies, functional derivatives or fragments thereof with an improved stability, wherein at least one codon for an D or N is replaced in the gene of the single variable domain of the polypeptide, in particular if this D or N is followed by a S or G and/or said DG, NG, DS or NS is within the CDR, preferably CDR2 or CDR3, more preferably CDR3, of the single variable domain of the polypeptide, and the prokaryotic or eukaryotic organism is transformed and the polypeptide, the fragment or derivative thereof with the desired activity is expressed.

The method is carried out for polypeptides comprising at least one single variable domain, e.g. Nanobodies or dAbs, preferably Nanobodies, functional derivatives or fragments thereof with an improved stability, wherein at least one codon for a M is replaced with A or T in the gene of the single variable domain of the polypeptide, in particular if this M is within the CDR, preferably CDR2 or CDR3, more preferably CDR3, or M is at position 77 (using KABAT numbering), of the single variable domain of the polypeptide, and the prokaryotic or eukaryotic organism is transformed and the polypeptide, the fragment or derivative thereof with the desired activity is expressed.

The method is carried out for polypeptides comprising at least one single variable domain, e.g. Nanobodies or dAbs, preferably Nanobodies, functional derivatives or fragments thereof with an improved stability, wherein E1 (Kabat numbering for Nanobody), is replaced, by D, and the prokaryotic or eukaryotic organism is transformed and the polypeptide, the fragment or derivative thereof with the desired activity is expressed.

The method or process according to the invention is used in such a manner that the polypeptides comprising at least a single variable domain (that is intended to be stabilized) is sequenced and the sequence of its domains is compared with the consensus sequences stated in the sequences of Kabat et al. (1991, below) or is continuously numbered. The amino acid positions are defined at a maximum homology or identity of the sequences. Subsequently one or several codons can be modified according to the invention, advantageously by mutagenesis. It turns out that the specific substitution of one codon can already lead to a considerable change in the stability of an antibody. However, two, three or more codons are preferably modified. An upper limit for the number of substitutions is reached when other properties of the antibody which are important for the desired application purpose (e.g. affinity, protease stability, selectivity) are adversely affected.

It is intended to elucidate the procedure on the basis of an example: The amino acid positions are firstly determined by a sequence comparison (maximum homology) with the tables of Kabat (1991, below).

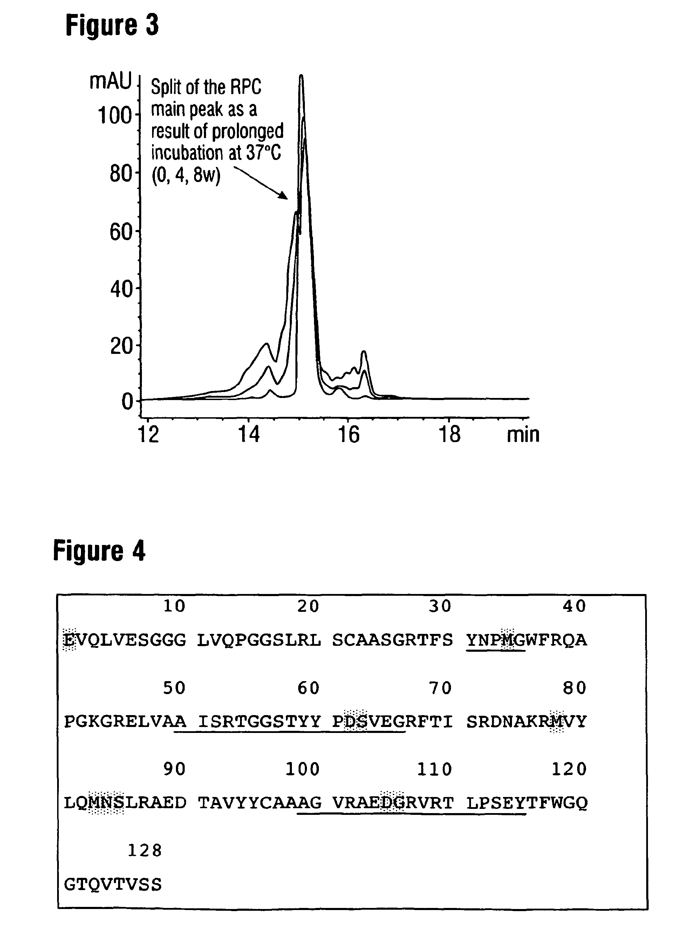

In the case of SEQ ID NO: 2, it is found that the amino acid D105 (consecutive numbering, see figures, e.g. Figure 4) undergoes isomerization and that this isomerization is the predominant molecular mechanism underlying loss of potency during long term storage at elevated temperature (see experimental part). However, replacement of D105 by E105, i.e. a D105E mutant of SEQ ID NO: 2 prevents mentioned loss of affinity over time at elevated temperature and has when formatted in a bivalent form, a comparable overall affinity to the wild type polypeptide with SEQ ID NO: 2 (see also experimental part). Hence, by using the method according to the invention, it is possible to obtain a stabilized version without loss of binding affinity of SEQ ID NO: 2, namely the a preferred mutant of SEQ ID NO: 2, i.e. the D105E mutant of SEQ ID NO: 2. Other examples are disclosed in the experimental part.

In another embodiment of the invention, there is provided a method/process for the production of functional polypeptides, functional derivatives or fragments thereof, e.g. polypeptides comprising Nanobodies or dAbs, preferably Nanobodies, in a suitable, e.g. eukaryotic or prokaryotic, organism by transformation with an expression vector which contains a recombinant gene which codes for said library of polypeptides, derivatives or fragments comprising any of the process steps as disclosed above and in addition

a) check whether any M is present and optionally check whether M is susceptible to forced oxidation, and if so generate further members in the library by replacing M by A or T, more preferably A; and
b) transform suitable organism with the gene modified in this manner and the polypeptide of the invention, the fragment or derivative with the desired activity is expressed.

The skilled person will understand that in the selection of an appropriate amino acid to replace M additional considerations can be applied, e.g. lowering the immunogenicity of the polypeptide, comprising Nanobodies or Dabs. For example in the context of a nanobody it may be advantageous to choose an amino acid for replacement of M which is present in the corresponding position of a human framework region (see experimental part, e.g. the M78T mutation of example 4).

In another embodiment of the invention, there is provided a method/process for the production of Nanobodies or dAbs, preferably Nanobodies, in a suitable, e.g. eukaryotic or prokaryotic, organism by transformation with an expression vector which contains a recombinant gene which codes for said library of Nanobodies comprising any of the process steps as disclosed above and in addition

a) check whether M is present at position 77 (using Kabat numbering) and optionally check whether M is susceptible to forced oxidation and if so generate further members in the library by replacing M by A or T, more preferably A; and
b) transform suitable organism with the gene modified in this manner and the Polypeptide of the Invention, the fragment or derivative with the desired activity is expressed.

In another embodiment of the invention, there is provided a method/process for the production of functional polypeptides, functional derivatives or fragments thereof, e.g. Nanobodies or dAbs, preferably Nanobodies, in a suitable, e.g. eukaryotic or prokaryotic, organism by transformation with an expression vector which contains a recombinant gene which codes for said polypeptides, derivative or fragment comprising any of the process steps as disclosed above and in addition comprises

a) check whether any M is present and optionally check whether any of the M is susceptible to forced oxidation, and if so replace at least one M by A or T, more preferably A; and
b) replace N-terminal E with D; and
c) transform eukaryotic organism with the gene modified in this manner and the polypeptides, the fragments or derivatives with the desired activity are expressed.

In order to stabilize a protein or polypeptide by the process according to the invention and to nevertheless preserve its other properties such as especially affinity for the antigen, amino acids are preferably substituted which as far as possible do not impair these properties. For this reason it is preferable to replace identified amino acids by conservative substitutions.

The polypeptides, derivatives and fragments thereof according to the invention can be produced according to methods for the production of recombinant proteins familiar to a person skilled in the art.

In order to produce the polypeptides modified according to the invention it is for example possible to synthesize the complete DNA of the variable domain (by means of oligonucleotide synthesis as described for example in Sinha et al., NAR 12 (1984), 4539-4557). The oligonucleotides can be coupled by PCR as described for example by Innis, Ed. PCR protocols, Academic Press (1990) and Better et al., J. Biol. Chem. 267 (1992), 16712-16118. The cloning and expression is carried out by standard methods as described for example in Ausubel et al, Eds. Current protocols in Molecular Biology, John Wiley and Sons, New York (1989) and in Robinson et al., Hum. Antibod. Hybridomas 2 (1991) 84-93. The specific antigen binding activity can for example be examined by a competition test as described in Harlow et al., Eds. Antibodies; A Laboratory Manual, Chapter 14, Cold Spring Harbor Laboratory, Cold Spring Harbor (1988) and Munson et al., Anal. Biochem. 407 (1980), 220-239.

Suitable host organisms are for example CHO cells, lymphocyte cell lines which produce no immunoglobulins, yeast, insect cells and prokaryotes such as E. coli.

A further subject matter of the invention is such a process in which the protein is isolated in a prokaryotic organism (e.g. E. coli) as denatured inclusion bodies and is activated by processes familiar to a person skilled in the art (cf. e.g. EP-A 0 364 926).

A further subject matter of the invention is a process in which the polypeptides of the invention are stabilized according to the invention in such a way that it is biologically actively formed in the cytosol with the desired activity and can be isolated directly from this and in an active form.

The methods/processes according to the invention improve the stability of polypeptides and proteins for all the aforementioned areas of application. Moreover new stable polypeptide variants can be produced according to the invention that were previously not obtainable in a stable enough form such as polypeptides which are suitable for use under un-physiological conditions.

Definitions

a) By the term "Target Molecule" or "Target Molecules" or "target" is meant a protein with a biological function in an organism including bacteria and virus, preferably animal, more preferably mammal most preferred human, wherein said biological function may be involved in the initiation or progression or maintenance of a disease. Preferably said protein is selected from the group consisting of: human growth hormone(hGH), N-methionyl human growth hormone, bovine growth hormone, parathyroid hormone, thyroxine, insulin A-chain, insulin B-chain, proinsulin, relaxin A-chain, relaxin B-chain, prorelaxin, glycoprotein hormones such as follicle stimulating hormones(FSH), thyroid stimulating hormone(TSH), and leutinizing hormone(LH), glycoprotein hormone receptors, calcitonin, glucagon, factor VIII, an antibody, a Nanobody, a molecule which is well tolerated by mammals in particularly humans and has a long half life when given systemically and/or locally, e.g. poly glycol chains of different size, e.g. PEG-20, PEG-30 or PEG40, lung surfactant, urokinase, streptokinase, human tissue-type plasminogen activator (t-PA), bombesin, factor IX, thrombin, hematopoietic growth factor, tumor necrosis factor (TNF)-alpha and TNF-beta, enkephalinase, human serum albumin, mullerian-inhibiting substance, mouse gonadotropin-associated peptide, a microbial protein, such as beta lactamase, tissue factor protein, inhibin, activin, vascular endothelial growth factor, receptors for hormones or growth factors; integrin, thrombopoietin, protein A or D, rheumatoid factors, nerve growth factors such as NGF-β, platelet-growth factor, transforming growth factors (TGF) such as TGF-alpha and TGF-beta, insulin-like growth factor-I and -II, insulin-like growth factor binding proteins, CD-4, DNase, latency associated peptide, erythropoietin, osteoinductive factors, interferons such as interferon-alpha, -beta, and -gamma, colony stimulating factors (CSFs) such as M-CSF, GF-CSF, and G-CSF, interleukins (ILs) such as IL-1, IL-2, IL-3, IL-4, IL-5, IL-6, von Willebrand factor, superoxide dismutase; decay accelerating factor, viral antigen, HIV envelope proteins such as GP120, GP140, atrial natriuretic peptides A, B or C, immunoglobulins, and fragments or variants of any of the above-listed proteins. Additionally, said protein can be a receptor for the above-mentioned factors/cytokines and/or a complex of the receptor and factor/cytokine. More preferably, said Target Molecule is a multimeric protein and even more preferred is a multimeric protein which subunits are selected from the group consisting of: von Willebrand Factor (vWF), IL-6, tumor necrosis factor-alpha and -beta and many others. A multimeric protein is a protein which is associated (typically by noncovalent interactions) in biological organism such as humans with others as subunits in a multimeric structure and typically only in the multimeric format is able to unfold its biological function. This is also called the quaternary structure of the protein. This association can also be stabilized by disulfide bonds and by noncovalent interactions with reacting substrates or cofactors.

b) The single variable domains that are present in the constructs of the invention may be any variable domain that forms a single antigen binding unit. Generally, such single variable domains will be amino acid sequences that essentially consist of 4 framework regions (FR1 to FR4 respectively) and 3 complementarity determining regions (CDR1 to CDR3 respectively); or any suitable fragment of such an amino acid sequence (which will then usually contain at least some of the amino acid residues that form at least one of the CDR's, as further described herein). Such single variable domains and fragments are most preferably such that they comprise an immunoglobulin fold or are capable for forming, under suitable conditions, an immunoglobulin fold. As such, the single variable domain may for example comprise a light chain variable domain sequence (e.g. a $V_L$-sequence) or a suitable fragment thereof; or a heavy chain variable domain sequence (e.g. a $V_H$-sequence or $V_{HH}$ sequence) or a suitable fragment thereof; as long as it is capable of forming a single antigen binding unit (i.e. a functional antigen binding unit that essentially consists of the single variable domain, such that the single antigen binding domain does not need to interact with another variable domain to form a functional antigen binding unit, as is for example the case for the variable domains that are present in for example conventional antibodies and ScFv fragments that need to interact with another variable domain - e.g. through a $V_H/N_L$ interaction - to form a functional antigen binding domain).

For example, the single variable domain may be a domain antibody (or an amino acid sequence that is suitable for use as a domain antibody), a single domain antibody (or an amino acid sequence that is suitable for use as a single domain antibody), a "dAb" or dAb (or an amino acid sequence that is suitable for use as a dAb) or a Nanobody™ (as defined herein, and including but not limited to a $V_{HH}$ sequence); other single variable domains, or any suitable fragment of any one thereof. For a general description of (single) domain antibodies, reference is also made to the prior art cited above, as well as to EP 0 368 684. For the term "dAb's", reference is for example made to Ward et al. (Nature 1989 Oct 12; 341 (6242): 544-6), to Holt et al., Trends Biotechnol., 2003, 21(11):484-490; as well as to for example WO 04/068820, WO 06/030220, WO 06/003388 and other published patent applications of Domantis Ltd. It should also be noted that, although less preferred in the context of the present invention because they are not of mammalian origin, single domain antibodies or single variable domains can be derived from certain species of shark (for example, the so-called "IgNAR domains", see for example WO 05/18629).

In particular, the amino acid sequence of the invention may be a Nanobody™ or a suitable fragment thereof *[Note: Nanobody™, Nanobodies™ and Nanoclone™ are trademarks of Ablynx N. V.]* For a further description of $V_{HH}$'s and Nanobodies, reference is made to the review article by Muyldermans in Reviews in Molecular Biotechnology 74(2001), 277-302; as well as to the following patent applications, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134231 and WO 02/48193 of Unilever; WO 97/49805, WO 01/21817, WO 03/035694, WO 03/054016 and WO 03/055527 of the Vlaams Instituut voor Biotechnologie (VIB); WO 03/050531 of Algonomics N.V. and Ablynx N.V.; WO 01/90190 by the National Research Council of Canada; WO 03/025020 (= EP 1 433 793) by the Institute of Antibodies; as well as WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V. and the further published patent applications by Ablynx N.V. Reference is also made to the further prior art mentioned in these applications, and in particular to the list of references mentioned on pages 41-43 of the International application WO 06/040153. As described in these references, Nanobodies (in particular $V_{HH}$ sequences and partially humanized Nanobodies) can in particular be characterized by the presence of one or more *"Hallmark residues"* in one or more of the framework sequences.

A further description of the Nanobodies, including humanization and/or camelization of Nanobodies, as well as other modifications, parts or fragments, derivatives or "Nanobody fusions", multivalent constructs (including some non-limiting examples of linker sequences) and different modifications to increase the half-life of the Nanobodies and their preparations can be found e.g. in WO07/104529.

c) By "high affinity" as used herein is meant a dissociation constant for a monovalent binding Nanobody of (Kd) of < 100 nM and preferably 10 nM and more preferably 1nM and even more preferably 100pM and most preferred 10 pM under physiological conditions and measured by standard procedures in the art.

d) By "high avidity" as used herein is meant a dissociation constant for a bi- or multivalent binding Nanobody of (Kd) of < 100 nM and preferably 10 nM and more preferably 1nM and even more preferably 100pM and most preferred 10 pM under physiological conditions and measured by standard procedures in the art.

e) By "rigid secondary structure" as used herein is meant any polypeptide segment exhibiting a regular repeated structure such as is found in; $\alpha$-helices, 310 helices, $\pi$-helices, parallel and antiparallel $\beta$-sheets, and reverse turns. Certain "non-ordered" structures that lack recognizable geometric order are also included in the definition of rigid secondary structure provided they form a domain or "patch" of amino acid residues capable of interaction with a target and that the overall shape of the structure is not destroyed by replacement of an amino acid within the structure. It is believed that some non-ordered structures are combinations of reverse turns. The geometry of these rigid secondary structures is well defined by $\varphi$ and .psi. torsional angles about the $\alpha$-carbons of the peptide "backbone". The requirement that the secondary structure be exposed to the surface of the polypeptide is to provide a domain or "patch" of amino acid residues that can be exposed to and bind with a target molecule. It is primarily these amino acid residues that are replaced by mutagenesis that form the "library" of structurally related (mutant) binding polypeptides that are displayed on the surface of the phage and from which novel polypeptide ligands are selected. Mutagenesis or replacement of amino acid residues directed toward the interior of the polypeptide is generally avoided so that the overall structure of the rigid secondary structure is preserved. Some replacement of amino acids on the interior region of the rigid secondary structures, especially with hydrophobic amino acid residues, may be tolerated since these conservative substitutions are unlikely to distort the overall structure of the polypeptide.

f) By "leader sequence" as used herein is meant a particular section of messenger RNA (mRNA) and the DNA that codes for it. It starts at the +1 position (where transcription begins) and ends just before the start codon (usually AUG) of the coding region. It usually contains a ribosome binding site (RBS), in bacteria also known as the Shine-Delgarno sequence (AGGAGGU). The 5' UTR may be a hundred or more nucleotides long, and the 3' UTR may be even longer (up to several kilobases in length) (Molecular Cell Biology, 5th edition, Lodish et al. p113, chapter 4.2). Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology" (6th Ed.), Garland Science Publishing/Churchill Livingstone, New York (2005), as well as to the general background art cited herein;

g) Unless indicated otherwise, the term "immunoglobulin sequence" - whether used herein to refer to a heavy chain antibody or to a conventional 4-chain antibody - is used as a general term to include both the full-size antibody, the individual chains thereof, as well as all parts, domains or fragments thereof (including but not limited to antigen-binding domains or fragments such as $V_{HH}$ domains or $V_H$/$V_L$ domains, respectively). In addition, the term "sequence" as used herein (for example in terms like "immunoglobulin sequence", "antibody sequence", "variable domain sequence", "$V_{HH}$ sequence" or "protein sequence"), should generally be understood to include both the relevant amino acid sequence as well as nucleic acids or nucleotide sequences encoding the same, unless the context requires a more limited interpretation. Also, the term "nucleotide sequence" as used herein also encompasses a nucleic acid molecule with said nucleotide sequence, so that the terms "nucleotide sequence" and "nucleic acid" should be considered equivalent and are used interchangeably herein;

h) Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein; as well as to for example the following reviews Presta, Adv. Drug Deliv. Rev. 2006, 58 (5-6): 640-56; Levin and Weiss, Mol. Biosyst. 2006, 2(1): 49-57; Irving et al., J. Immunol. Methods, 2001, 248(1-2), 31-45; Schmitz et al., Placenta, 2000, 21 Suppl. A, S106-12, Gonzales et al., Tumour Biol., 2005, 26(1), 31-43, which describe techniques for protein engineering, such as affinity maturation and other techniques for improving the specificity and other desired properties of proteins such as immunoglobulins.

i) Amino acid residues will be indicated according to the standard three-letter or one-letter amino acid code, as mentioned in Table A-2;

**Table A-2: one-letter and three-letter amino acid code**

| Nonpolar, uncharged (at pH 6.0 - 7.0)[3] | Alanine | Ala | A |
|---|---|---|---|
| | Valine | Val | V |
| | Leucine | Leu | L |
| | Isoleucine | Ile | I |
| | Phenylalanine | Phe | F |
| | Methionine[1] | Met | M |
| | Tryptophan | Trp | W |
| | Proline | Pro | P |
| Polar, | Glycine[2] | Gly | G |

(continued)

| uncharged (at pH 6.0-7.0) | Serine | Ser | S |
|---|---|---|---|
| | Threonine | Thr | T |
| | Cysteine | Cys | C |
| | Asparagine | Asn | N |
| | Glutamine | Gln | Q |
| | Tyrosine | Tyr | Y |
| Polar, charged (at pH 6.0-7.0) | Lysine | Lys | K |
| | Arginine | Arg | R |
| | Histidine[4] | His | H |
| | Aspartate | Asp | D |
| | Glutamate | Glu | E |

Notes:

[1] Sometimes also considered to be a polar uncharged amino acid.

[2] Sometimes also considered to be a nonpolar uncharged amino acid.

[3] As will be clear to the skilled person, the fact that an amino acid residue is referred to in this Table as being either charged or uncharged at pH 6.0 to 7.0 does not reflect in any way on the charge said amino acid residue may have at a pH lower than 6.0 and/or at a pH higher than 7.0; the amino acid residues mentioned in the Table can be either charged and/or uncharged at such a higher or lower pH, as will be clear to the skilled person.

[4] As is known in the art, the charge of a His residue is greatly dependant upon even small shifts in pH, but a His residu can generally be considered essentially uncharged at a pH of about 6.5.

j) When comparing two amino acid sequences, the term *"amino acid difference"* refers to an insertion, deletion or substitution of a single amino acid residue on a position of the first sequence, compared to the second sequence; it being understood that two amino acid sequences can contain one, two or more such amino acid differences;

k) When a nucleotide sequence or amino acid sequence is said to "comprise" another nucleotide sequence or amino acid sequence, respectively, or to "essentially consist of" another nucleotide sequence or amino acid sequence, this may mean that the latter nucleotide sequence or amino acid sequence has been incorporated into the first mentioned nucleotide sequence or amino acid sequence, respectively, but more usually this generally means that the first mentioned nucleotide sequence or amino acid sequence comprises within its sequence a stretch of nucleotides or amino acid residues, respectively, that has the same nucleotide sequence or amino acid sequence, respectively, as the latter sequence, irrespective of how the first mentioned sequence has actually been generated or obtained (which may for example be by any suitable method described herein). By means of a non-limiting example, when a Nanobody of the invention is said to comprise a CDR sequence, this may mean that said CDR sequence has been incorporated into the Nanobody of the invention, but more usually this generally means that the Nanobody of the invention contains within its sequence a stretch of amino acid residues with the same amino acid sequence as said CDR sequence, irrespective of how said Nanobody of the invention has been generated or obtained. It should also be noted that when the latter amino acid sequence has a specific biological or structural function, it preferably has essentially the same, a similar or an equivalent biological or structural function in the first mentioned amino acid sequence (in other words, the first mentioned amino acid sequence is preferably such that the latter sequence is capable of performing essentially the same, a similar or an equivalent biological or structural function). For example, when a Nanobody of the invention is said to comprise a CDR sequence or framework sequence, respectively, the CDR sequence and framework are preferably capable, in said Nanobody, of functioning as a CDR sequence or framework sequence, respectively. Also, when a nucleotide sequence is said to comprise another nucleotide sequence, the first mentioned nucleotide sequence is preferably such that, when it is expressed into an expression product (e.g. a polypeptide), the amino acid sequence encoded by the latter nucleotide sequence forms part of said expression product (in other words, that the latter nucleotide sequence is in the same reading frame as the first mentioned, larger nucleotide sequence).

l) A nucleic acid sequence or amino acid sequence is considered to be *"(in) essentially isolated (form)"* - for example, compared to its native biological source and/or the reaction medium or cultivation medium from which it has been obtained - when it has been separated from at least one other component with which it is usually associated in said source or medium, such as another nucleic acid, another protein/polypeptide, another biological component or macromolecule or at least one contaminant, impurity or minor component. In particular, a nucleic acid sequence or amino acid sequence is considered "essentially isolated" when it has been purified at least 2-fold, in particular at least 10-fold, more in particular at least 100-fold, and up to 1000-fold or more. A nucleic acid sequence or amino acid sequence that is "in essentially isolated form" is preferably essentially homogeneous, as determined using a suitable technique, such as a suitable chromatographical technique, such as polyacrylamide-gel electrophoresis;

m) The term *"domain"* as used herein generally refers to a globular region of an amino acid sequence (such as an antibody chain, and in particular to a globular region of a heavy chain antibody), or to a polypeptide that essentially consists of such a globular region. Usually, such a domain will comprise peptide loops (for example 3 or 4 peptide loops) stabilized, for example, as a sheet or by disulfide bonds. The term *"binding domain"* refers to such a domain that is directed against an antigenic determinant (as defined herein);

n) The term *"antigenic determinant"* refers to the epitope on the antigen recognized by the antigen-binding molecule (such as a Nanobody or a polypeptide of the invention) and more in particular by the antigen-binding site of said molecule. The terms *"antigenic determinant"* and *"epitope"* may also be used interchangeably herein.

o) An amino acid sequence (such as a Nanobody, an antibody, a polypeptide of the invention, or generally an antigen binding protein or polypeptide or a fragment thereof) that can (specifically) bind to, that has affinity for and/or that has specificity for a specific antigenic determinant, epitope, antigen or protein (or for at least one part, fragment or epitope thereof) is said to be *"against"* or *"directed against"* said antigenic determinant, epitope, antigen or protein.

p) The term *"specificity"* refers to the number of different types of antigens or antigenic determinants to which a particular antigen-binding molecule or antigen-binding protein (such as a Nanobody or a polypeptide of the invention) molecule can bind. The specificity of an antigen-binding protein can be determined based on affinity and/or avidity. The affinity, represented by the equilibrium constant for the dissociation of an antigen with an antigen-binding protein ($K_D$), is a measure for the binding strength between an antigenic determinant and an antigen-binding site on the antigen-binding protein: the lesser the value of the $K_D$, the stronger the binding strength between an antigenic determinant and the antigen-binding molecule (alternatively, the affinity can also be expressed as the affinity constant ($K_A$), which is $1/K_D$). As will be clear to the skilled person (for example on the basis of the further disclosure herein), affinity can be determined in a manner known per se, depending on the specific antigen of interest. Avidity is the measure of the strength of binding between an antigen-binding molecule (such as a Nanobody or polypeptide of the invention) and the pertinent antigen. Avidity is related to both the affinity between an antigenic determinant and its antigen binding site on the antigen-binding molecule and the number of pertinent binding sites present on the antigen-binding molecule. Typically, antigen-binding proteins (such as the amino acid sequences, Nanobodies and/or polypeptides of the invention) will bind to their antigen with a dissociation constant ($K_D$) of $10^{-5}$ to $10^{-12}$ moles/liter or less, and preferably $10^{-7}$ to $10^{-12}$ moles/liter or less and more preferably $10^{-8}$ to $10^{-12}$ moles/liter (i.e. with an association constant ($K_A$) of $10^5$ to $10^{12}$ liter/ moles or more, and preferably $10^7$ to $10^{12}$ liter/moles or more and more preferably $10^8$ to $10^{12}$ liter/moles). Any $K_D$ value greater than $10^4$ mol/liter (or any $K_A$ value lower than $10^4$ M$^{-1}$) liters/mol is generally considered to indicate non-specific binding. Preferably, a monovalent immunoglobulin sequence of the invention will bind to the desired antigen with an affinity less than 500 nM, preferably less than 200 nM, more preferably less than 10 nM, such as less than 500 pM. Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as the other techniques mentioned herein. The dissociation constant may be the actual or apparent dissociation constant, as will be clear to the skilled person. Methods for determining the dissociation constant will be clear to the skilled person, and for example include the techniques mentioned herein. In this respect, it will also be clear that it may not be possible to measure dissociation constants of more then $10^{-4}$ moles/liter or $10^{-3}$ moles/liter (e.g., of $10^{-2}$ moles/liter).

Optionally, as will also be clear to the skilled person, the (actual or apparent) dissociation constant may be calculated on the basis of the (actual or apparent) association constant ($K_A$), by means of the relationship $[K_D = 1/K_A]$.

The affinity denotes the strength or stability of a molecular interaction. The affinity is commonly given as by the $K_D$, or dissociation constant, which has units of mol/liter (or M). The affinity can also be expressed as an association constant, $K_A$, which equals $1/K_D$ and has units of (mol/liter)$^{-1}$ (or M$^{-1}$). In the present specification, the stability of the interaction between two molecules (such as an amino acid sequence, Nanobody or polypeptide of the invention and its intended target) will mainly be expressed in terms of the $K_D$ value of their interaction; it being clear to the skilled person that in view of the relation $K_A = 1/K_D$, specifying the strength of molecular interaction by its $K_D$ value can also be used to calculate the corresponding $K_A$ value. The $K_D$-value characterizes the strength of a molecular interaction also in a thermodynamic sense as it is related to the free energy (DG) of binding by the well known relation DG=RT.ln($K_D$) (equivalently DG=-RT.ln($K_A$)), where R equals the gas constant, T equals the absolute temperature and ln denotes the natural logarithm. The $K_D$ for biological interactions which are considered meaningful (e.g. specific) are typically in the range of $10^{-10}$M (0.1 nM) to $10^{-5}$M (10000 nM). The stronger an interaction is, the lower is its $K_D$. The $K_D$ can also be expressed as the ratio of the dissociation rate constant of a complex, denoted as $k_{off}$, to the rate of its association, denoted $k_{on}$ (so that

$K_D = k_{off}/k_{on}$ and $K_A = k_{on}/k_{off}$). The off-rate $k_{off}$ has units $s^{-1}$ (where s is the SI unit notation of second). The on-rate $k_{on}$ has units $M^{-1}s^{-1}$. The on-rate may vary between $10^2$ $M^{-1}s^{-1}$ to about $10^7$ $M^{-1}s^{-1}$, approaching the diffusion-limited association rate constant for bimolecular interactions. The off-rate is related to the half-life of a given molecular interaction by the relation $t_{1/2} = \ln(2)/k_{off}$. The off-rate may vary between $10^{-6}$ $s^{-1}$ (near irreversible complex with a $t_{1/2}$ of multiple days) to 1 $s^{-1}$ ($t_{1/2} = 0.69$ s).

The affinity of a molecular interaction between two molecules can be measured via different techniques known per se, such as the well known surface plasmon resonance (SPR) biosensor technique (see for example Ober et al., Intern. Immunology, 13, 1551-1559, 2001) where one molecule is immobilized on the biosensor chip and the other molecule is passed over the immobilized molecule under flow conditions yielding $k_{on}$, $k_{off}$ measurements and hence $K_D$ (or $K_A$) values. This can for example be performed using the well-known BIACORE instruments.

It will also be clear to the skilled person that the measured $K_D$ may correspond to the apparent $K_D$ if the measuring process somehow influences the intrinsic binding affinity of the implied molecules for example by artefacts related to the coating on the biosensor of one molecule. Also, an apparent $K_D$ may be measured if one molecule contains more than one recognition sites for the other molecule. In such situation the measured affinity may be affected by the avidity of the interaction by the two molecules.

Another approach that may be used to assess affinity is the 2-step ELISA (Enzyme-Linked Immunosorbent Assay) procedure of Friguet et al. (J. Immunol. Methods, 77, 305-19, 1985). This method establishes a solution phase binding equilibrium measurement and avoids possible artefacts relating to adsorption of one of the molecules on a support such as plastic.

However, the accurate measurement of $K_D$ may be quite labor-intensive and as consequence, often apparent $K_D$ values are determined to assess the binding strength of two molecules. It should be noted that as long all measurements are made in a consistent way (e.g. keeping the assay conditions unchanged) apparent $K_D$ measurements can be used as an approximation of the true $K_D$ and hence in the present document $K_D$ and apparent $K_D$ should be treated with equal importance or relevance.

Finally, it should be noted that in many situations the experienced scientist may judge it to be convenient to determine the binding affinity relative to some reference molecule. For example, to assess the binding strength between molecules A and B, one may e.g. use a reference molecule C that is known to bind to B and that is suitably labelled with a fluorophore or chromophore group or other chemical moiety, such as biotin for easy detection in an ELISA or FACS (Fluorescent activated cell sorting) or other format (the fluorophore for fluorescence detection, the chromophore for light absorption detection, the biotin for streptavidin-mediated ELISA detection). Typically, the reference molecule C is kept at a fixed concentration and the concentration of A is varied for a given concentration or amount of B. As a result an $IC_{50}$ value is obtained corresponding to the concentration of A at which the signal measured for C in absence of A is halved. Provided $K_{D\ ref}$, the $K_D$ of the reference molecule, is known, as well as the total concentration $c_{ref}$ of the reference molecule, the apparent $K_D$ for the interaction A-B can be obtained from following formula: $K_D = IC_{50}/(1+c_{ref}/K_{D\ ref})$. Note that if $c_{ref}$ $<<K_{D\ ref}$, $K_D \approx IC_{50}$. Provided the measurement of the $IC_{50}$ is performed in a consistent way (e.g. keeping $c_{ref}$ fixed) for the binders that are compared, the strength or stability of a molecular interaction can be assessed by the $IC_{50}$ and this measurement is judged as equivalent to $K_D$ or to apparent $K_D$ throughout this text.

q) The half-life of an amino acid sequence, compound or polypeptide of the invention can generally be defined as the time taken for the serum concentration of the amino acid sequence, compound or polypeptide to be reduced by 50%, in vivo, for example due to degradation of the sequence or compound and/or clearance or sequestration of the sequence or compound by natural mechanisms. The in vivo half-life of an amino acid sequence, compound or polypeptide of the invention can be determined in any manner known per se, such as by pharmacokinetic analysis. Suitable techniques will be clear to the person skilled in the art, and may for example generally involve the steps of suitably administering to a warm-blooded animal (i.e. to a human or to another suitable mammal, such as a mouse, rabbit, rat, pig, dog or a primate, for example monkeys from the genus *Macaca* (such as, and in particular, cynomologus monkeys (*Macaca fascicularis*) and/or rhesus monkeys (*Macaca mulatta*)) and baboon *(Papio ursinus)*) a suitable dose of the amino acid sequence, compound or polypeptide of the invention; collecting blood samples or other samples from said animal; determining the level or concentration of the amino acid sequence, compound or polypeptide of the invention in said blood sample; and calculating, from (a plot of) the data thus obtained, the time until the level or concentration of the amino acid sequence, compound or polypeptide of the invention has been reduced by 50% compared to the initial level upon dosing. Reference is for example made to the Experimental Part below, as well as to the standard handbooks, such as Kenneth, A et al: Chemical Stability of Pharmaceuticals: A Handbook for Pharmacists and Peters et al, Pharmacokinete analysis: A Practical Approach (1996). Reference is also made to "Pharmacokinetics", M Gibaldi & D Perron, published by Marcel Dekker, 2nd Rev. edition (1982).

As will also be clear to the skilled person (see for example pages 6 and 7 of WO 04/003019 and in the further references cited therein), the half-life can be expressed using parameters such as the t1/2-alpha, t1/2-beta and the area under the curve (AUC). In the present specification, an "increase in half-life" refers to an increase in any one of these parameters, such as any two of these parameters, or essentially all three these parameters. As used herein "increase in half-life" or "increased half-life" in particular refers to an increase in the t1/2-beta, either with or without an increase in the t1/2-alpha and/or the AUC or both.

r) In respect of a target or antigen, the term "interaction site" on the target or antigen means a site, epitope, antigenic determinant, part, domain or stretch of amino acid residues on the target or antigen that is a site for binding to a ligand, receptor or other binding partner, a catalytic site, a cleavage site, a site for allosteric interaction, a site involved in multimerisation (such as homomerization or heterodimerization) of the target or antigen; or any other site, epitope, antigenic determinant, part, domain or stretch of amino acid residues on the target or antigen that is involved in a biological action or mechanism of the target or antigen. More generally, an "interaction site" can be any site, epitope, antigenic determinant, part, domain or stretch of amino acid residues on the target or antigen to which an amino acid sequence or polypeptide of the invention can bind such that the target or antigen (and/or any pathway, interaction, signalling, biological mechanism or biological effect in which the target or antigen is involved) is modulated (as defined herein).

s) An amino acid sequence or polypeptide is said to be *"specific for"* a first target or antigen compared to a second target or antigen when is binds to the first antigen with an affinity (as described above, and suitably expressed as a $K_D$ value, $K_A$ value, $K_{off}$ rate and/or $K_{on}$ rate) that is at least 10 times, such as at least 100 times, and preferably at least 1000 times, and up to 10.000 times or more better than the affinity with which said amino acid sequence or polypeptide binds to the second target or polypeptide. For example, the first antigen may bind to the target or antigen with a $K_D$ value that is at least 10 times less, such as at least 100 times less, and preferably at least 1000 times less, such as 10.000 times less or even less than that, than the $K_D$ with which said amino acid sequence or polypeptide binds to the second target or polypeptide. Preferably, when an amino acid sequence or polypeptide is "specific for" a first target or antigen compared to a second target or antigen, it is directed against (as defined herein) said first target or antigen, but not directed against said second target or antigen.

t) As further described herein, the total number of amino acid residues in a Nanobody can be in the region of 110-130, is preferably 112-115, and is most preferably 113. It should however be noted that parts, fragments, analogs or derivatives (as further described herein) of a Nanobody are not particularly limited as to their length and/or size, as long as such parts, fragments, analogs or derivatives meet the further requirements outlined herein and are also preferably suitable for the purposes described herein;

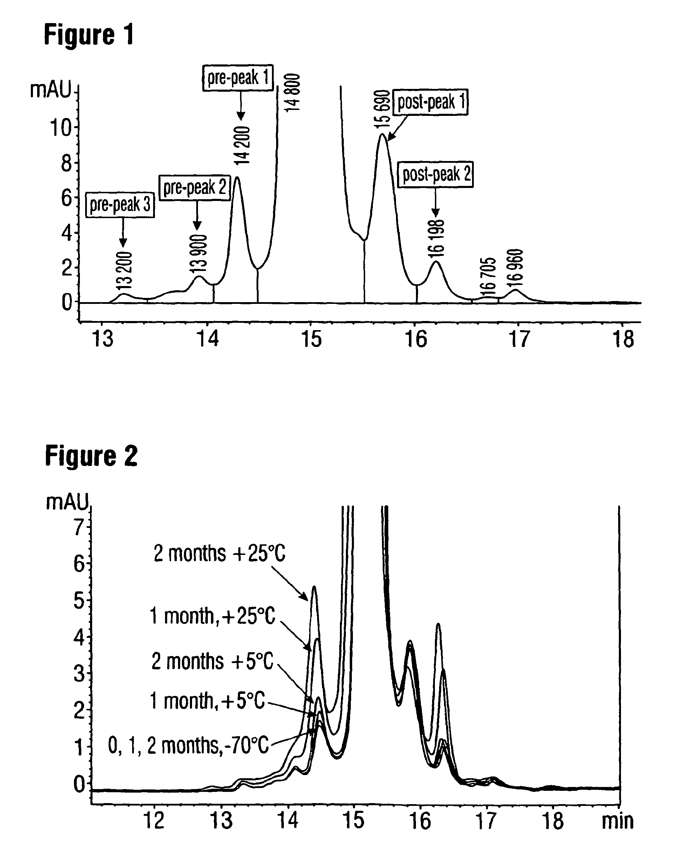

u) The amino acid residues of a Nanobody are generally numbered according to the general numbering for $V_H$ domains given by Kabat et al. ("Sequence of proteins of immunological interest", US Public Health Services, NIH Bethesda, MD, Publication No. 91) unless specified otherwise, as applied to $V_{HH}$ domains from Camelids in the article of Riechmann and Muyldermans, J. Immunol. Methods 2000 Jun 23; 240 (1-2): 185-195 (see for example Figure 2 of this publication); or referred to herein. According to this numbering, FR1 of a Nanobody comprises the amino acid residues at positions 1-30, CDR1 of a Nanobody comprises the amino acid residues at positions 31-35, FR2 of a Nanobody comprises the amino acids at positions 36-49, CDR2 of a Nanobody comprises the amino acid residues at positions 50-65, FR3 of a Nanobody comprises the amino acid residues at positions 66-94, CDR3 of a Nanobody comprises the amino acid residues at positions 95-102, and FR4 of a Nanobody comprises the amino acid residues at positions 103-113. [In this respect, it should be noted that - as is well known in the art for $V_H$ domains and for $V_{HH}$ domains - the total number of amino acid residues in each of the CDR's may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Generally, however, it can be said that, according to the numbering of Kabat and irrespective of the number of amino acid residues in the CDR's, position 1 according to the Kabat numbering corresponds to the start of FR1 and vice versa, position 36 according to the Kabat numbering corresponds to the start of FR2 and vice versa, position 66 according to the Kabat numbering corresponds to the start of FR3 and vice versa, and position 103 according to the Kabat numbering corresponds to the start of FR4 and vice versa.]. Alternative methods for numbering the amino acid residues of $V_H$ domains, which methods can also be applied in an analogous manner to $V_{HH}$ domains from Camelids and to Nanobodies, are the method described by Chothia et al. (Nature 342, 877-883 (1989)), the so-called "AbM definition" and the so-called "contact definition". However, in the present description, aspects and figures, the numbering according to Kabat as applied to $V_{HH}$ domains by Riechmann and Muyldermans will be followed, unless indicated otherwise; and

v) Stability as referred to herein relates to the chemical stability over time, i.e. the stability of structural primary sequence of the protein, polypeptide or single variable domain of the inventions. E.g. if the skilled person in the art tests a compound of interested and cannot find any alteration (or only minor alteration e.g. less than 1%, 2%, 3%, 4%, 5%, 10% other material in a given sample, e.g. by separation technique such as RPC or HIC) in the primary sequence of the protein or polypeptide, e.g. the primary amino acid sequence, over time, then the protein or polypeptide is considered stable over the measured time period and the given conditions such as e.g. temperature.

w) The Figures, Sequence Listing and the Experimental Part/Examples are only given to further illustrate the invention and should not be interpreted or construed as limiting the scope of the invention and/or of the appended aspects in any way, unless explicitly indicated otherwise herein.

Without being limited thereto, Nanobodies, (single) domain antibodies or "dAb's" can be derived from the variable region of a 4-chain antibody as well as from the variable region of a heavy chain antibody. In accordance with the terminology used in the references below, the variable domains present in naturally occurring heavy chain antibodies will also be referred to as $"V_{HH}$ domains", in order to distinguish them from the heavy chain variable domains that are present in conventional 4-chain antibodies (which will be referred to hereinbelow as $"V_H$ domains") and from the light chain variable domains that are present in conventional 4-chain antibodies (which will be referred to hereinbelow as $"Y_L$ domains").

Thus - without being limited thereto - the polypeptide or protein of the invention has an amino acid sequence that comprises or essentially consists of four framework regions (FR1 to FR4, respectively) and three complementarity determining regions (CDR1 to CDR3, respectively). Such an amino acid sequence preferably contains between 80 and 200 amino acid residues, such as between 90 and 150 amino acid residues, such as about 100-130 amino acid residues (although suitable fragments of such an amino acid sequence - i.e. essentially as described herein for the Nanobodies of the invention or equivalent thereto - may also be used), and is preferably such that it forms an immunoglobulin fold or such that, under suitable conditions, it is capable of forming an immunoglobulin fold (i.e. by suitable folding). The amino acid sequence is preferably chosen from Nanobodies, domain antibodies, single domain antibodies or "dAb's", and is most preferably a Nanobody as defined herein. The CDR's may be any suitable CDR's that provide the desired property to the polypeptide or protein.

A further advantage of the invention is that polypeptides of the invention and in particular of Nanobodies can be produced according to the invention in a stable and less immunogenic form. The process according to the invention is therefore of major importance for the therapeutic use of recombinant polypeptide hybrids.

In addition polypeptides of the invention can be tailor-made to suit a large number of effector functions by selection in the immune system. This natural protein engineering system has an unrivalled efficiency. The cytoplasmic expression of special functional single variable domains enables such effector functions to be introduced into the cells. Applications are advantageous which result in the modulation of the activity of cellular proteins. This can for example be achieved by stabilizing the target protein by protein-single variable domains complex formation. This can lead to a change in the degradation kinetics. Allosteric effector actions are also possible. The approximation of two effectors by the formation and stabilization of a ternary complex creates a further possibility for influencing metabolic paths for example by artificial multi-enzyme complexes or the local increase of metabolite concentrations of inducible operators. However, the cytoplasmic expression of catalytic antibodies is particularly advantageous and the associated possibility of selecting for catalytic efficiency. A cytoplasmic expression of functional single variable domains can be accomplished in a simple manner for polypeptides stabilized according to the invention.

The invention also relates to a polypeptide selected from the group consisting of SEQ ID NO: 25 and SEQ ID NO: 26 and a nucleotide sequence selected from the group consisting of SEQ ID NO: 33 and SEQ ID NO: 34.

The amino acid sequences, Nanobodies, polypeptides and nucleic acids of the invention can be prepared in a manner known per se, as will be clear to the skilled person from the further description herein. For example, the Nanobodies and polypetides of the invention can be prepared in any manner known per se for the preparation of antibodies and in particular for the preparation of antibody fragments (including but not limited to (single) domain anti-bodies and ScFv fragments). Some preferred, but non-limiting methods for preparing the amino acid sequences, Nanobodies, polypeptides and nucleic acids include the methods and techniques described herein.

Other embodiments of this invention are the proteins, polypeptides, single variable domains, libraries, nucleotides or selection thereof derived or obtainable or directly obtainable by the methods described herein.

As will be clear to the skilled person, one particularly useful method for preparing an amino acid sequence, Nanobody and/or a polypeptide of the invention generally comprises the steps of:

i) the expression, in a suitable host cell or host organism (also referred to herein as a "host of the invention") or in another suitable expression system of a nucleic acid that encodes said amino acid sequence, Nanobody or polypeptide of the invention (also referred to herein as a *"nucleic acid of the invention"),* optionally followed by:

ii) isolating and/or purifying the amino acid sequence, Nanobody or polypeptide of the invention thus obtained.

In particular, such a method may comprise the steps of:

i) cultivating and/or maintaining a host of the invention under conditions that are such that said host of the invention expresses and/or produces at least one amino acid sequence, Nanobody and/or polypeptide of the invention; optionally followed by:

ii) isolating and/or purifying the amino acid sequence, Nanobody or polypeptide of the invention thus obtained.

A nucleic acid of the invention can be in the form of single or double stranded DNA or RNA, and is preferably in the form of double stranded DNA. For example, the nucleotide sequences of the invention may be genomic DNA, cDNA or synthetic DNA (such as DNA with a codon usage that has been specifically adapted for expression in the intended host cell or host organism).

According to one aspect of the invention, the nucleic acid of the invention is in essentially isolated from, as defined herein. The nucleic acid of the invention may also be in the form of, be present in and/or be part of a vector, such as for example a plasmid, cosmid or YAC, which again may be in essentially isolated form. The nucleic acids of the invention can be prepared or obtained in a manner known per se, based on the information on the amino acid sequences for the polypeptides of the invention given herein, and/or can be isolated from a suitable natural source. To provide analogs, nucleotide sequences encoding naturally occurring $V_{HH}$ domains can for example be subjected to site-directed mutagenesis, so at to provide a nucleic acid of the invention encoding said analog. Also, as will be clear to the skilled person, to prepare a nucleic acid of the invention, also several nucleotide sequences, such as at least one nucleotide sequence encoding a Nanobody and for example nucleic acids encoding one or more linkers can be linked together in a suitable manner. Techniques for generating the nucleic acids of the invention will be clear to the skilled person and may for instance include, but are not limited to, automated DNA synthesis; site-directed mutagenesis; combining two or more naturally occurring and/or synthetic sequences (or two or more parts thereof), introduction of mutations that lead to the expression of a truncated expression product; introduction of one or more restriction sites (e.g. to create cassettes and/or regions that may easily be digested and/or ligated using suitable restriction enzymes), and/or the introduction of mutations by means of a PCR reaction using one or more "mismatched" primers. These and other techniques will be clear to the skilled person, and reference is again made to the standard handbooks, such as Sambrook et al. and Ausubel et al., mentioned above, as well as the Examples below.

The nucleic acid of the invention may also be in the form of, be present in and/or be part of a genetic construct, as will be clear to the person skilled in the art. Such genetic constructs generally comprise at least one nucleic acid of the invention that is optionally linked to one or more elements of genetic constructs known per se, such as for example one or more suitable regulatory elements (such as a suitable promoter(s), enhancer(s), terminator(s), etc.) and the further elements of genetic constructs referred to herein. Such genetic constructs comprising at least one nucleic acid of the invention will also be referred to herein as "genetic constructs of the invention".

The genetic constructs of the invention may be DNA or RNA, and are preferably doublestranded DNA. The genetic constructs of the invention may also be in a form suitable for transformation of the intended host cell or host organism, in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e. a vector that can provide for expression in vitro and/or in vivo (e.g. in a suitable host cell, host organism and/or expression system).

In a preferred but non-limiting aspect, a genetic construct of the invention comprises

i) at least one nucleic acid of the invention; operably connected to
ii) one or more regulatory elements, such as a promoter and optionally a suitable terminator; and optionally also
iii) one or more further elements of genetic constructs known per se;

in which the terms "regulatory element", "promoter", "terminator" and "operably connected" have their usual meaning in the art (as further described herein); and in which said "further elements" present in the genetic constructs may for example be 3'- or 5'-UTR sequences, leader sequences, selection markers, expression markers/reporter genes, and/or elements that may facilitate or increase (the efficiency of) transformation or integration. These and other suitable elements for such genetic constructs will be clear to the skilled person, and may for instance depend upon the type of construct used, the intended host cell or host organism; the manner in which the nucleotide sequences of the invention of interest are to be expressed (e.g. via constitutive, transient or inducible expression); and/or the transformation technique to be used. For example, regulatory requences, promoters and terminators known per se for the expression and production of antibodies and antibody fragments (including but not limited to (single) domain antibodies and ScFv fragments) may be used in an essentially analogous manner.

Preferably, in the genetic constructs of the invention, said at least one nucleic acid of the invention and said regulatory elements, and optionally said one or more further elements, are "operably linked" to each other, by which is generally meant that they are in a functional relationship with each other. For instance, a promoter is considered "operably linked" to a coding sequence if said promoter is able to initiate or otherwise control/regulate the transcription and/or the expression of a coding sequence (in which said coding sequence should be understood as being "under the control of" said promotor). Generally, when two nucleotide sequences are operably linked, they will be in the same orientation and usually also in the same reading frame. They will usually also be essentially contiguous, although this may also not be required.

Preferably, the regulatory and further elements of the genetic constructs of the invention are such that they are capable of providing their intended biological function in the intended host cell or host organism.

For instance, a promoter, enhancer or terminator should be "operable" in the intended host cell or host organism, by which is meant that (for example) said promoter should be capable of initiating or otherwise controlling/regulating the transcription and/or the expression of a nucleotide sequence - e.g. a coding sequence - to which it is operably linked (as defined herein).

Some particularly preferred promoters include, but are not limited to, promoters known per se for the expression in the host cells mentioned herein; and in particular promoters for the expression in the bacterial cells, such as those mentioned herein and/or those used in the Examples.

A selection marker should be such that it allows - i.e. under appropriate selection conditions - host cells and/or host organisms that have been (successfully) transformed with the nucleotide sequence of the invention to be distinguished from host cells/organisms that have not been (successfully) transformed. Some preferred, but non-limiting examples of such markers are genes that provide resistance against antibiotics (such as kanamycin or ampicillin), genes that provide for temperature resistance, or genes that allow the host cell or host organism to be maintained in the absence of certain factors, compounds and/or (food) components in the medium that are essential for survival of the non-transformed cells or organisms.

A leader sequence should be such that - in the intended host cell or host organism - it allows for the desired post-translational modifications and/or such that it directs the transcribed mRNA to a desired part or organelle of a cell. A leader sequence may also allow for secretion of the expression product from said cell. As such, the leader sequence may be any pro-, pre-, or prepro-sequence operable in the host cell or host organism. Leader sequences may not be required for expression in a bacterial cell. For example, leader sequences known per se for the expression and production of antibodies and antibody fragments (including but not limited to single domain antibodies and ScFv fragments) may be used in an essentially analogous manner.

An expression marker or reporter gene should be such that - in the host cell or host organism - it allows for detection of the expression of (a gene or nucleotide sequence present on) the genetic construct. An expression marker may optionally also allow for the localisation of the expressed product, e.g. in a specific part or organelle of a cell and/or in (a) specific cell(s), tissue(s), organ(s) or part(s) of a multicellular organism. Such reporter genes may also be expressed as a protein fusion with the amino acid sequence of the invention. Some preferred, but non-limiting examples include fluorescent proteins such as GFP.

Some preferred, but non-limiting examples of suitable promoters, terminator and further elements include those that can be used for the expression in the host cells mentioned herein; and in particular those that are suitable for expression in bacterial cells, such as those mentioned herein and/or those used in the Examples below. For some (further) non-limiting examples of the promoters, selection markers, leader sequences, expression markers and further elements that may be present/used in the genetic constructs of the invention - such as terminators, transcriptional and/or translational enhancers and/or integration factors - reference is made to the general handbooks such as Sambrook et al. and Ausubel et al. mentioned above, as well as to the examples that are given in WO 95/07463, WO 96/23810, WO 95/07463, WO 95/21191, WO 97/11094, WO 97/42320, WO 98/06737, WO 98/21355, US-A-7,207,410, US-A- 5,693,492 and EP 1 085 089. Other examples will be clear to the skilled person. Reference is also made to the general background art cited above and the further references cited herein.

The genetic constructs of the invention may generally be provided by suitably linking the nucleotide sequence(s) of the invention to the one or more further elements described above, for example using the techniques described in the general handbooks such as Sambrook et al. and Ausubel et al., mentioned above. Often, the genetic constructs of the invention will be obtained by inserting a nucleotide sequence of the invention in a suitable (expression) vector known per se. Some preferred, but non-limiting examples of suitable expression vectors are those used in the Examples below, as well as those mentioned herein.

The nucleic acids of the invention and/or the genetic constructs of the invention may be used to transform a host cell or host organism, i.e. for expression and/or production of the amino acid sequence, Nanobody or polypeptide of the invention. Suitable hosts or host cells will be clear to the skilled person, and may for example be any suitable fungal, prokaryotic or eukaryotic cell or cell line or any suitable fungal, prokaryotic or eukaryotic organism, for example:

- a bacterial strain, including but not limited to gram-negative strains such as strains of *Escherichia coli;* of *Proteus,* for example of *Proteus mirabilis;* of *Pseudomonas,* for example of *Pseudomonas fluorescens;* and gram-positive strains such as strains of *Bacillus,* for example of *Bacillus subtilis* or of *Bacillus brevis;* of *Streptomyces,* for example of *Streptomyces lividans;* of *Staphylococcus,* for example of *Staphylococcus carnosus;* and of *Lactococcus,* for example of *Lactococcus lactis;*

- a fungal cell, including but not limited to cells from species of *Trichoderma,* for example from *Trichoderma reesei;* of *Neurospora,* for example from *Neurospora crassa;* of *Sordaria,* for example from *Sordaria macrospora;* of *Aspergillus,* for example from *Aspergillus niger* or from *Aspergillus sojae;* or from other filamentous fungi;

- a yeast cell, including but not limited to cells from species of *Saccharomyces,* for example of *Saccharomyces*

*cerevisiae;* of *Schizosaccharomyces,* for example of *Schizosaccharomyces pombe;* of *Pichia,* for example of *Pichia pastoris* or of *Pichia methanolica;* of *Hansenula,* for example of *Hansenula polymorpha;* of *Kluyveromyces,* for example of *Kluyveromyces lactis;* of *Arxula,* for example of *Arxula adeninivorans;* of *Yarrowia,* for example of *Yarrowia lipolytica;*

- an amphibian cell or cell line, such as *Xenopus oocytes;*
- an insect-derived cell or cell line, such as cells/cell lines derived from lepidoptera, including but not limited to *Spodoptera* SF9 and Sf21 cells or cells/cell lines derived from *Drosophila,* such as Schneider and Kc cells;
- a plant or plant cell, for example in tobacco plants; and/or
- a mammalian cell or cell line, for example a cell or cell line derived from a human, a cell or a cell line from mammals including but not limited to CHO-cells, BHK-cells (for example BHK-21 cells) and human cells or cell lines such as HeLa, COS (for example COS-7) and PER.C6 cells;

as well as all other hosts or host cells known per se for the expression and production of antibodies and antibody fragments (including but not limited to (single) domain antibodies and ScFv fragments), which will be clear to the skilled person. Reference is also made to the general background art cited hereinabove, as well as to for example WO 94/29457; WO 96/34103; WO 99/42077; Frenken et al., (1998), supra; Riechmann and Muyldermans, (1999), supra; van der Linden, (2000), supra; Thomassen et al., (2002), supra; Joosten et al., (2003), supra; Joosten et al., (2005), supra; and the further references cited herein.

The amino acid sequences, Nanobodies and polypeptides of the invention can also be introduced and expressed in one or more cells, tissues or organs of a multicellular organism, for example for prophylactic and/or therapeutic purposes (e.g. as a gene therapy). For this purpose, the nucleotide sequences of the invention may be introduced into the cells or tissues in any suitable way, for example as such (e.g. using liposomes) or after they have been inserted into a suitable gene therapy vector (for example derived from retroviruses such as adenovirus, or parvoviruses such as adeno-associated virus). As will also be clear to the skilled person, such gene therapy may be performed in vivo and/or in situ in the body of a patient by administering a nucleic acid of the invention or a suitable gene therapy vector encoding the same to the patient or to specific cells or a specific tissue or organ of the patient; or suitable cells (often taken from the body of the patient to be treated, such as explanted lymphocytes, bone marrow aspirates or tissue biopsies) may be treated in vitro with a nucleotide sequence of the invention and then be suitably (re-)introduced into the body of the patient. All this can be performed using gene therapy vectors, techniques and delivery systems which are well known to the skilled person, and for example described in Culver, K. W., "Gene Therapy", 1994, p. xii, Mary Ann Liebert, Inc., Publishers, New York, N.Y); Giordano, Nature F Medicine 2 (1996), 534-539; Schaper, Circ. Res. 79 (1996), 911-919; Anderson, Science 256 (1992),808-813; Verma, Nature 389 (1994),239; Isner, Lancet 348 (1996),370-374; Muhlhauser, Circ. Res. 77 (1995),1077-1086; Onodera, Blood 91; (1998),30- 36; Verma, Gene Ther. 5 (1998),692-699; Nabel, Ann. N.Y. Acad. Sci. : 811 (1997), 289-292; Verzeletti, Hum. Gene Ther. 9 (1998), 2243-51; Wang, Nature Medicine 2 (1996),714-716; WO 94/29469; WO 97/00957, US 5,580,859; US 5,5895466; or Schaper, Current Opinion in Biotechnology 7 (1996), 635-640. For example, in situ expression of ScFv fragments (Afanasieva et al., Gene Ther., 10, 1850-1859 (2003)) and of diabodies (Blanco et al., J. Immunol, 171, 1070-1077 (2003)) has been described in the art.

For expression of the Nanobodies in a cell, they may also be expressed as so-called "intrabodies", as for example described in WO 94/02610, WO 95/22618 and US-A-7004940; WO 03/014960; in Cattaneo, A. & Biocca, S. (1997) Intracellular Antibodies: Development and Applications. Landes and Springer-Verlag; and in Kontermann, Methods 34, (2004), 163-170.

The amino acid sequences, Nanobodies and polypeptides of the invention can for example also be produced in the milk of transgenic mammals, for example in the milk of rabbits, cows, goats or sheep (see for example US-A-6,741,957, US-A-6,304,489 and US-A-6,849,992 for general techniques for introducing transgenes into mammals), in plants or parts of plants including but not limited to their leaves, flowers, fruits, seed, roots or turbers (for example in tobacco, maize, soybean or alfalfa) or in for example pupae of the silkworm *Bombix mori.*

Furthermore, the amino acid sequences, Nanobodies and polypeptides of the invention can also be expressed and/or produced in cell-free expression systems, and suitable examples of such systems will be clear to the skilled person. Some preferred, but non-limiting examples include expression in the wheat germ system; in rabbit reticulocyte lysates; or in the *E. coli* Zubay system.

As mentioned above, one of the advantages of the use of Nanobodies is that the polypeptides based thereon can be prepared through expression in a suitable bacterial system, and suitable bacterial expression systems, vectors, host cells, regulatory elements, etc., will be clear to the skilled person, for example from the references cited above. It should however be noted that the invention in its broadest sense is not limited to expression in bacterial systems.

Preferably, in the invention, an (in vivo or in vitro) expression system, such as a bacterial expression system, is used that provides the polypeptides of the invention in a form that is suitable for pharmaceutical use, and such expression systems will again be clear to the skilled person. As also will be clear to the skilled person, polypeptides of the invention suitable for pharmaceutical use can be prepared using techniques for peptide synthesis.

For production on industrial scale, preferred heterologous hosts for the (industrial) production of Nanobodies or Nanobody-containing protein therapeutics include strains of *E. coli, Pichia pastoris, S. cerevisiae* that are suitable for large scale expression/production/fermentation, and in particular for large scale pharmaceutical (i.e. GMP grade) expression/production/fermentation. Suitable examples of such strains will be clear to the skilled person. Such strains and production/expression systems are also made available by companies such as Biovitrum (Uppsala, Sweden).

Alternatively, mammalian cell lines, in particular Chinese hamster ovary (CHO) cells, can be used for large scale expression/production/fermentation, and in particular for large scale pharmaceutical expression/production/fermentation. Again, such expression/production systems are also made available by some of the companies mentioned above.

The choice of the specific expression system would depend in part on the requirement for certain post-translational modifications, more specifically glycosylation. The production of a Nanobody-containing recombinant protein for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression. Preferably, either a human cell or cell line is used (i.e. leading to a protein that essentially has a human glycosylation pattern) or another mammalian cell line is used that can provide a glycosylation pattern that is essentially and/or functionally the same as human glycosylation or at least mimics human glycosylation. Generally, prokaryotic hosts such as *E. coli* do not have the ability to glycosylate proteins, and the use of lower eukaryotes such as yeast usually leads to a glycosylation pattern that differs from human glycosylation. Nevertheless, it should be understood that all the foregoing host cells and expression systems can be used in the invention, depending on the desired amino acid sequence, Nanobody or polypeptide to be obtained.

Thus, according to one non-limiting aspect of the invention, the amino acid sequence, Nanobody or polypeptide of the invention is glycosylated. According to another non-limiting aspect of the invention, the amino acid sequence, Nanobody or polypeptide of the invention is non-glycosylated.

According to one preferred, but non-limiting aspect of the invention, the amino acid sequence, Nanobody or polypeptide of the invention is produced in a bacterial cell, in particular a bacterial cell suitable for large scale pharmaceutical production, such as cells of the strains mentioned above.

According to another preferred, but non-limiting aspect of the invention, the amino acid sequence, Nanobody or polypeptide of the invention is produced in a yeast cell, in particular a yeast cell suitable for large scale pharmaceutical production, such as cells of the species mentioned above.

According to yet another preferred, but non-limiting aspect of the invention, the amino acid sequence, Nanobody or polypeptide of the invention is produced in a mammalian cell, in particular in a human cell or in a cell of a human cell line, and more in particular in a human cell or in a cell of a human cell line that is suitable for large scale pharmaceutical production, such as the cell lines mentioned hereinabove.

When expression in a host cell is used to produce the amino acid sequences, Nanobodies and the polypeptides of the invention, the amino acid sequences, Nanobodies and polypeptides of the invention can be produced either intracellularly (e.g. in the cytosol, in the periplasma or in inclusion bodies) and then isolated from the host cells and optionally further purified; or can be produced extracellularly (e.g. in the medium in which the host cells are cultured) and then isolated from the culture medium and optionally further purified. When eukaryotic host cells are used, extracellular production is usually preferred since this considerably facilitates the further isolation and downstream processing of the Nanobodies and proteins obtained. Bacterial cells such as the strains of *E. coli* mentioned above normally do not secrete proteins extracellularly, except for a few classes of proteins such as toxins and hemolysin, and secretory production in *E. coli* refers to the translocation of proteins across the inner membrane to the periplasmic space. Periplasmic production provides several advantages over cytosolic production. For example, the N-terminal amino acid sequence of the secreted product can be identical to the natural gene product after cleavage of the secretion signal sequence by a specific signal peptidase. Also, there appears to be much less protease activity in the periplasm than in the cytoplasm. In addition, protein purification is simpler due to fewer contaminating proteins in the periplasm. Another advantage is that correct disulfide bonds may form because the periplasm provides a more oxidative environment than the cytoplasm. Proteins overexpressed in *E. coli* are often found in insoluble aggregates, so-called inclusion bodies. These inclusion bodies may be located in the cytosol or in the periplasm; the recovery of biologically active proteins from these inclusion bodies requires a denaturation/refolding process. Many recombinant proteins, including therapeutic proteins, are recovered from inclusion bodies. Alternatively, as will be clear to the skilled person, recombinant strains of bacteria that have been genetically modified so as to secrete a desired protein, and in particular an amino acid sequence, Nanobody or a polypeptide of the invention, can be used.

Thus, according to one non-limiting aspect of the invention, the amino acid sequence, Nanobody or polypeptide of the invention is an amino acid sequence, Nanobody or polypeptide that has been produced intra-cellularly and that has been isolated from the host cell, and in particular from a bacterial cell or from an inclusion body in a bacterial cell. According to another non-limiting aspect of the invention, the amino acid sequence, Nanobody or polypeptide of the invention is an amino acid sequence, Nanobody or polypeptide that has been produced extracellularly, and that has been isolated from the medium in which the host cell is cultivated.

Some preferred, but non-limiting promoters for use with these host cells include,

- for expression in *E. coli:* lac promoter (and derivatives thereof such as the lacUV5 promoter); arabinose promoter; left- (PL) and rightward (PR) promoter of phage lambda; promoter of the trp operon; hybrid lac/trp promoters (tac and trc); T7-promoter (more specifically that of T7-phage gene 10) and other T-phage promoters; promoter of the Tn10 tetracycline resistance gene; engineered variants of the above promoters that include one or more copies of an extraneous regulatory operator sequence;
- for expression in *S. cerevisiae:* constitutive: ADH1 (alcohol dehydrogenase 1), ENO (enolase), CYC1 (cytochrome c iso-1), GAPDH (glyceraldehydes-3-phosphate dehydrogenase), PGK1 (phosphoglycerate kinase), PYK1 (pyruvate kinase); regulated: GAL1,10,7 (galactose metabolic enzymes), ADH2 (alcohol dehydrogenase 2), PHO5 (acid phosphatase), CUP1 (copper metallothionein); heterologous: CaMV (cauliflower mosaic virus 35S promoter);
- for expression in *Pichia pastoris:* the AOX1 promoter (alcohol oxidase I);
- for expression in mammalian cells: human cytomegalovirus (hCMV) immediate early enhancer/promoter; human cytomegalovirus (hCMV) immediate early promoter variant that contains two tetracycline operator sequences such that the promoter can be regulated by the Tet repressor; Herpes Simplex Virus thymidine kinase (TK) promoter; Rous Sarcoma Virus long terminal repeat (RSV LTR) enhancer/promoter; elongation factor 1α (hEF-1α) promoter from human, chimpanzee, mouse or rat; the SV40 early promoter; HIV-1 long terminal repeat promoter; β-actin promoter;

Some preferred, but non-limiting vectors for use with these host cells include:

- vectors for expression in mammalian cells: pMAMneo (Clontech), pcDNA3 (Invitrogen), pMC1neo (Stratagene), pSG5 (Stratagene), EBO-pSV2-neo (ATCC 37593), pBPV-1 (8-2) (ATCC 37110), pdBPV-MMTneo (342-12) (ATCC 37224), pRSVgpt (ATCC37199), pRSVneo (ATCC37198), pSV2-dhfr (ATCC 37146), pUCTag (ATCC 37460) and 1ZD35 (ATCC 37565), as well as viral-based expression systems, such as those based on adenovirus;
- vectors for expression in bacterial cells: pET vectors (Novagen) and pQE vectors (Qiagen);
- vectors for expression in yeast or other fungal cells: pYES2 (Invitrogen) and *Pichia* expression vectors (Invitrogen);
- vectors for expression in insect cells: pBlueBacII (Invitrogen) and other baculovirus vectors
- vectors for expression in plants or plant cells: for example vectors based on cauliflower mosaic virus or tobacco mosaic virus, suitable strains of *Agrobacterium,* or Ti-plasmid based vectors.

Some preferred, but non-limiting secretory sequences for use with these host cells include:

- for use in bacterial cells such as *E. coli*: PelB, Bla, OmpA, OmpC, OmpF, OmpT, StII, PhoA, PhoE, MalE, Lpp, LamB, and the like; TAT signal peptide, hemolysin C-terminal secretion signal;
- for use in yeast: α-mating factor prepro-sequence, phosphatase (pho1), invertase (Suc), etc.;
- for use in mammalian cells: indigenous signal in case the target protein is of eukaryotic origin; murine Ig κ-chain V-J2-C signal peptide; etc.

Suitable techniques for transforming a host or host cell of the invention will be clear to the skilled person and may depend on the intended host cell/host organism and the genetic construct to be used. Reference is again made to the handbooks and patent applications mentioned above.

After transformation, a step for detecting and selecting those host cells or host organisms that have been successfully transformed with the nucleotide sequence/genetic construct of the invention may be performed. This may for instance be a selection step based on a selectable marker present in the genetic construct of the invention or a step involving the detection of the amino acid sequence of the invention, e.g. using specific antibodies.

The transformed host cell (which may be in the form or a stable cell line) or host organisms (which may be in the form of a stable mutant line or strain) form further aspects of the present invention.

Preferably, these host cells or host organisms are such that they express, or are (at least) capable of expressing (e.g. under suitable conditions), an amino acid sequence, Nanobody or polypeptide of the invention (and in case of a host organism: in at least one cell, part, tissue or organ thereof). The invention also includes further generations, progeny and/or offspring of the host cell or host organism of the invention, that may for instance be obtained by cell division or by sexual or asexual reproduction.

To produce/obtain expression of the amino acid sequences of the invention, the transformed host cell or transformed host organism may generally be kept, maintained and/or cultured under conditions such that the (desired) amino acid sequence, Nanobody or polypeptide of the invention is expressed/produced. Suitable conditions will be clear to the skilled person and will usually depend upon the host cell/host organism used, as well as on the regulatory elements that control the expression of the (relevant) nucleotide sequence of the invention. Again, reference is made to the handbooks and patent applications mentioned above in the paragraphs on the genetic constructs of the invention.

Generally, suitable conditions may include the use of a suitable medium, the presence of a suitable source of food and/or suitable nutrients, the use of a suitable temperature, and optionally the presence of a suitable inducing factor or compound (e.g. when the nucleotide sequences of the invention are under the control of an inducible promoter); all of which may be selected by the skilled person. Again, under such conditions, the amino acid sequences of the invention may be expressed in a constitutive manner, in a transient manner, or only when suitably induced.

It will also be clear to the skilled person that the amino acid sequence, Nanobody or polypeptide of the invention may (first) be generated in an immature form (as mentioned above), which may then be subjected to post-translational modification, depending on the host cell/host organism used. Also, the amino acid sequence, Nanobody or polypeptide of the invention may be glycosylated, again depending on the host cell/host organism used.

The amino acid sequence, Nanobody or polypeptide of the invention may then be isolated from the host cell/host organism and/or from the medium in which said host cell or host organism was cultivated, using protein isolation and/or purification techniques known per se, such as (preparative) chromatography and/or electrophoresis techniques, differential precipitation techniques, affinity techniques (e.g. using a specific, cleavable amino acid sequence fused with the amino acid sequence, Nanobody or polypeptide of the invention) and/or preparative immunological techniques (i.e. using antibodies against the amino acid sequence to be isolated).

Generally, for pharmaceutical use, the polypeptides of the invention may be formulated as a pharmaceutical preparation or compositions comprising at least one polypeptide of the invention and at least one pharmaceutically acceptable carrier, diluent or excipient and/or adjuvant, and optionally one or more further pharmaceutically active polypeptides and/or compounds. By means of non-limiting examples, such a formulation may be in a form suitable for oral administration, for parenteral administration (such as by intravenous, intramuscular or subcutaneous injection or intravenous infusion), for topical administration, for administration by inhalation, by a skin patch, by an implant, by a suppository, etc.. Such suitable administration forms - which may be solid, semi-solid or liquid, depending on the manner of administration - as well as methods and carriers for use in the preparation thereof, will be clear to the skilled person, and are further described herein.

Thus, in a further aspect, the invention relates to a pharmaceutical composition that contains at least one amino acid of the invention, at least one Nanobody of the invention or at least one polypeptide of the invention and at least one suitable carrier, diluent or excipient (i.e. suitable for pharmaceutical use), and optionally one or more further active substances.

Generally, the amino acid sequences, Nanobodies and polypeptides of the invention can be formulated and administered in any suitable manner known per se, for which reference is for example made to the general background art cited above (and in particular to WO 04/041862, WO 04/041863, WO 04/041865 and WO 04/041867) as well as to the standard handbooks, such as Remington's Pharmaceutical Sciences, 18th Ed., Mack Publishing Company, USA (1990) or Remington, the Science and Practice of Pharmacy, 21 th Edition, Lippincott Williams and Wilkins (2005).

For example, the amino acid sequences, Nanobodies and polypeptides of the invention may be formulated and administered in any manner known per se for conventional antibodies and antibody fragments (including ScFv's and diabodies) and other pharmaceutically active proteins. Such formulations and methods for preparing the same will be clear to the skilled person, and for example include preparations suitable for parenteral administration (for example intravenous, intraperitoneal, subcutaneous, intramuscular, intraluminal, intra-arterial or intrathecal administration) or for topical (i.e. transdermal or intradermal) administration.

Preparations for parenteral administration may for example be sterile solutions, suspensions, dispersions or emulsions that are suitable for infusion or injection. Suitable carriers or diluents for such preparations for example include, without limitation, sterile water and aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

The amino acid sequences, Nanobodies and polypeptides of the invention can also be administered using gene therapy methods of delivery. See, *e.g.* U.S. Patent No. 5,399,346, which is incorporated by reference in its entirety. Using a gene therapy method of delivery, primary cells transfected with the gene encoding an amino acid sequence, Nanobody or polypeptide of the invention can additionally be transfected with tissue specific promoters to target specific organs, tissue, grafts, tumours, or cells and can additionally be transfected with signal and stabilization sequences for subcellularly localized expression.

Thus, the amino acid sequences, Nanobodies and polypeptides of the invention may be systemically administered, e.g., orally, in combination with a pharmaceutically acceptable vehicle such as an inert diluent or a carrier. They may be enclosed in hard or soft shell gelatin capsules, may be compressed into tablets, or may be incorporated directly with the food of the patient's diet. For oral therapeutic administration, the amino acid sequences, Nanobodies and polypeptides of the invention may be combined with one or more excipients and used in the form of ingestible tablets, buccal tablets, troches, capsules, elixirs, suspensions, syrups, wafers, and the like. Such compositions and preparations should contain at least 0.1% of the amino acid sequence, Nanobody or polypeptide of the invention. Their percentage in the compositions and preparations may, of course, be varied and may conveniently be between about 2 to about 60% of the weight of a given unit dosage form. The amount of the amino acid sequence, Nanobody or polypeptide of the invention in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the amino acid sequences, Nanobodies and polypeptides of the invention, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the amino acid sequences, Nanobodies and polypeptides of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The amino acid sequences, Nanobodies and polypeptides of the invention may also be administered intravenously or intraperitoneally by infusion or injection. Solutions of the amino acid sequences, Nanobodies and polypeptides of the invention or their salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of micro-organisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the amino acid sequences, Nanobodies and polypeptides of the invention in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the amino acid sequences, Nanobodies and polypeptides of the invention may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, hydroxyalkyls or glycols or water-alcohol/glycol blends, in which the amino acid sequences, Nanobodies and polypeptides of the invention can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the amino acid sequences, Nanobodies and polypeptides of the invention to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the amino acid sequences, Nanobodies and polypeptides of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the amino acid sequences, Nanobodies and polypeptides of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the amino acid sequences, Nanobodies and polypeptides of the invention required for use in treatment will vary not only with the particular amino acid sequence, Nanobody or polypeptide selected but also with the route of administration, the nature of the condition being treated and the age and condition of the patient and will be ultimately at the discretion of the attendant physician or clinician. Also the dosage of the amino acid sequences, Nanobodies and polypeptides of the invention varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The stabilized polypeptides according to the invention can be used advantageously in all areas of application for example in the therapeutics of cancer and infections, as an immunotoxin, for drug-targeting and in gene therapy. A use in imaging and in diagnostics is equally advantageous for example in order to analyse antigen-binding substances.

The process according to the invention is particularly advantageous for stabilizing single variable domains which have already been modified for other reasons such as for example humanized or chimeric single variable domains. This modification of the amino acids can result in a destabilization and the process according to the invention can restore or even improve the original stability of the single variable domains by an additional modification of these single variable domains outside the CDR regions.

The invention assumes that the naturally occurring immunoglobulin sequences are a canonical collection of sequences whose sum total should be compatible for all aspects of single variable domain functions.

The invention is described in more detail by the following experimental part, figures, tables and the sequence protocol.

DESCRIPTION OF THE FIGURES

**Figure 1:** Magnification of an RP-HPLC chromatogram of SEQ ID NO: 1 to illustrate the product-related substances that show up as pre- and post-peaks (i.e. that elute before and after the main peak). The nomenclature used in labeling of peaks present in SEQ ID NO: 1 master reference standard, BATCH 1, is indicated.

**Figure 2:** Relevant part of the RP-HPLC chromatograms of SEQ ID NO: 1 batch 2 after 0, 1 and 2 months storage at -70°C, +5°C and +25°C.

**Figure 3:** Relevant part of the RP-HPLC chromatograms of SEQ ID NO: 1 BATCH 1 after 0, 4 and 8 weeks incubation at 37°C.

**Figure 4:** Amino acid sequence and ordinal numbering of SEQ ID NO: 2, the monovalent building block of the bivalent Nanobody® SEQ ID NO: 1. SEQ ID NO: 1 is a single polypeptide chain which consists of two identical copies of the SEQ ID NO: 2 immunoglobulin domain that are fused head-to-tail with in between a three alanine residues linker (259 residues in total). The CDR regions are underlined. The residues that are discussed in the present report are highlighted; these residues are numbered according to their position on the monovalent SEQ ID NO: 2, i.e.: E1, M34, M78, M83, D62/S63, D105/G106, and N84/S85. The reader will realize that, in the context of SEQ ID NO: 1, these designations refer to two equivalent positions, one in each of the two identical domains and that, for example, M34 also refers to the methionine residue present at position 165 in SEQ ID NO:1. Also it is pointed out that this numbering does not correspond to the KABAT numbering system referred to elsewhere also in this application.

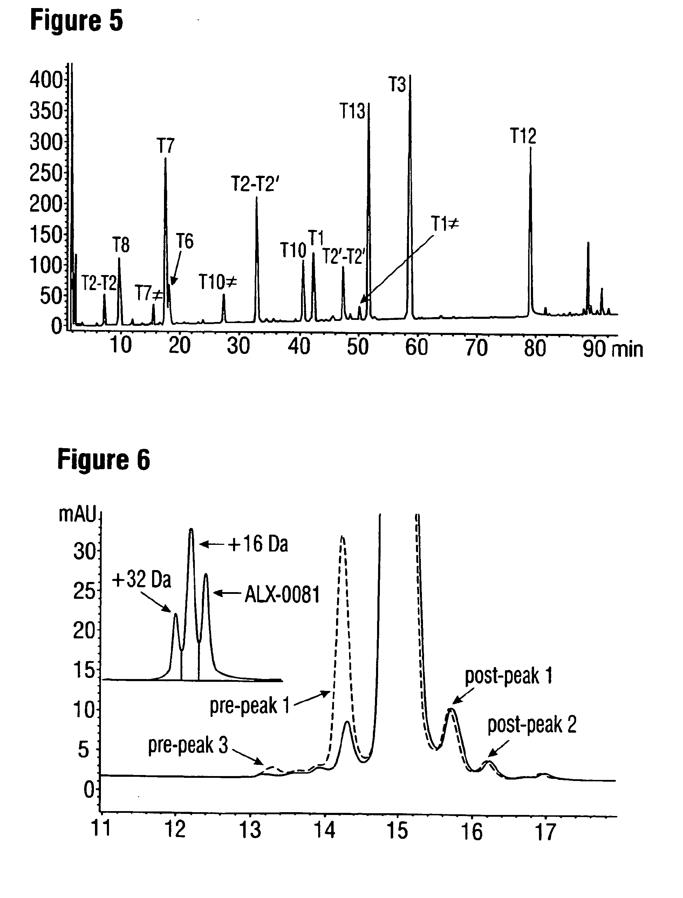

**Figure 5:** RP-HPLC chromatogram, recorded at 214nm, of a tryptic digest of SEQ ID NO:1 BATCH 1 at time zero (blue chromatogram) and after incubation at 37°C for 8 weeks (red chromatogram). The various peaks that have been characterized to date are labeled; their identity is shown in Table B-3. The identity of peaks that are not labeled remains to be determined. The peaks that elute later than T12 are probably partial digestion products; intact SEQ ID NO: 1 also elutes at around 90 min.

**Figure 6:** Comparison of the relevant part of the RPC chromatograms of deliberately oxidized (dashed line) and reference (full line; BATCH 1) SEQ ID NO:1. The insert shows the profile after continued forced oxidation; clearly, not only mono-oxidized (+16Da) but also double oxidized (+32Da) SEQ ID NO: 1 is formed in the course of the treatment with $H_2O_2$.

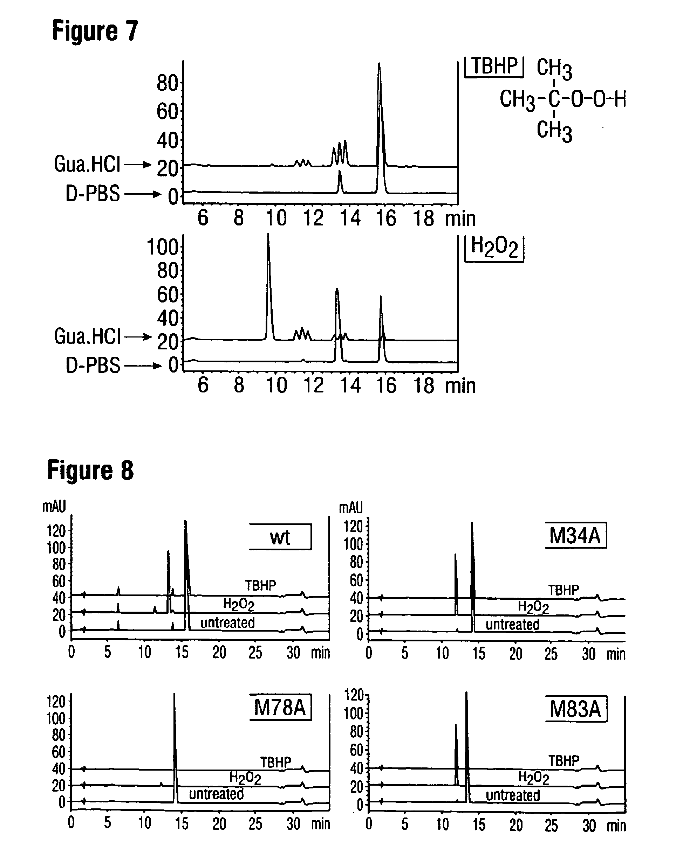

**Figure 7:** Deliberate oxidation of unfolded and native SEQ ID NO:2 with hydrogen peroxide ($H_2O_2$, lower panel) and tertiary butyl hydroxyperoxide (TBHP, upper panel). In the presence of 4M guanidinium hydrochloride (Gua.HCl), SEQ ID NO:1 oxidizes readily when treated with TBHP and even more so with hydrogen peroxide. In addition to the non-oxidized material (i.e. the rightmost peak) seven peaks can be discerned in the RPC chromatograms: an MS analysis (data not shown) indicated that these represent the three possible mono-oxidized forms (+16Da), the three different doubly oxidized variants (+32Da), and finally, most leftward, the triple oxidized species (+48Da). The profiles obtained when the oxidations are performed on SEQ ID NO:2 in D-PBS clearly indicate that in essence only a single methionine residue is susceptible in the native SEQ ID NO:2 molecule. The minor peaks may be taken as evidence that a second methionine residue is somewhat sensitive to $H_2O_2$-treatment. The RPC profile of the $H_2O_2$-treated M78A mutant in Figure 8 confirms this notion. The various mixtures were subjected to SEC (NAP5 desalting column, GE Healthcare) prior to RPC analysis.

**Figure 8:** Deliberate oxidation of SEQ ID NO:2 wild type (wt) and the three different M->A mutants. In each case, the RPC profile of the untreated variant (blue) is compared with the chromatograms obtained after oxidation with either tertiary butyl hydroxyperoxide (TBHP; green) or hydrogen peroxide ($H_2O_2$; red). Note that the batch of wt SEQ ID NO:2 used in the present experiment seems to contain a contaminant (compare with Figure 7). The various mixtures were subjected to SEC (NAP5 desalting column, GE Healthcare) prior to RPC analysis.

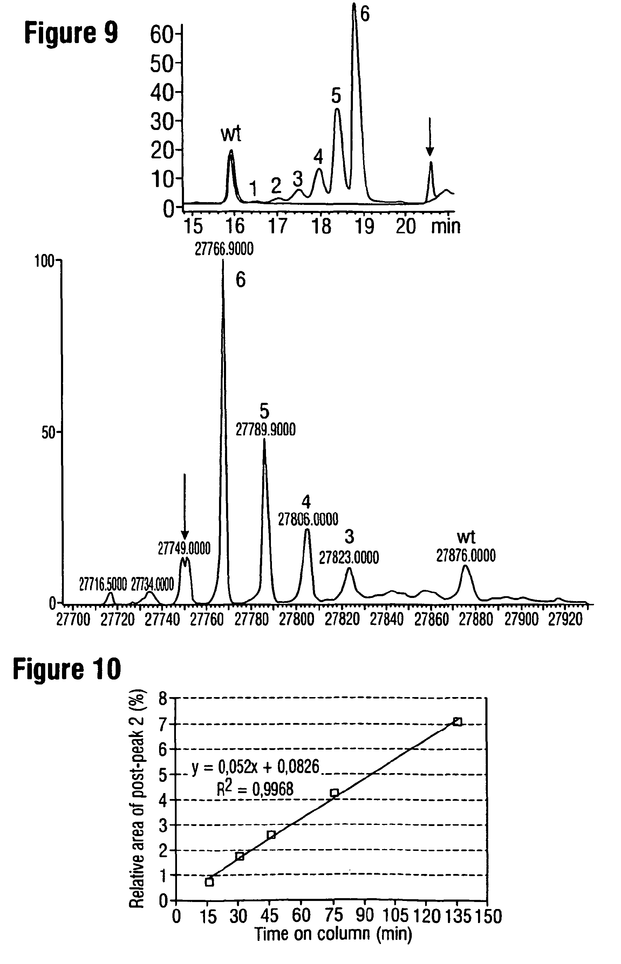

**Figure 9:** RPC (left panel) and MS (right panel) analysis of an SEQ ID NO:1 variant with global replacement of all methionines by norleucine residues. The left panel shows an overlay of the RPC profiles of wt SEQ ID NO:1 (2.5μg BATCH 1) and the norleucine variant (25μg). Apparently, the preparation of the norleucine variant includes all possible partially substituted variants as well as some wt SEQ ID NO:1 (see text for details; the various species are labeled according to the number of norleucines they incorporate). The following molecular masses were experimentally determined: 27876 (wt, predicted mass of 27876); 27823.5 (3, the predicted mass of a variant with 3 norleucines is 27822); 27805 (4, predicted mass 27804); 27786.5 (5, predicted mass 27786); 27768 (6, predicted mass 27768).

**Figure 10:** Correlation between the area of post-peak 2 and the residence time on the RPC column. The first time point corresponds to the residence/retention time. For the other experiments the start of the gradient was delayed by 15min, 30min, 60 min or 120 min.

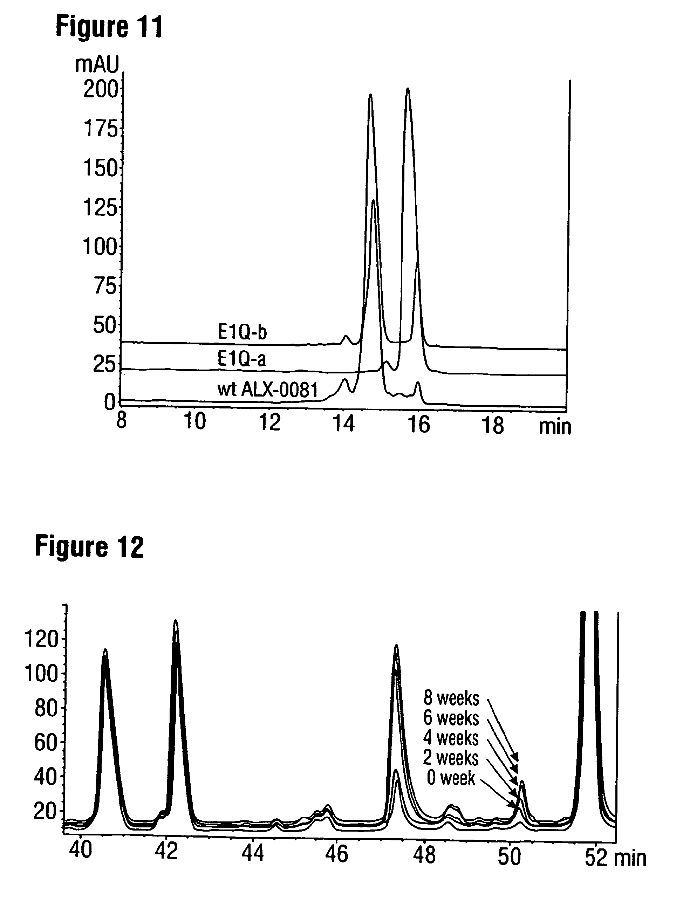

**Figure 11:** RPC profiles of BATCH 1 after 4 weeks of storage at 37°C, the E1Q-a variant, and the E1Q-b variant of SEQ ID NO:1.

**Figure 12:** Part of the peptide map of SEQ ID NO:1 stored at 37°C. BATCH 1 was stored at 37°C during 2, 4, 6 or 8 weeks before trypsin digestion and RPC analysis; the blue profile represents the time zero control. RP-HPLC was performed at 70°C using a Zorbax 300SB-C3 column and a gradient from 5% to 36.7% ACN at 0.33% per minute.

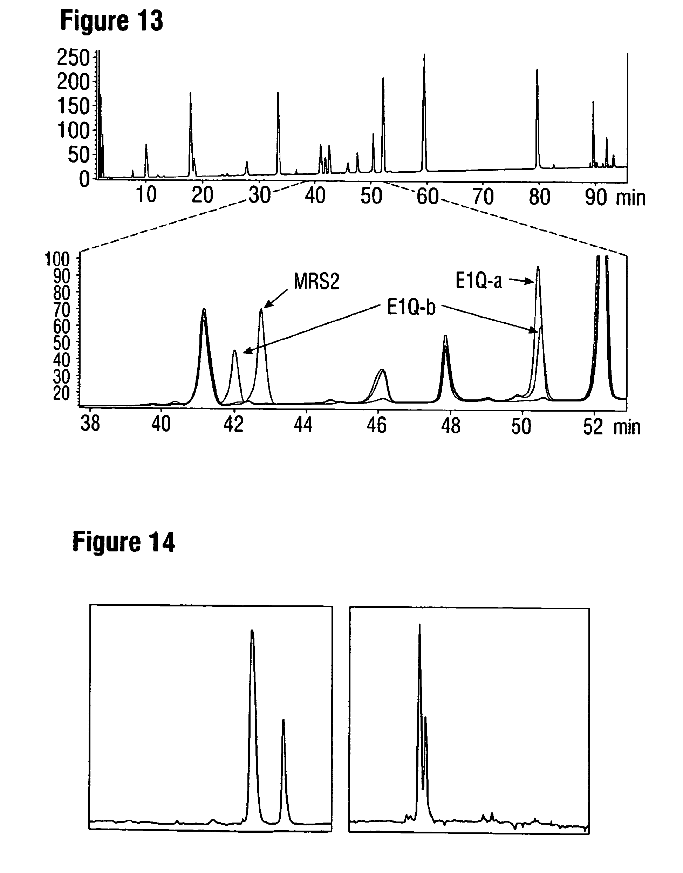

**Figure 13:** Peptide mapping of BATCH 1 and the SEQ ID NO:1 E1Q-a and E1Q-b variants. The proteins were digested with trypsin (1/50 w/w ratio) during 24hrs at 37°C; 25 μg of each digested protein was applied onto the column. The RPC conditions were the same as for the experiment in Figure 12.

**Figure 14:** Identification of the pyroglutamate modification by cIEF. An enlargement of the relevant part of the electropherograms is shown to make the pI differences more obvious. Left panel: cIEF analysis of a mixture of BATCH 1 and the E1Q-a variant (the injected sample contains Batch 1 at 0.23mg/mL and E1Q-a at 0.09mg/mL). Right panel: cIEF analysis of a mixture of BATCH 1 and the E1Q-b variant (injected sample contains batch 1 at 0.23mg/mL and E1Q-b at 0.15mg/mL). The samples were desalted with Zeba desalt spin columns (Pierce) prior to analysis on the ICE280 isoelectric focuser.

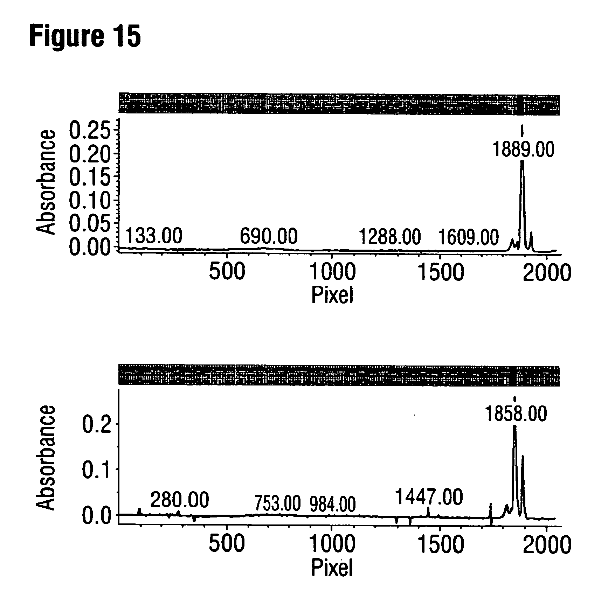

**Figure 15:** Identification of the pyroglutamate modification in stability samples by cIEF. Upper panel: electropherogram of a sample containing BATCH 1 stored at 37°C for 6 weeks. Lower panel: electropherogram of a mixture of the same BATCH 1 stability study sample and the E1Q-a variant (batch 1 at 0.42mg/mL and E1Q-a at 0.09mg/mL). The samples were desalted with Zeba desalt spin columns (Pierce) prior to analysis on the ICE280 isoelectric focuser.

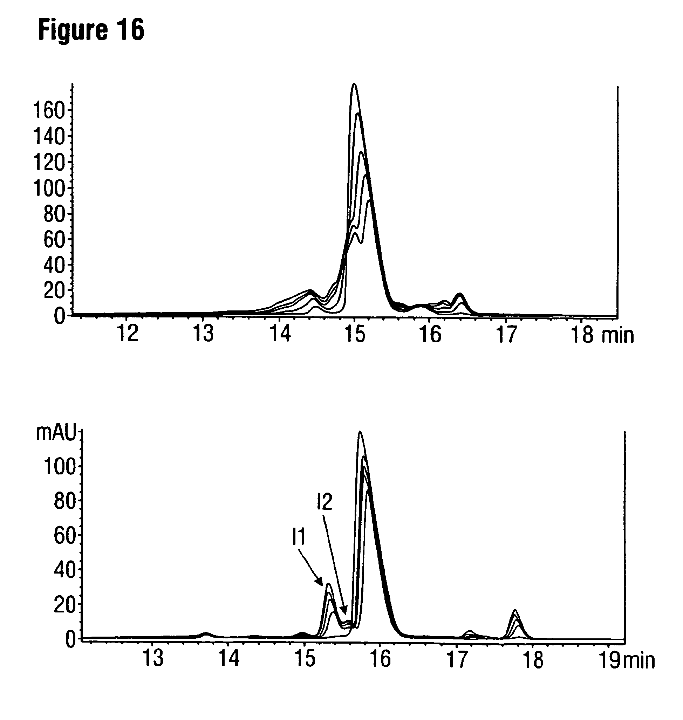

**Figure 16:** RP-HPLC profiles of SEQ ID NO: 1 Batch 1 (upper panel) and SEQ ID NO: 2 (lower panel) versus storage time at 37°C. Analysis was performed on the material as such (control, curve with highest main peak), as well as after 2 weeks (curve with second highest main peak), 4 weeks(curve with third main highest peak), 6 weeks(curve with forth highest main peak) and 8 weeks (curve with lowest main peak) of storage at 37°C. Samples were chromatographed on a C3 column kept at 70°C and the absorbance was measured at 280nm. The RPC profiles on SEQ ID NO:2 reveal two new earlier eluting molecular species, referred to as I1 and I2.

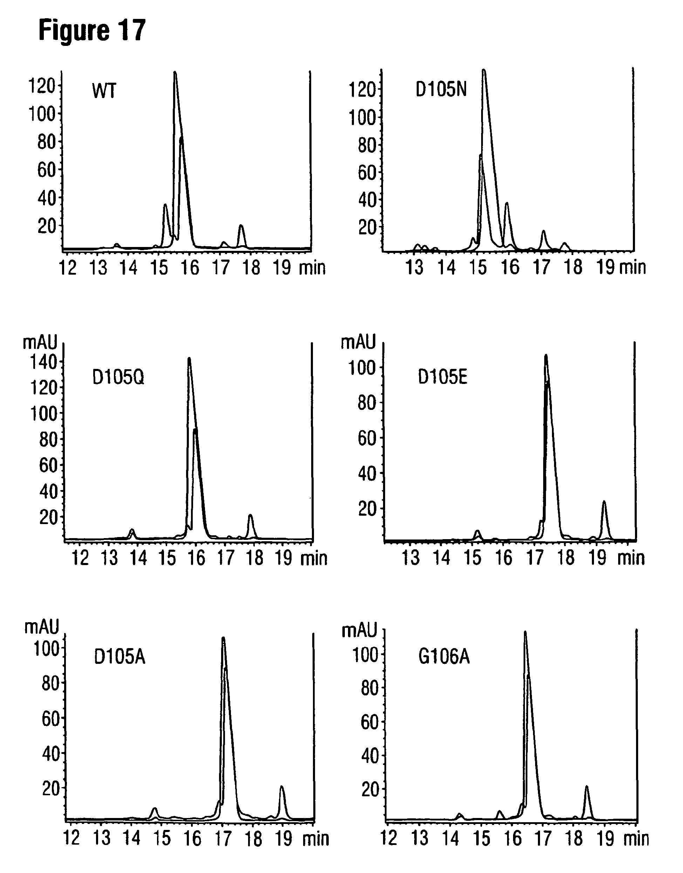

**Figure 17:** Comparison of the RPC profiles of wt SEQ ID NO:2 and its mutant forms D105A, D105E, D105Q, D105N, and G106A at time zero (blue line/ upper trace) and after 8 weeks storage at 37°C (red line/lower trace).

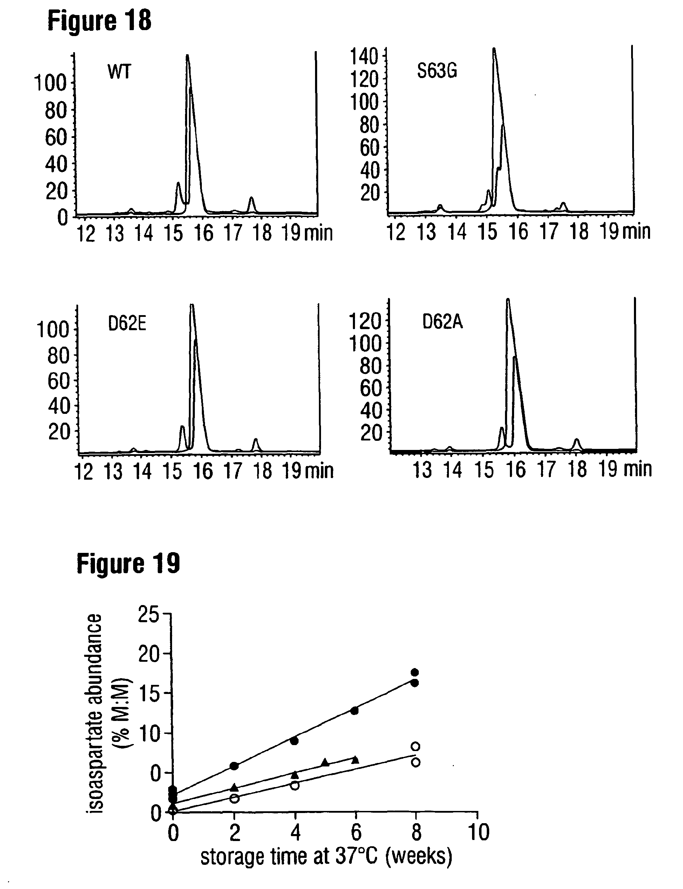

**Figure 18:** Comparison of the RPC profiles of wild type SEQ ID NO:2 (WT) and its mutant forms D62A, D62E, and S63G at time zero (red trace/ upper trace) and after 4 weeks of storage at 37°C (blue line / lower trace).

**Figure 19:** Formation of isoaspartate in SEQ ID NO: 1 and SEQ ID NO: 2 stored at 37°C. Isoaspartate was measured on intact SEQ ID NO: 1 (open circles), on a trypsin digested SEQ ID NO:1 (closed circles) or on trypsin digested SEQ ID NO:2 (triangles) after various incubation periods. The level of isoaspartic acid measured in the intact protein was markedly lower, indicating that a digestion was required to minimize the effect of protein structure on the detection of isoaspartic acid residues. The isoaspartate abundance in the protein is expressed in percent (%M:M; mole isoaspartate:mole Nanobody®). For certain samples, two or three independent determinations of the isoaspartate content were performed.

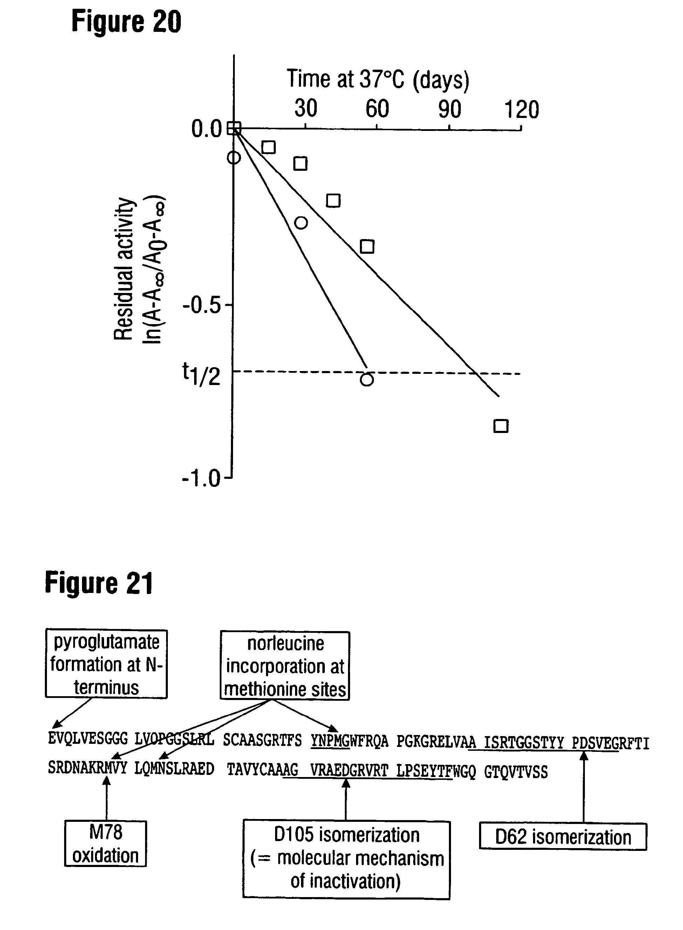

**Figure 20:** Loss of activity during storage at 37°C for SEQ ID NO: 1 (circles) and its monovalent counterpart SEQ ID NO: 2 (squares). The data were fitted to a pseudofirst-order kinetic model (see eq. 2 in the text) to deduce an apparent rate constant for the isomerization process which appears to be the molecular mechanism of inactivation. The half-life is ~58 days and ~100 days for SEQ ID NO: 1 and SEQ ID NO:2, respectively.

**Figure 21:** Major product related variants of SEQ ID NO: 1 that are present at the time of manufacturing or that arise during storage. The amino acid sequence of SEQ ID NO: 2, the monovalent building block of the bivalent Nanobody® SEQ ID NO:1, is shown. The CDR regions are underlined. The residues that are discussed in the present report are shown in red, i.e.: E1, M34, M78, M83, D62/S63, D105/G106 (using numbering according to figure 4). Except for E1, these designations refer to two equivalent positions, one in each of the two identical domains of SEQ ID NO: 1 .

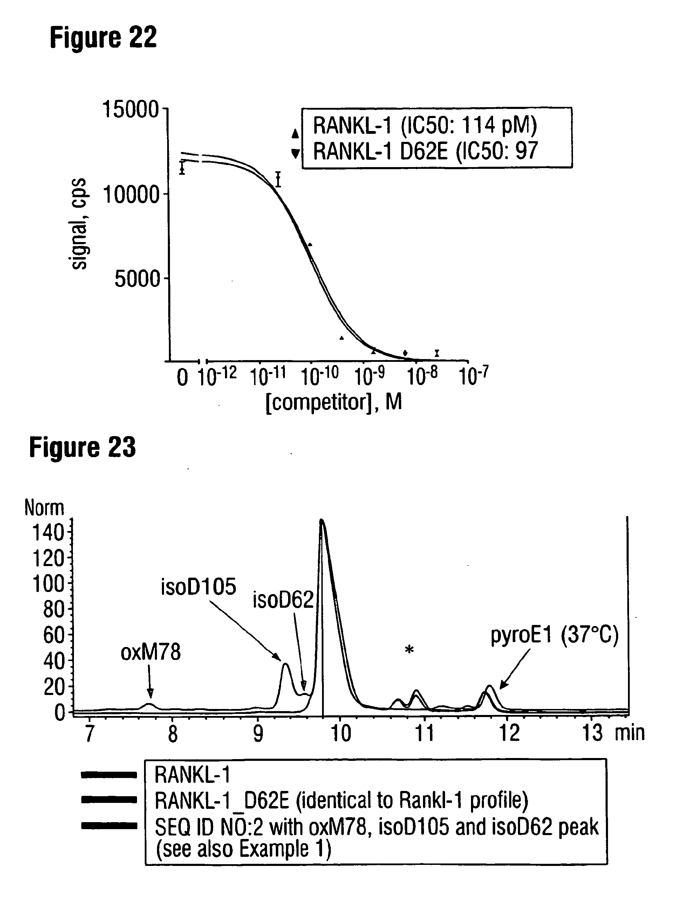

**Figure 22:** RANKL-1 and RANKL-1_D62E display similar potencies as measured in AlphaScreen assay (AlphaScreen™ Technology available e.g. from Perkin Elmer, UK).

**Figure 23:** RPC analysis of RANKL-1 + RANKL-1_D62E (lines more or less identical). The SEQ ID NO: 1 profile with the isoD62, isoD105, and oxM78 peaks (see also example 1) is included as a positive control. * indicates peaks also present in the start material and reference material. IsoD62 and isoD105 correspond to isomerization of Asp-105 and Asp-62 respectively. oxM78 corresponds to oxidation of Met-78. pyroE1 corresponds to formation of N-terminal cyclic pyroglutamate.

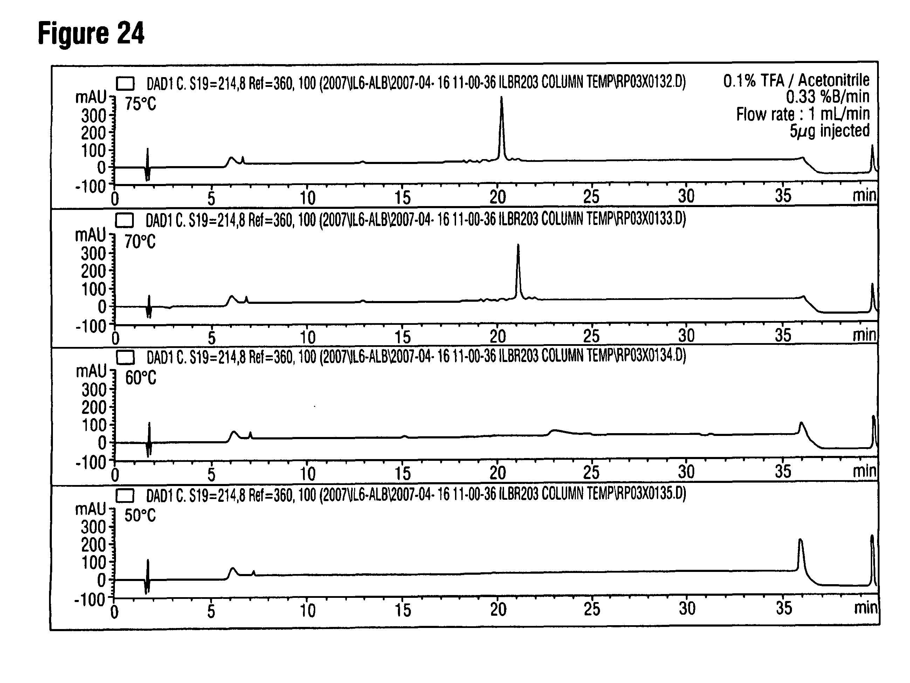

**Figure 24 :** 214 nm RP-HPLC chromatograms of analysis of IL6R203 on ZORBAX C3 at a flow rate of 1 mL/min with a gradient slope of 0.33 %B/min and using TFA as ion pairing agent, but at different column temperatures (50°C - 60°C - 70°C - 75°C). Peak elution time increases and area decreases with decreasing column temperature.

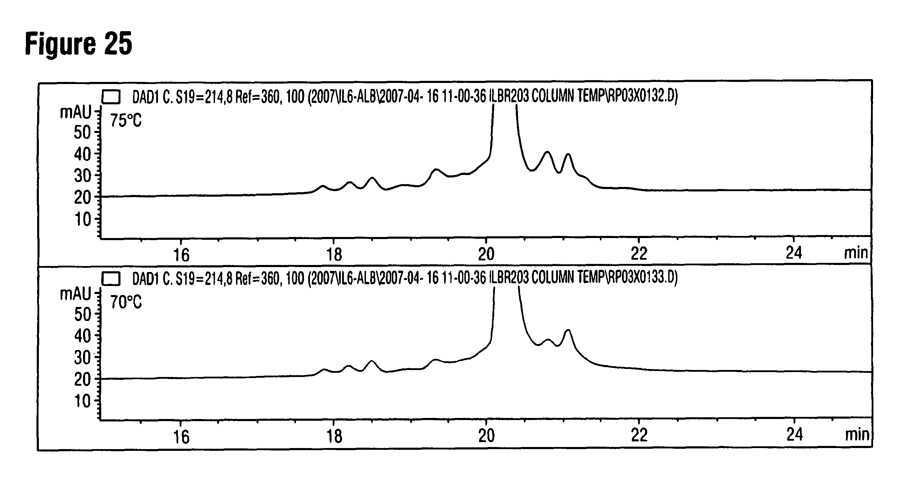

**Figure 25:** Zoom in on peak base region of samples analyzed at 75 and 70°C. The 75°C shows a considerable increase in the first post-peak. It should be noted that this may be a temperature effect.

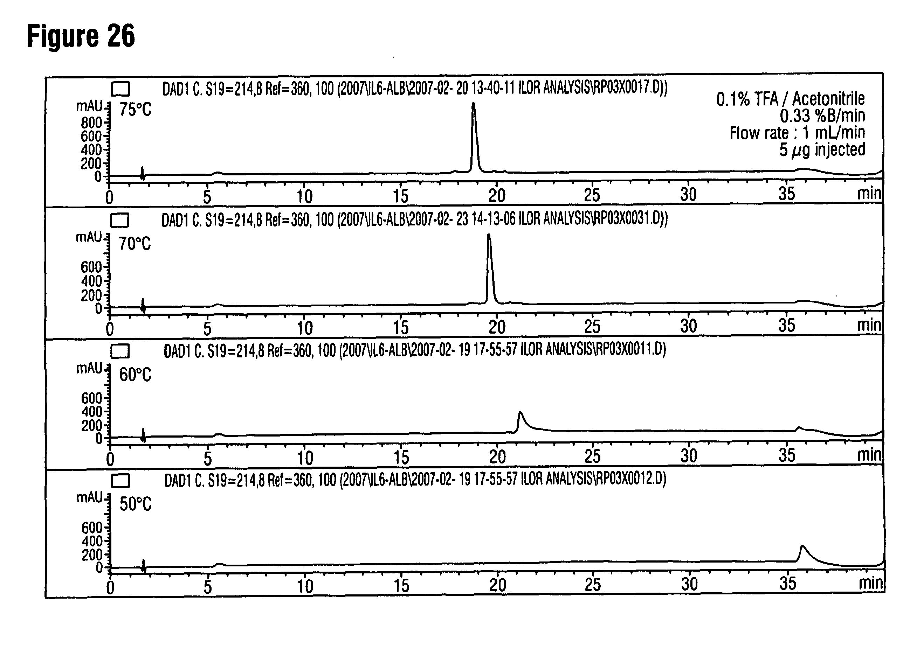

**Figure 26:** Effect of different column temperatures on RP-HPLC profile of IL6R202 is dramatic - this was also seen with IL6R203. Minimum temperature to obtain a usable chromatogram is 70°C.

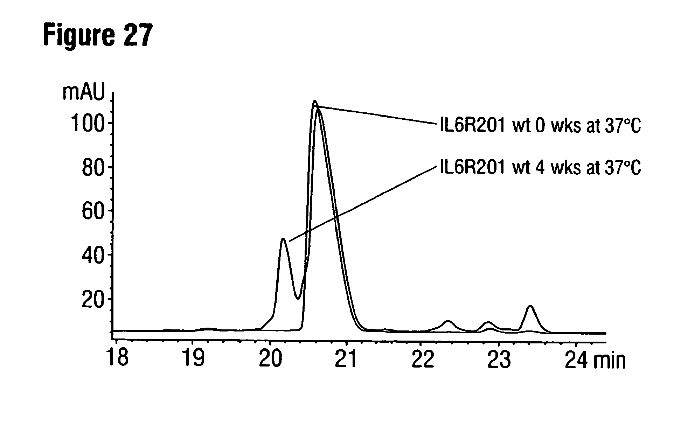

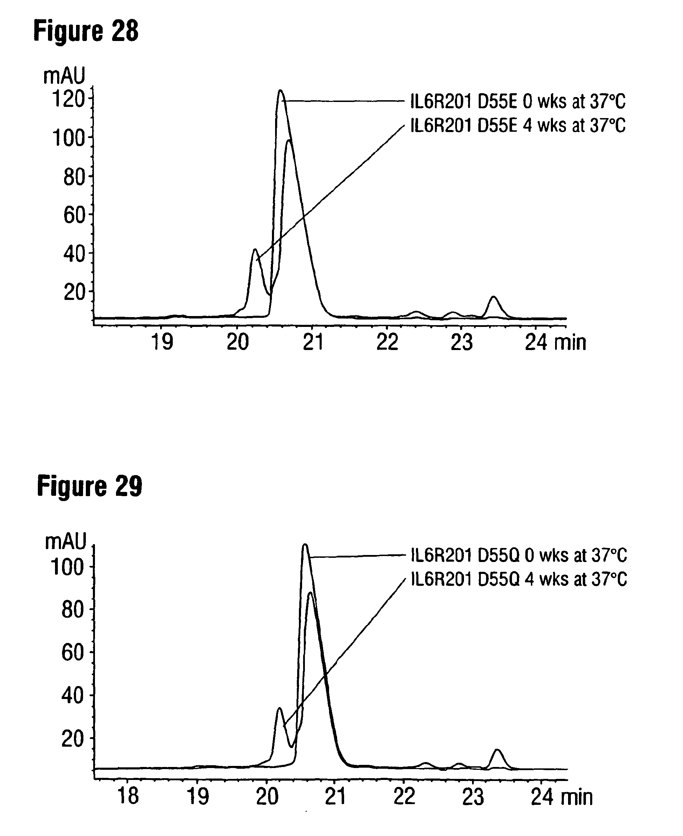

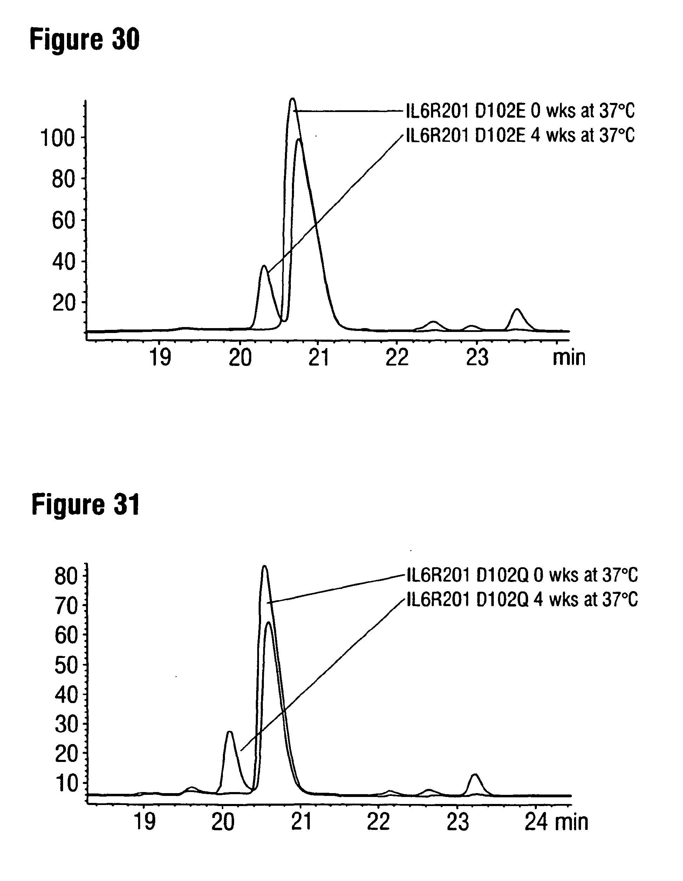

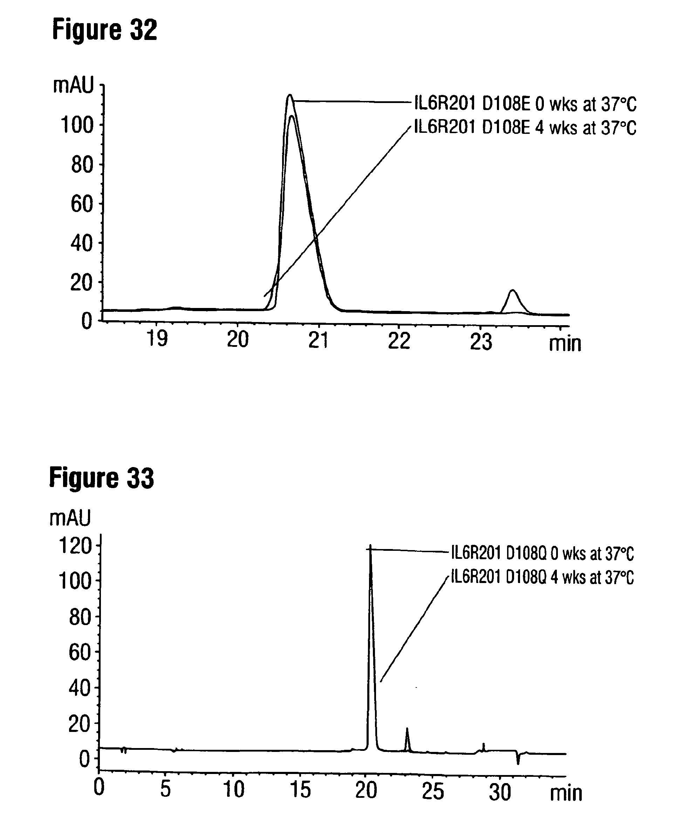

**Figures 27 to 33:** IL6R201 wildtype and mutants were analysed by RPC: The column used was a ZORBAX 300SB C3 (5 μm) column on an Agilent system. The column had a temperature of 70°C during the experiments. Buffer A used during experiments was 0.1% trifluoroacetic acid and buffer B was 0.1% trifluoroacetic acid / 99.9% acetonitril.

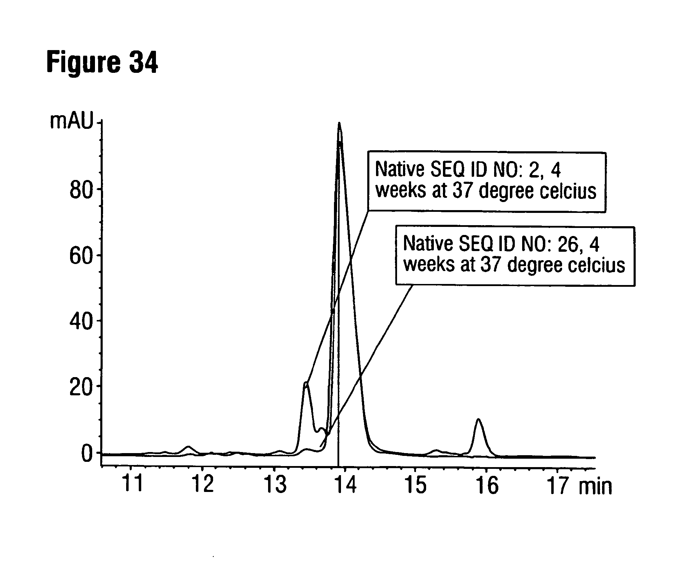

**Figure 34:** Both, SEQ ID NO: 2 and SEQ ID NO: 26, were put on storage at 37°C. After 4 weeks, samples were analyzed by RPC; an overlay of the chromatograms is shown in Figure 34.

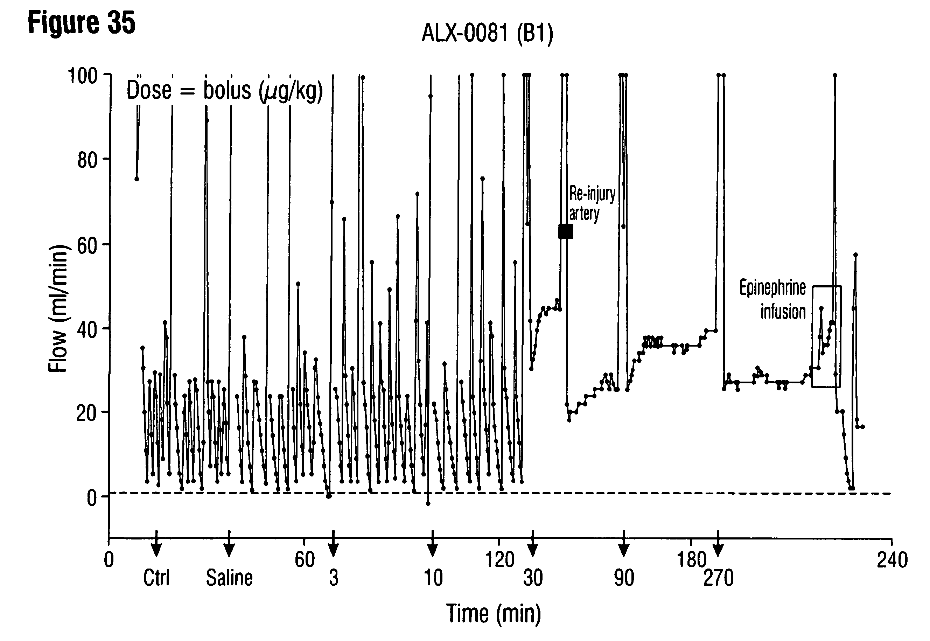

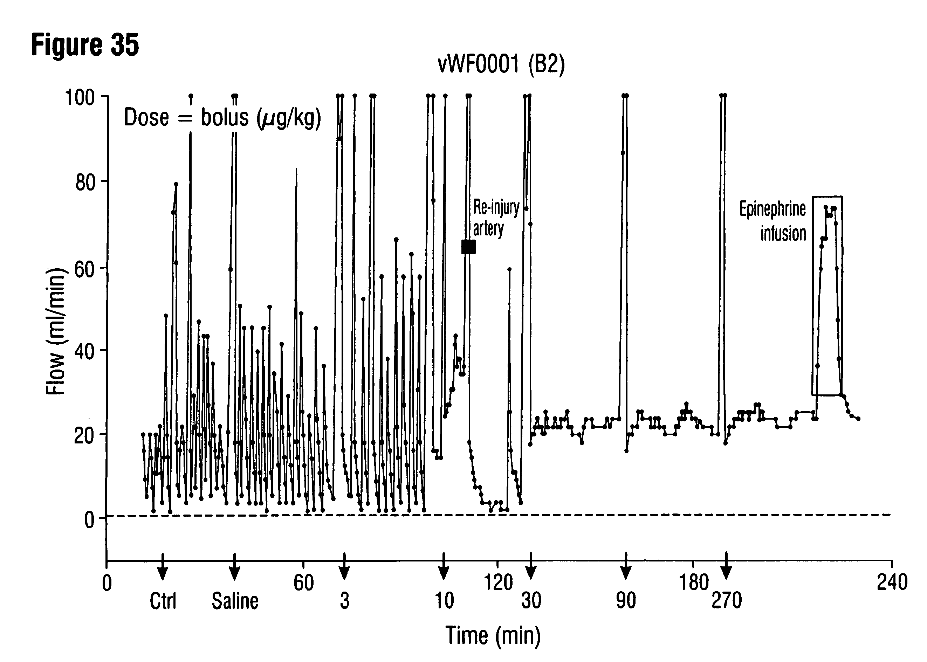

**Figure 35:** Folts' studies with ALX-0081 and VWF0001. The blood flow is shown in function of time, indicating the CFRs. CFRs were measured for 30 minutes after injury and stenosis of the femoral artery (control). Saline was injected and the effect on CFRs was measured for 30 minutes. ALX-0081 or VWF0001 was injected, increasing the dose every 30 minutes. The dosing times are indicated with a red arrow, doses are indicated in red ($\mu$g/kg).

When complete inhibition of CFRs was observed, a new injury was applied. After the highest dose of test item, Epinephrine was injected.

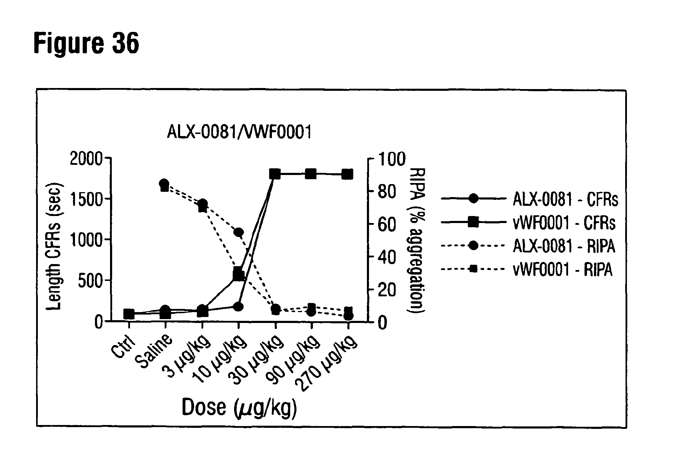

**Figure 36:** Ristocetin-induced aggregation (%) and length of CFRs (seconds) in function of all doses for the baboons treated with ALX-0081 and VWF0001.

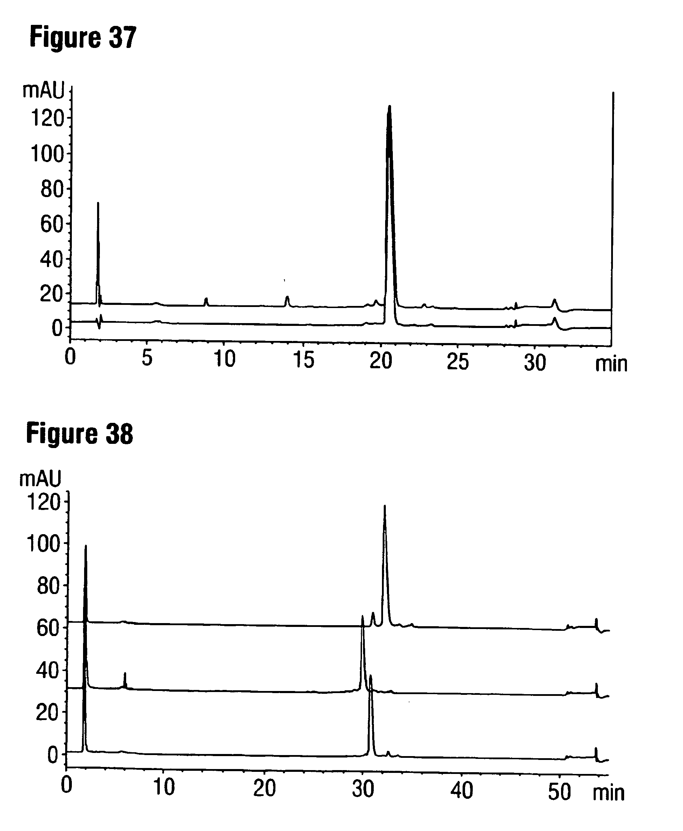

**Figure 37:** Superimposed RP-HPLC chromatograms at 280nm of forced oxidation experiment of VHH fragment IL6R201. The lower trace is the chromatogram of non-treated reference material IL6R201. The upper trace is the chromatogram of IL6R201 which was treated with 10mM $H_2O_2$ for 2 hours.

**Figure 38:** Superimposed RP-HPLC chromatograms at 280nm of forced oxidation experiment with 119A3. Upper trace: non-treated 119A3 reference sample; middle trace: 119A3 sample treated with $H_2O_2$ in the presence of 6 M guanidine; lower trace: 119A3 sample treated with $H_2O_2$.

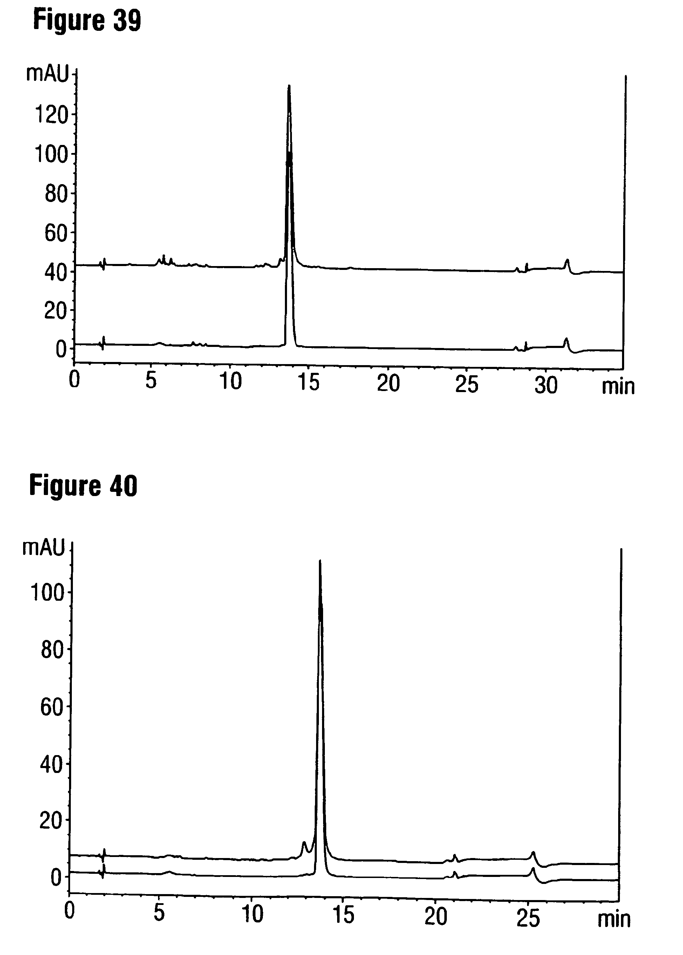

**Figure 39:** RP/HPLC /RPC chromatograms recorded at 280nm of 1 SEQ ID NO: 26 incubated for 8 weeks at 37 °C (upper trace) and reference material stored at -80 °C (lower trace).

**Figure 40:** RP/HPLC chromatograms recorded at 280nm of SEQ ID NO: 25 after incubation at 37°C for 8 weeks (upper trace) and reference material stored at -80 °C (lower trace).

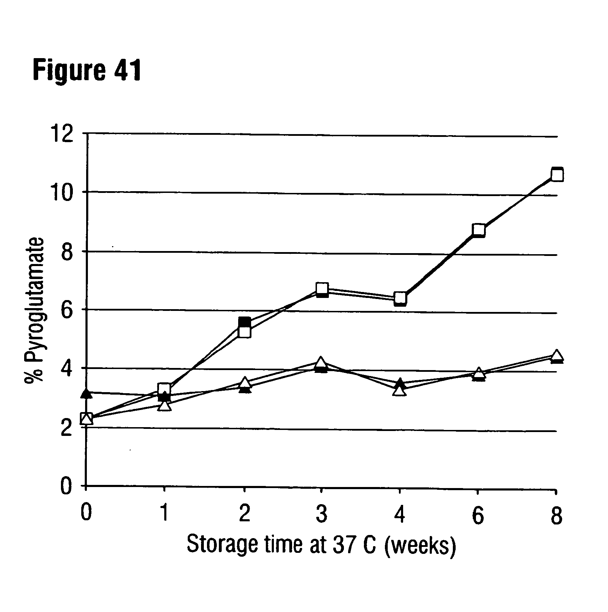

**Figure 41:** The pH-dependent formation of N-terminal cyclic pyroglutamate in RANKL-1. RANKL-1 was formulated in different buffers and stored for up to 8 weeks at 37°C. RPC analysis of the RANKL-1 storage samples in 10 mM $NaH_2PO_4$, pH 7 at 63 mg/ml (closed square), 10 mM $NaH_2PO_4$, pH 7 at 30 mg/ml (open square), Na-acetate, pH 5.5 at 63 mg/ml (closed triangle) or 10 mM Na-acetate, pH 5.5 at 30 mg/ml (open triangle) showed that the formation of the pyroglutamate variant increases with incubation time and with buffer pH. The peak surface area of the pyroglutamate, expressed as percentage of the total integrated surface area, is higher at pH 7 than at pH 5.5.

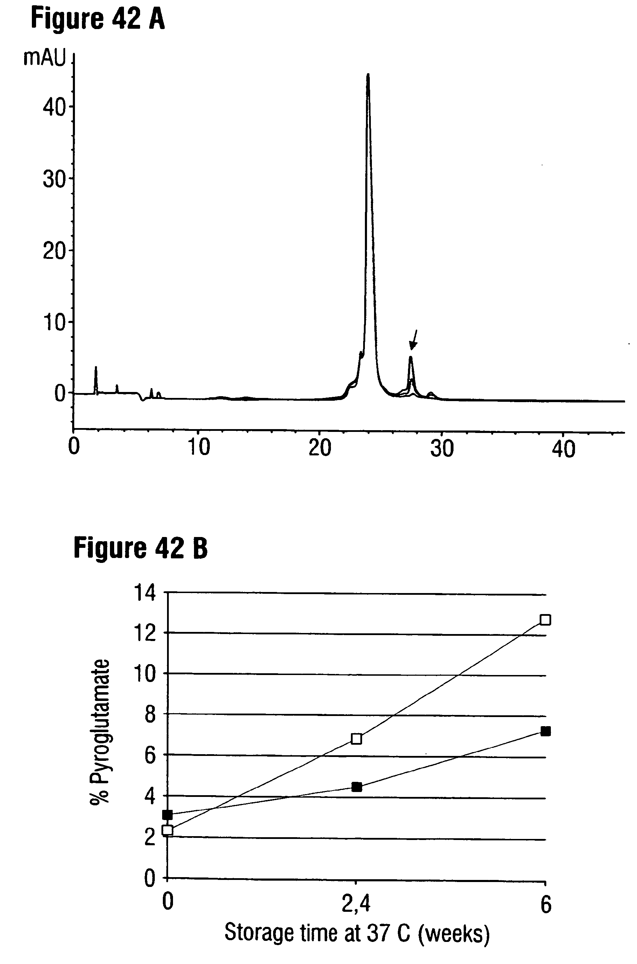

**Figure 42:** The pH-dependent formation of N-terminal cyclic pyroglutamate in nanobody 321. The stability of nanobody 321 was assessed in 2 buffers: histidine 20 mM at pH6 versus histidine 20 mM at pH 6.5, both at a protein concentration of 5 mg/mL. The samples were stressed for up to 6 weeks at 37°C. RPC analysis of the nanobody 321 storage samples showed that the formation of the pyroglutamate variant increased with incubation time and with buffer pH. Upper panel (Fig. 42 A), example of the RPC chromatograms after 6 weeks at 37°C (lower trace: -80°C reference; middle trace: pH6; upper trace: pH6.5). Lower panel (Fig 42 B), graph showing the peak surface area of the pyroglutamate, expressed as percentage of the total integrated surface area, which was higher at pH 6.5 (open square) than at pH 6 (closed square).

## EXPERIMENTAL PART

### Example 1: Stabilization of a vWF binding Nanobody and Nanobody construct (SEQ ID NO: 1: SEQ ID NO: 2)

#### 1) Chemical Characterizations of SEQ ID NO: 1

SEQ ID NO:1 is a bivalent Nanobody®; the single polypeptide chain consists of two identical copies of an immunoglobulin domain that are fused head-to-tail with in between a three alanine residues linker (see SEQ ID NO: 98 of WO2006122825 and specification for generation of SEQ ID NO: 1). One disulfide bond is present in each domain. The analytical package will assess most of the typically observed protein degradation / modification mechanisms, more specifically hydrolysis, oxidation, deamidation, disulfide bond modification, and aggregation / precipitation. Note that certain modifications, such as dephosphorylation and deglycosylation are not applicable since these post-translational modifications do not occur.

**Table B-1:** Test results obtained with particular methods for various batches of SEQ ID NO: 1.

| Tests | | Testing results | | | | |
|---|---|---|---|---|---|---|
| | | Batch 1 | Batch 2 | Batch 3 | Batch 4 | Batch 5 |
| **Biacore potency** | % of Batch 1 | 100 | 95 | 95 | 94 | 96 |
| **cIEF (capillary isoelectric focusing)** | Main peak | 97.8 | 97.7 | 98.1 | 98.1 | 97.6 |
| **SEC (size exclusion chromatography)** | Main peak | 99.6 | 99.9 | 99.5 | 99.6 | 99.5 |
| **RPC** | Main peak | 93.6 | 93.8 | 93.4 | 92.8 | 93.1 |
| | Pre peaks 1+2+3 | 1.9 | 1.8 | 2.1 | 2.6 | 2.3 |
| | Post peak 1 | 3.7 | 3.5 | 3.6 | | 3.6 |
| | Other post peaks | 0.8 | 0.9 | 0.9 | 4.6 | 0.9 (#) |
| All the values shown in the table are percentages. (#) For Batch 5, the % area of post peak 2 is reported separately; the % area of other peaks for this sample is 0%. | | | | | | |

In general, the analytical methods like cIEF, SEC, RPC and surface plasmon resonance revealed a high degree of consistency among the various batches of SEQ ID NO: 1. This is illustrated in Table B-1 which shows the results of the most relevant product specific analysis methods. Reversed Phase Chromatography (RPC) is in our hands the most informative method in that it resolves the SEQ ID NO:1 drug substance (DS) into a number of different species (refer to Figure 1). Alternatively, HIC (hydrophobic interaction chromatography) may also be a suitable method to analyze the of diversity in the DS. In addition to the main peak, pre-peaks (substance eluting before the main material) and a number of post-peaks can be discerned. In the batches produced so far, the pre-peaks and post-peak 1 consistently represent about 2% and 3.6% respectively, whereas the other post-peaks account for <1 % of the DS.

A characterization was previously performed on RPC pre- and post-peak 1. The pre-peak was shown by ESI mass spectrometry to be 16Da heavier than the intended material; this, in combination with forced oxidation experiments, consisting of a treatment with $H_2O_2$, suggested that the pre-peak results from oxidation. It was hypothesized that this oxidation occurs at one or more methionine residues (note that SEQ ID NO: 1 contains three methionine residues in each of the two identical domains). It was furthermore demonstrated that the extent of forced oxidation (within certain limits) has no effect on the bio-activity. Mass spectrometry and amino acid analyses have demonstrated that post-peak 1 is 18Da lighter than the main material and that this is the result of the mis-incorporation of a norleucine residue at a methionine site. Recovery of post-peak 1 material after RPC suggested that this product related substance is also functional. The experimental work revealed that the oxidized and the norleucine product related substances can both be present in the culture medium at the time of harvest and their levels are reduced to some extent during downstream processing.

The stability studies on the various batches of SEQ ID NO: 1 indicate that the relative abundance of certain product related substances increases with time and temperature. Figure 2 clearly shows this is the case for the pre-peaks as well as post-peak 2. In contrast, and quite surprisingly, the relative peak area of post-peak 1 is apparently not affected by either incubation time or temperature. Additionally, the RPC main peak observed with SEQ ID NO: 1 appears to divide into several different species upon prolonged incubation, especially at elevated temperatures (≥25°C; see Figure 3). The data indicate that some earlier eluting new molecular species are generated during storage.

The following single letter codes for amino acids are used: A, alanine; D, aspartic acid; E, glutamic acid; G, glycine; K, lysine; M, methionine; N, asparagine; Q, glutamine; R, arginine; S, serine; T, threonine.

Several different preparations/samples of SEQ ID NO: 1 were used for the various experiments discussed in the present report, including e.g. SEQ ID NO: 1 Batch 1 and other see Table B-1. The bivalent SEQ ID NO: 1 Nanobody® consists of two identical copies of an immunoglobulin domain that are fused head-to-tail with in between a three alanine linker. The analysis of the monovalent building block, referred to as SEQ ID NO:2 (see SEQ ID NO: 90 of WO2006122825 and specification for generation of SEQ ID NO: 2)., turned out to be very valuable; this is to be attributed to the reduced complexity compared to SEQ ID NO: 1 and the fact that the intended molecule and its product related variants are better resolved by RP-HPLC in the case of SEQ ID NO: 2 than with the bivalent SEQ ID NO: 1. In addition, a mutational analysis was performed and various mutant forms of both SEQ ID NO: 1 and SEQ ID NO: 2 were constructed, purified and analyzed (see Table B-2). The amino acid sequence of the monovalent building block SEQ ID NO: 2 is shown in Figure 4 and the residues that are discussed in the present report are highlighted. At least one batch does not contain RPC post-peak 1 (see Figure 1), which was found to be the result of the mis-incorporation of norleucine during biosynthesis (see below).

**Table B-2:** List of modified/mutant forms of SEQ ID NO: 1 or SEQ ID NO: 2 used in the present characterization study. All recombinant proteins were produced in *E. coli* and purified on ion exchange chromatography followed by size exclusion chromatography.

| Names | SEQ ID NO: | Amino Acid Sequence |
|---|---|---|
| SEQ ID NO: 1, ALX-0081 | 1 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEDGRVRTLPSEYTFWGQGTQVTVSS AAAEVQLVESGGGLVQPGGSLRLSCAASGRTFSYNPMGWFRQ APGKGRELVAAISRTGGSTYYPDSVEGRFTISRDNAKRMVYLQ MNSLRAEDTAVYYCAAAGVRAEDGRVRTLPSEYTFWGQGTQV TVSS |
| SEQ ID NO: 2, 12A2H1, vWF-12A2h1 | 2 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEDGRVRTLPSEYTFWGQGTQVTVSS |
| SEQ ID NO:1 E1Q | 3 | QVQLVESGGGLVQPGGSLRLSCAASGRTFSYNPMGWFRQAPG KGRELVAAISRTGGSTYYPDSVEGRFTISRDNAKRMVYLQMNS LRAEDTAVYYCAAAGVRAEDGRVRTLPSEYTFWGQGTQVTVS SAAAEVQLVESGGGLVQPGGSLRLSCAASGRTFSYNPMGWFRQ APGKGRELVAAISRTGGSTYYPDSVEGRFTISRDNAKRMVYLQ MNSLRAEDTAVYYCAAAGVRAEDGRVRTLPSEYTFWGQGTQV TVSS |
| SEQ ID NO:1 (global methionine to norleucine substitution) | 4 | EVQLVESGGGLVQPGGSLRLSCAASGRTFSYNPXGWFRQAPGK GRELVAAISRTGGSTYYPDSVEGRFTISRDNAKRXVYLQXNSLR AEDTAVYYCAAAGVRAEDGRVRTLPSEYTFWGQGTQVTVSSA AAEVQLVESGGGLVQPGGSLRLSCAASGRTFSYNPXGWFRQAP GKGRELVAAISRTGGSTYYPDSVEGRFTISRDNAKRXVYLQXNS LRAEDTAVYYCAAAGVRAEDGRVRTLPSEYTFWGQGTQVTVS S |
| SEQ ID NO:2 M34A | 5 | EVQLVESGGGLVQPGGSLRLSCAASGRTFSYNPAGWFRQAPGK GRELVAAISRTGGSTYYPDSVEGRFTISRDNAKRMVYLQMNSL RAEDTAVYYCAAAGVRAEDGRVRTLPSEYTFWGQGTQVTVSS |

| Names | SEQ ID NO: | Amino Acid Sequence |
|---|---|---|
| SEQ ID NO:2 D62E | 6 | EVQLVESGGGLVQPGGSLRLSCAASGRTFSYNPMGWFRQAPGK GRELVAAISRTGGSTYYPESVEGRFTISRDNAKRMVYLQMNSL RAEDTAVYYCAAAGVRAEDGRVRTLPSEYTFWGQGTQVTVSS |
| SEQ ID NO:2 D62A | 7 | EVQLVESGGGLVQPGGSLRLSCAASGRTFSYNPMGWFRQAPGK GRELVAAISRTGGSTYYPASVEGRFTISRDNAKRMVYLQMNSL RAEDTAVYYCAAAGVRAEDGRVRTLPSEYTFWGQGTQVTVSS |
| SEQ ID NO:2 S63G | 8 | EVQLVESGGGLVQPGGSLRLSCAASGRTFSYNPMGWFRQAPGK GRELVAAISRTGGSTYYPDGVEGRFTISRDNAKRMVYLQMNSL RAEDTAVYYCAAAGVRAEDGRVRTLPSEYTFWGQGTQVTVSS |
| SEQ ID NO:2 M78A | 9 | EVQLVESGGGLVQPGGSLRLSCAASGRTFSYNPMGWFRQAPGK GRELVAAISRTGGSTYYPDSVEGRFTISRDNAKRAVYLQMNSLR AEDTAVYYCAAAGVRAEDGRVRTLPSEYTFWGQGTQVTVSS |
| SEQ ID NO:2 M83A | 10 | EVQLVESGGGLVQPGGSLRLSCAASGRTFSYNPMGWFRQAPGK GRELVAAISRTGGSTYYPDSVEGRFTISRDNAKRMVYLQANSLR AEDTAVYYCAAAGVRAEDGRVRTLPSEYTFWGQGTQVTVSS |
| SEQ ID NO:2 N84D | 11 | EVQLVESGGGLVQPGGSLRLSCAASGRTFSYNPMGWFRQAPGK GRELVAAISRTGGSTYYPDSVEGRFTISRDNAKRMVYLQMDSL RAEDTAVYYCAAAGVRAEDGRVRTLPSEYTFWGQGTQVTVSS |
| SEQ ID NO:2 D105E | 12 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEEGRVRTLPSEYTFWGQGTQVTVSS |
| SEQ ID NO:2 D105A | 13 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEGRVRTLPSEYTFWGQGTQVTVSS |
| SEQ ID NO:2 D105N | 14 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAENGRVRTLPSEYTFWGQGTQVTVSS |

(continued)

| Names | SEQ ID NO: | Amino Acid Sequence |
|---|---|---|
| SEQ ID NO:2 D105Q | 15 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEQGRVRTLPSEYTFWGQGTQVTVSS |
| SEQ ID NO:2 D105S | 16 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAESGRVRTLPSEYTFWGQGTQVTVSS |
| SEQ ID NO:2 D105T | 17 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAETGRVRTLPSEYTFWGQGTQVTVSS |
| SEQ ID NO:2 G106A | 18 | EVQLVESGGGLVQPGGSLRLSCAASGRTFSYNPMGWFRQAPGK GRELVAAISRTGGSTYYPDSVEGRFTISRDNAKRMVYLQMNSL RAEDTAVYYCAAAGVRAEDARVRTLPSEYTFWGQGTQVTVSS |

Other mutants of SED ID NO: 2:

| Mutants | SEQ ID NO: | |
|---|---|---|
| VWF0001; SEQ ID NO:1 with | 25 | DVQLVESGGGLVQPGGSLRLSCAASGRTFSYNPMGWFRQAPGKG RELVAAISRTGGSTYYPESVEGRFTISRDNAKRTVYLQMNSLRAE DTAVYYCAAAGVRAEQGRVRTLPSEYTFWGQGTQVTVSSAAAE VQLVESGGGLVQPGGSLRLSCAASGRTFSYNPMGWFRQAPGKGR |
| E1D, D62E, M78T, D105Q, D193E, M209T, D236Q | | ELVAAISRTGGSTYYPESVEGRFTISRDNAKRTVYLQMNSLRAED TAVYYCAAAGVRAEQGRVRTLPSEYTFWGQGTQVTVSS |
| Stab. 12A2H1; SEQ ID NO: 2 with E1D, D62E, M78T, D105Q | 26 | DVQLVESGGGLVQPGGSLRLSCAASGRTFSYNPMGWFRQAPGKG RELVAAISRTGGSTYYPESVEGRFTISRDNAKRTVYLQMNSLRAE DTAVYYCAAAGVRAEQGRVRTLPSEYTFWGQGTQVTVSS |
| SEQ ID NO:1 with E1D, D62E, M78A, D105Q, D193E, M209A, D236Q | 39 | DVQLVESGGGLVQPGGSLRLSCAASGRTFSYNPMGWFRQAPGKG RELVAAISRTGGSTYYPESVEGRFTISRDNAKRAVYLQMNSLRAE DTAVYYCAAAGVRAEQGRVRTLPSEYTFWGQGTQVTVSSAAAE VQLVESGGGLVQPGGSLRLSCAASGRTFSYNPMGWFRQAPGKGR ELVAAISRTGGSTYYPESVEGRFTISRDNAKRAVYLQMNSLRAED TAVYYCAAAGVRAEQGRVRTLPSEYTFWGQGTQVTVSS |
| SEQ ID NO: 2 with E1D, D62E, M78A, D105Q | 40 | DVQLVESGGGLVQPGGSLRLSCAASGRTFSYNPMGWFRQAPGKG RELVAAISRTGGSTYYPESVEGRFTISRDNAKRAVYLQMNSLRAE DTAVYYCAAAGVRAEQGRVRTLPSEYTFWGQGTQVTVSS |

## 2) Methods:

### Generation of mutants of SEQ ID NO: 1 and SEQ ID NO: 2 (Table B-2):

Mutations were introduced with the QuikChange site-directed mutagenesis kit (Stratagene, CA, USA). The QuikChange site-directed mutagenesis method is performed using *PfuTurbo®* DNA polymerase and a temperature cycler. *PfuTurbo* DNA polymerase replicates both plasmid strands with high fidelity and without displacing the mutant oligonucleotide primers. The basic procedure utilizes a supercoiled double-stranded DNA (dsDNA) vector with an insert of interest and two synthetic oligonucleotide primers containing the desired mutation. The oligonucleotide primers, each complementary to opposite strands of the vector, are extended during temperature cycling by *PfuTurbo* DNA polymerase. Incorporation of the oligonucleotide primers generates a mutated plasmid containing staggered nicks. Following temperature cycling, the product is treated with *Dpn* I. The *Dpn* I endonuclease (target sequence: 5'-Gm6ATC-3') is specific for methylated and hemimethylated DNA and is used to digest the parental DNA template and to select for mutation-containing synthesized DNA. DNA isolated from almost all *E. coli* strains is dam-methylated and therefore susceptible to *Dpn* I digestion.

### 2.1) Binding potency of SEQ ID NO: 1, SEQ ID NO: 2 and mutants thereof

The binding potency of SEQ ID NO: 1 was determined using a parallel line. The method is designed in such a way that bi-functionality is observed; therefore, SEQ ID NO: 2, the monovalent building block of SEQ ID NO: 1 possesses no potency.

The affinity of wt and mutant forms of SEQ ID NO: 2 for the A1 domain of vWF was determined by means of SPR technology (BIAcore). Various concentrations of SEQ ID NO: 2 (0.5 to 20 nM range) were passed over an A1 sensor-chip. The kinetic constants, $k_{on}$ and $k_{off}$, were deduced from the obtained sensorgrams and used to calculate the equilibrium dissociation constant, $K_D$. In certain experiments, samples of wt and mutant SEQ ID NO: 2 were compared by measuring the initial binding rate to an A1 sensor-chip. For these experiments, the samples were diluted to a 2nM concentration. The initial binding rate was determined from the association phase of the sensorgram by linear fitting of the data points obtained between 5 and 25 seconds after the injection start and the reported value is the average of 5 independent measurements.

### 2.2) RP-HPLC analysis of SEQ ID NO: 1, SEQ ID NO: 2 and mutants thereof

Most of the RPC analyses on intact protein were in essence performed as described elsewhere (A.A. Wakankar et al., Biochemistry 2007, 46, 1534-1544). Relevant deviations are mentioned in the present report. RPC analysis of tryptic digests is described in more detail below.

### 2.3) Determination of the concentration of SEQ ID NO: 1 by RPC

### Reverse Phase-HPLC (RP-HPLC or RPC)

The column used was a ZORBAX 300SB C3 (5 μm) column on an Agilent system. The column had a temperature of 70°C during the experiments. Buffer A used during experiments was 0.1% trifluoroacetic acid and buffer B was 0.1% trifluoroacetic acid / 99.9% acetonitril. The program used is described below:

*Program*

| Time (min) | %B |
|---|---|
| 2 | 10 |
| 3 | 27,5 |
| 25,5 | 35 |
| 26 | 100 |
| 28,5 | 100 |
| 29 | 10 |
| 35 | 10 |

As a general rule, the concentration of SEQ ID NO:1, SEQ ID NO:2 and mutants thereof was determined spectrophotometrically. The material contained in certain RP-HPLC fractions was recovered for direct measurement of the bio-activity. The recovery yield was typically quite low and the concentration of SEQ ID NO:1 (product-related variant) in the reconstituted sample did not allow for the spectrophotometric determination of the concentration. In such cases, the concentration was determined by re-analysis of the collected material by RPC and comparison of the peak area against a calibration curve which was established with SEQ ID NO:1 BATCH 1 and which relates the amount of SEQ ID NO:1 to the RPC total peak area.

### 2.4) Mass spectrometry

The mass spectrometric analyses were performed with an MSD ESI-TOF instrument from Agilent. The instrument was coupled to an Agilent 1100 HPLC in case it was used as an MS detector to determine the masses of the various peaks in the RPC method. For the mass determination of separate samples, a Poros-1 column was used for buffer exchange prior to MS analysis.

The MS and MS/MS experiments performed at the Ghent University, Laboratory of Protein Biochemistry and Protein Engineering were run on 4700 Proteomics Analyzer (MALDI-TOF/TOF, Applied Biosystems) or a quadrupole time-of-flight instrument (Q-TOF I, Micromass/Waters) operating in the positive ion mode. At Ablynx and at the Free University of Brussels, ESI mass spectra were acquired using a Q-TOF Ultima (Micromass/Waters).

For the MALDI-analyses, $0.5\mu L$ of the tryptic digestion was mixed with $10\mu L$ $\alpha$-cyano-4-hydroxycinnamic acid (10mg in 1mL of 50% ACN, 10% ethanol and 0.1% TFA) and spotted on the MALDI target plate. Spectra were recorded in the m/z-range of 400 to 4500 Th. For ESI-MS analyses, spectra were recorded in the m/z range of 400 to 1200 Th. The final spectra were deconvoluted and the molecular mass of proteins was determined using MaxEnt software (Micromass/Waters). ESI-MS/MS analyses were performed via collision induced 6 dissociation (CID)[6]. The MaxEnt3 algorithm (Micromass/Waters) was used to derive sequence information from the MS/MS spectra.

### 2.5) Tryptic peptide map of SEQ ID NO:1

Samples of SEQ ID NO:1 or SEQ ID NO:2 (ca. 1 mg/mL final concentration) were digested with trypsin modified by reductive methylation (Promega; ca. 20 $\mu$g/mL final concentration). Dilutions were carried out with D-PBS if required and the hydrolysis was performed at 37°C during 24h before freezing the mixture at -20°C. Peptide fractionations were performed on a C3-column operated at 70°C. Typically, 25 $\mu$L of the digest (ca. 20 $\mu$g of protein) is injected onto the column and the peptides eluted with an acetonitrile gradient increasing from 5% ACN - 0.1% TFA to 36.7% ACN - 0.1 % TFA over 97 minutes. The peaks were detected at 214nm. Figure 5 presents the chromatogram obtained after tryptic digestion, overlaid with the profile obtained after 8 weeks incubation at 37°C. The identity of most of the peaks was established by mass spectrometry (see Table B-3).

**Table B-3:** Identity of peaks observed in a tryptic map of SEQ ID NO: 1. T2-T2' represents the expected disulfide linked peptides. T2-T2 and T2'-T2' are unexpected; the fact that these are also observed with SEQ ID NO:2 indicates that they reflect some artifactual disulfide reshuffling and are not the result of erroneous disulfide bonding.

| Peptide | Sequence (modifications identified in the present study are indicated) |
|---|---|
| T2-T2 | LSCAASGR + LSCAASGR |
| T8 | FTISR |
| T7≠ | TGGSTYYPisoDSVEGR (isomerization of D62, see below) |
| T7 | TGGSTYYPDSVEGR |
| T6 | ELVAAISR |
| T10≠ | primarily MVYLQMN (resulting from non-specific fragmentation) and MoxVYLQMNSLR (oxidation of M78, see below) |
| T2-T2' | LSCAASGR + AEDTAVYYCAAAGVR |
| T10 | MVYLQMNSLR |
| T1 | EVQLVESGGGLVQPGGSLR |
| T2'-T2' | AEDTAVYYCAAAGVR + AEDTAVYYCAAAGVR |
| T1≠ | pyroEVQLVESGGGLVQPGGSLR (pyroglutamate, see below) |
| T13 | TLPSEYTFWGQGTQVTVSS |
| T3 | TFSYNPMGWFR |
| T12 | TLPSEYTFWGQGTQVTVSSAAAEVQLVESGGGLVQPGGSLR |

### 2.6) Enzymatic quantification of the amount of isoaspartic acid

The ISOQUANT® Isoaspartate Detection Kit (Promega Corporation, Madison, WI, USA) was used for quantitative detection of isoaspartic acid residues in SEQ ID NO:1 and SEQ ID NO:2. The ISOQUANT® method uses the enzyme Protein Isoaspartyl Methyltransferase (PIMT), an enzyme which mediates the conversion of an atypical β-aspartic acid peptide bond to a normal peptide bond. PIMT catalyzes the transfer of the active methyl group from S-adenosyl-L-methionine (SAM) to isoaspartic acid at the α-carboxyl position, to form an O-methyl ester and generating S-adenosyl homocysteine (SAH) in the process. Spontaneous decomposition of this methyl ester results in the release of methanol and formation of a succinimide intermediate (i.e. the same cyclic imide intermediate that forms during the deamidation of asparagine residues and rearrangement of aspartic acid residues). The cyclic imide then slowly hydrolyzes to form a mixture of aspartate and isoaspartate. With each cycle of methylation, 15-30% of the atypical peptide bond is converted to a normal peptide bond. The methylation-dependent conversion of isopeptide bonds to normal peptide bonds supports a biological role for PIMT in the repair of age-damaged proteins.

Enzymatic methylation provides a highly sensitive and quantitative assay for determination of isoaspartate in peptides or proteins. In one format, the ISOQUANT® kit detects a co-product of the methylation reaction, SAH. Since this is a relatively small molecule, it can usually be isolated from peptides and quantitated by reverse phase high pressure liquid chromatography (RP-HPLC using the Everest C18 column). The amount of isoaspartic acid to target protein in test samples is determined by comparison with reactions performed using a reference standard peptide (sequence: WAGG-IsoD-ASGE) and the aid of SAH HPLC standard (both reagents are provided with the kit). Typically, ~82% of the expected isoaspartate was recovered in experiments with the positive control peptide. The assays were performed according to the instructions of the manufacturer of the ISOQUANT® kit. We found that it was necessary to fragment samples of SEQ ID NO:1 or SEQ ID NO:2 in order to minimize the effect of protein structure on the detection of isoaspartic acid residues. It was indeed observed that the number of picomoles of isoaspartic acid measured in the intact protein was markedly lower than the number of picomoles in the digested protein. Protease digestions of the samples were performed with trypsin (1:50; w:w) during 24 h at 37°C and the reactions were stopped with the protease inhibitor PMSF (1mM final concentration; phenylmethanesulphonylfluoride). Adequate blank/control reactions were included in the ISOQUANT® analyses to confirm the absence of residual trypsin activity and to account for any isoaspartic acid residues present in the trypsin protease.

### 3) Results

### 3.1) Analysis of RPC pre-peak 1 (and pre-peak 3)

The RPC pre-peaks (substance eluting before the main material) include a predominant pre-peak 1. The relative peak area of this pre-peak 1 increases with storage time and temperature (see Figure 2). It was previously shown by ESI mass spectrometry and forced oxidation experiments (refer to Figure 6) that this pre-peak 1 represents a mono-oxidized form which is 16Da heavier than the intended SEQ ID NO: 1. It was hypothesized that this oxidation occurs at one or more methionine residues based on the fact that this is a common type of chemical modification of proteins. SEQ ID NO:1 contains six methionine residues, three in each of the two identical domains (Figure 4). A deliberate oxidation can generate additional (multiple) oxidized species, most notably the +32Da species indicated in Figure 6. All of the oxidation variants seem to be active as evidenced by the observation that material which consists of 14% main peak and as much as 86% pre-peaks is fully functional.

The contention that the pre-peak is fully active was further substantiated by direct analysis of the pre-peak 1 material, collected during RP-HPLC separation. To this end, the elution fraction representing pre-peak 1 was dried using a speedvac (miVAc concentrator, Genevac), re-solubilized in PBS with 0.02% Tween 80 and, after determination of the concentration, analyzed in the potency assay. The identity of the isolated material was confirmed by re-analysis by RPC. This also permitted the quantification of the pre-peak 1 material by comparison of the peak area against an calibration curve which was established with SEQ ID NO:1 (see section 6.3; note that the relatively low amounts of pre-peak 1 material did not allow for the spectrophotometric determination of the concentration). The RPC main peak material was isolated, reconstituted and quantified using the same methodology by way of control. It was found that the potencies of the RPC main peak and pre-peak 1 are practically identical; the relative potencies were 66.7% and 65.5% for the main peak and the pre-peak, respectively. The equality of the recovered main peak and pre-peak 1 material strongly supports the notion that oxidized SEQ ID NO:1 is as active as the authentic protein.

The oxidation of the SEQ ID NO: 1 Nanobody® was further investigated using SEQ ID NO:2, i.e. the monovalent building block of SEQ ID NO:1. We anticipated this would be advantageous because the SEQ ID NO: 2 sub-domain of SEQ ID NO:1 contains only three methionine residues, thus reducing the complexity, i.e. the theoretically possible number of oxidation variants. We also discovered that the non-oxidized molecule and the various oxidation variants are better resolved in the case of SEQ ID NO:2 as compared to SEQ ID NO:1. Figure 7 shows that oxidation of the native SEQ ID NO:2 Nanobody takes for the most part place on one single methionine residue; in contrast, all three methionine residues are susceptible to oxidation when the molecule is unfolded by guanidinium hydrochloride treatment. The identification of the residue that is vulnerable to oxidation, and, at the same time, conclusive demonstration that the oxidation indeed takes place at a methionine, was obtained by analysis of the three different methionine-to-alanine mutants, i.e. M34A, M78A, and M83A. These analyses, shown in Figure 8, clearly indicate that oxidation takes place at the methionine residue at position 78; the M34A and M83A mutants are still susceptible to oxidation whereas substitution of M78 by an alanine appears to result in a SEQ ID NO:2 variant that is essentially resistant to forced oxidation (at least under the test conditions). Unlike the M34A and M83A mutants, the M78A variant does not contain a pre-peak at the time of production, indicating that the pre-peak is solely caused by M78 oxidation (data not shown). Additionally, the small pre-peak seen in the case of M34A and M83A was also found to increase upon prolonged storage (e.g. 6 weeks; data not shown) at 37°C. The latter observation confirms that air oxidation and forced peroxide-mediated oxidation target the same M78 residue. The conclusion that basically only a single methionine in the monovalent SEQ ID NO:2 is susceptible to oxidation is in line with the accumulation of both a singly and doubly oxidized species during continued oxidation of the bivalent SEQ ID NO:1 Nanobody® (see Figure 6). It would appear that this doubly oxidized form corresponds to pre-peak 3 in the RPC profile.

It is also of note that the affinities of all three methionine-to-alanine mutants for the immobilized vWF A1 domain are in the same range as the binding constant of the wt SEQ ID NO:2 Nanobody (1.2nM). The equilibrium dissociation constants of M34A, M78A, and M83A were found to be 1.96nM, 1.46nM, and 1.29nM, respectively. The result is in line with the finding that methionine oxidation does not affect the potency of SEQ ID NO:1. It would therefore appear that the methionine residues are not implicated in the binding to the vWF A1 domain.

The oxidation of SEQ ID NO:1 was also examined with the use of the tryptic peptide map. Peak T10≠ (see Figure 5 and Table B-3) was shown to contain a peptide of 1269.6 Da, exactly corresponding to a single oxidized variant of T10 (1253.5 + 16Da). This does not permit to locate the oxidation sensitive methionine residue since the T10 peptide encompasses both M78 and M83. Moreover, the T10≠ peak was found to consist predominantly of a non-specific cleavage product of 897.4 Da (MVYLQMN; see Table B-3 and peptide mapping development report), which also includes the methionines at position 78 and 83. The latter fragment accounts for the observation that the peak area of T10≠ relative to T10 is already high at time zero and does not markedly increase during incubation at 37°C. Indeed, oxidation will have opposing effects on the two fragments present in T10≠, on the one hand an increase of the T10+16Da variant and, on the other hand, a decrease of the non-specific cleavage product. Analysis of the peptide mapping data also shows that the area of the T10 peak is gradually decreasing during storage at 37°C. While this is consistent with an oxidation of the methionine residue at position 78, the results have to be treated with caution given the possible interference with non-specific cleavage. The 1269.6 Da fragment, which was observed in SEQ ID NO:1 after 8 weeks incubation at 37°C was subjected to ESI-Q-TOF MS/MS analysis. The partial sequence data obtained in this experiment did not allow direct confirmation of a methionyl sulfoxide at position 78. This could however be inferred from the presence of an unmodified methionine at position 83.

### 3.2) Analysis of RPC post-peak 1

Post-peak 1 results from the mis-incorporation of a norleucine residue at one methionine site during biosynthesis. This was conclusively demonstrated by a combination of mass spectrometry and amino acid analysis. EletroSpray Ionization Time of flight (ESI-TOF) measurements have shown that post-peak 1 is dominated by a protein with a mass of 27858 Da, an -18 Da lower molecular mass than the authentic disulfide bonded SEQ ID NO:1 Nanobody®. One of the possible explanations for a -18 Da difference is the substitution of a norleucine for a methionine residue. This assumption was confirmed by amino acid analysis of the purified main peak and post-peak which indicated that only the post-peak 1 material contained norleucine and a proportionally lower amount of methionine. It should be noted that norleucine which is typically used as an internal standard in amino acid analyses was omitted in these experiments.

To determine the potency (i.e. looking at affinity measurements) of the -18Da product related variant, post-peak 1 was collected during RP-HPLC separation and directly measured in the PLA potency assay. Similar to what was done for pre-peak 1, the elution fraction representing post-peak 1 was first dried using a speedvac (miVac Duo Concentrator, Genevac) and then re-solubilized in PBS. The RPC main peak was purified in the same way as a control. Three independent but slightly different experiments were performed. In a first experiment the concentration after reconstitution was determined spectrophotometrically and no Tween was added to the PBS. In a second experiment, 0.02% Tween was present in the PBS at the time of reconstitution of the dried material (to prevent loss of protein from the relatively dilute samples) and the concentration was deduced from the peak area in a re-analysis by RP-HPLC. The RPC re-analysis also confirmed the identity of the isolated material. In a third experiment, we additionally diluted the purified main peak material to about the same concentration as the post-peak variant so to eliminate as much as possible any dissimilarities between the main and the post-peak samples. In a first experiment, the main peak and post-peak 1 fraction were collected after RPC analysis, concentrated on a centrifugal filter unit (Microcon YM-3, 3kDa NMW, Millipore), and the buffer exchanged into D-PBS by gelfiltration on a Sephadex G-25 spin column. During the experiment, precipitation of part of the material was observed and there was some uncertainty about the concentrations used in the potency assay. The potencies of the main peak and of post-peak 1 were found to be 91.9% and 51.6% respectively of that of reference SEQ ID NO:1. In a second experiment, the protein was reconstituted from the RPC fractions by drying and re-solubilization in water. In contrast to the first measurement, main peak and post-peak 1 were found to possess a potency of 50% and 81 %, respectively. In total, the above results provide strong evidence that post-peak 1 has the same potency as authentic SEQ ID NO:1. The observed variation was attributed to the difficulty in recovering intact material from the $H_2O$-TFA-ACN solvent; the recovery by itself was found to be quite low, especially in the case of the low-abundant product related variant.

To further substantiate the hypothesis that norleucine has no adverse effect on potency, irrespective of the site of incorporation in SEQ ID NO:1, we produced an SEQ ID NO:1 variant with global replacement of the methionines by norleucine residues. This SEQ ID NO:1 variant was produced in a methionine auxotrophic strain grown in a minimal medium supplemented with L-norleucine. More specifically, the cells were first grown in rich medium under non-inducing conditions, then collected by centrifugation and resuspended in minimal medium containing L-norleucine, and finally, following a short incubation time, induced by the addition of IPTG. This method has been described previously (Cirino et al., 2003, Biotechnol. Bioeng. 83, 729-734). The SEQ ID NO:1 preparation was characterized by RPC, peptide mapping and MS; in essence, these methods confirmed the exchange of all six methionines by norleucine residues. It is clear from Figure 9 however that the preparation contains, in addition to the intended norleucine variant (labeled 6 in Figure 9), smaller amounts of all possible partially substituted variants (labeled 1 to 5 in Figure 9) as well as some wt SEQ ID NO:1. The presence of wt SEQ ID NO:1 is in all likelihood the result of leaky expression as a consequence of incomplete shut-off of the promoter during precultivation of the cells in rich medium. The partially substituted forms clearly indicate the presence of residual methionine after shift to the minimal medium + norleucine. The relative potency of the multiple substituted variant was found to be 80% (average of three measurements: 67.9%, 85.5% and 86.7%). Hence, it would appear that the bio-activity of SEQ ID NO:1 is not even affected in case of the complete substitution of all six methionine residues by norleucines, a result which necessarily implies that a single norleucine exchange, irrespective of its position, also has no effect on the potency.

The conclusion that the potency of SEQ ID NO:1 is not (measurably) affected by substitution of a single methionine by a norleucine residue nor by the oxidation of methionine-78 is in accordance with the observation that the affinities of all three methionine-to-alanine mutants (see section 7.1) for the immobilized vWF A1 domain are in the same range as the binding constant of the wt SEQ ID NO:2 Nanobody. The equilibrium dissociation constants of wild type, M34A, M78A, and M83A were found to be 1.2nM, 1.96nM, 1.46nM, and 1.29nM, respectively. Such relatively small differences, assuming they are genuine, are unlikely to be revealed under the avid conditions of binding of the bivalent SEQ ID NO:1 to immobilized vWF A1 domain. Taken together, the data indicate that the methionine residues are not critical for the bio-activity of SEQ ID NO:1.

## 3.3) Analysis of RPC post-peak 2

Post-peak 2 is to some extent formed by the RPC analysis method

Post-peak 2 amounts to about 0.9% of the total peak area at the time of production. It was observed that this post-peak increases when the RPC run is changed such that the material stays on the column at 70°C for a longer time before it is eluted. An experiment where the elution was delayed by 15min, 30min, 60 min or 120 min indicated that the post-peak 2 area correlates linearly with the residence time on the column (see Figure 10). Extrapolation to time zero indicates that post-peak 2 would nearly not occur in the impracticable case of zero residence time on the column. These findings let us to conclude that the SEQ ID NO:1 variant that is represented by post-peak 2 is as good as absent from the various batches of SEQ ID NO:1 at the time of production and only forms during relatively short exposure to the 70°C temperature on the RPC column or during storage (see Figure 2 and SEQ ID NO: 1).

Post-peak 2 represents SEQ ID NO:1 with an N-terminal pyroglutamate

Post-peak 2 does form during prolonged incubation at 5°C, 25°C and 40°C. This is also illustrated in Figure 2. The % peak area is however an exaggeration since part of the variant develops during RPC analysis on the column. We have shown that post-peak 2 is to be attributed to the conversion of the N-terminal glutamate residue to a cyclic pyroglutamate. The various experiments that led to this conclusion are discussed below.

The post-peak 2 variant is 18Da lighter than authentic SEQ ID NO:1

To identify the protein eluting as post-peak 2, the post-peak was collected after RP-HPLC separation and its mass determined by ESI-TOF mass spectrometry. Due to the too low concentration available in the BATCH 1 standard, the material used for the ESI-QTOF analysis was a BATCH 1 preparation stored during 4 weeks at 37°C. The major peak from the RPC fraction had a mass of 27858 Da, i.e. 18Da lighter than native SEQ ID NO:1 (27876Da). While there may be several different explanations for such a decrease in mass, it is consistent with the loss of a water molecule as a result of the cyclization of the N-terminal glutamic acid residue leading to a pyroglutamate. (The formal possibility that post-peak 2, similar to post-peak 1, represents an SEQ ID NO:1 variant where a methionine residue is replaced by norleucine during bio-synthesis is rejected because the relative peak area is in that case not influenced by time or temperature of incubation.)

An engineered pyroglutamate variant has the same retention time as RPC post-peak 2

In general, a pyroglutamate appears more readily in case of an N-terminal glutamine residue as compared to a glutamate (Gadgil et al., 2006, J. of the American Society of Mass Spectrometry 17, 867-872). We therefore decided to construct an SEQ ID NO: 1 variant where a glutamine substitutes for the N-terminal glutamic acid (E1Q mutant). This SEQ ID NO:1 mutant could be fractionated on a monoS column at pH 4; two about equally abundant species, designated E1Q-a and E1Q-b, were found to elute. Both fractions were desalted and subjected to ESI-TOF mass spectrometry; E1Q-b was found to have a mass of 27874 Da (the expected mass of the E1Q variant is 27875 Da) whereas E1Q-a had a mass of 27857 Da (cyclization of the N-terminal glutamine results in loss of an $NH_3$ molecule or 17Da; the expected mass is 27858 Da). From these data it was concluded that E1Q-b corresponds to SEQ ID NO:1 with an N-terminal glutamine residue while E1Q-a represents the cyclized pyroglutamate form. RPC analysis showed that E1Q-a has the same retention time as post-peak 2 whereas E1Q-b profile contains two peaks, coinciding with BATCH 1 main peak and post-peak 2 (see Figure 11). Most probably, the latter result must be attributed to the fact that a significant part of the N-terminal glutamine is converted to pyroglutamate during the RPC analysis as is observed for the wild-type SEQ ID NO:1 molecule. Taken together, the data support the notion that RPC post-peak 2 is to be attributed to the formation of the cyclic pyroglutamate form.

Peptide mapping confirms the formation of pyroglutamate during storage

The formation of a pyroglutamate residue at the N-terminus was confirmed by peptide mapping (see section 2.5). RPC analysis of the tryptic peptides reveals the presence of a peak (i.e. peak T1≠ according to Table B-3) whose mass corresponds to that of the N-terminal fragment minus 18Da. Partial sequence determination by MS/MS analysis has confirmed that this peptide indeed corresponds to the N-terminal T1 tryptic fragment. In accordance with what is observed for RPC post-peak 2, the relative peak area of peak T1≠ in the peptide map was found to increase with storage time; the pyroglutamate N-terminal peptide does (almost) not exist in BATCH 1 whereas it is well present after storage at 37°C for 8 weeks (see Figure 13). Concurrent with the increase in the T1≠ peak area, the area of the unmodified N-terminal T1 peptide decreases with storage time (data not shown). The identity of the T1≠ peak was additionally demonstrated by performing a peptide mapping on the E1Q-a and E1Q-b variants discussed above. Consistent with the notion that E1Q-a represents SEQ ID NO:1 with a cyclic pyroglutamate at the N-terminus, this variant was found to lack the T1 peptide and instead to yield a fragment that coincides with the T1≠ peak. The E1Q-b variant was found to produce the same T1≠ peak as well as another peak which we assume to correspond to the T1-peptide containing a glutamine rather than a glutamic acid residue at position 1 (see Figure 13).

SEQ ID NO:1 and its pyroglutamate variant are equally potent

The potency, i.e. binding affinities, of E1Q-a, i.e. the pyroglutamate variant of SEQ ID NO:1 (see above), was determined as above. The average value for the potency relative to that of BATCH 1 was 105%; the 95% CL in the two experiments was 105%-113% and 96%-106%. It is concluded that the potencies of SEQ ID NO:1 and its pyroglutamate form are not significantly different.

Detection of the SEQ ID NO:1 pyroglutamate variant by cIEF

The pyroglutamate modification was found to induce a shift in the isoelectric point (pI) of SEQ ID NO:1; this is manifested by the cIEF analyses of BATCH 1 and a mixture of BATCH 1 and the above mentioned E1Q-a pyroglutamate variant (see Figure 14). Pyroglutamate formation clearly yields a distinct peak with a higher pI. Figure 14 also shows that replacement of the glutamate by a glutamine (i.e. the E1Q-b variant) has also an effect on the pI, albeit less pronounced than the cyclic pyroglutamate. The apparent effects on the pI of the pyroglutamate on the one hand and the E1Q-b variant on the other hand are not easily rationalized.

The electropherogram of SEQ ID NO:1 incubated at 37°C for 6 weeks clearly shows that storage results in the appearance of a number of new peaks in addition to the main peak (Figure 15). The most prominent new peak, characterized by a higher pI, can now unambiguously be assigned to the formation of the N-terminal pyroglutamate ring. Figure 15 shows that this higher-pI variant coincides with the E1Q-a species. The N-terminal pyroglutamate product can thus be detected as post-peak 2 by RP-HPLC and as a higher-pI variant in cIEF. In contrast to post-peak 2, the higher-pI variant is not observed in SEQ ID NO:1 batches that have not been put on storage; this observation supports the notion that the RPC conditions themselves induce some pyroglutamate formation. When this effect is taken into account, the amounts of pyroglutamate formation as deduced from the relative peak area using the two different techniques are in good agreement (data not shown).

Pyroglutamate formation has also been observed for other nanobody constructs (see Figures 41 and 42). Please note that the amino acid sequence of nanobody 321 is listed in SEQ ID NO: 38.

Amino Acid Sequence of Nanobody 321 - SEO ID NO: 38:

EVQLLESGGGLVQPGGSLRLSCAASGRIFSLPASGNIFNLLTIAWYRQAPGKGRELVA

TINSGSRTYYADSVKGRFTISRDNSKKTLYLQMNSLRPEDTAVYYCQTSGSGSPNFW

GQGTLVTVSSGGGGSGGGSEVQLVESGGGLVQPGNSLRLSCAASGFTFSSFGMSWVR

QAPGKGLEWVSSISGSGSDTLYADSVKGRFTISRDNAKTTLYLQMNSLRPEDTAVYY

CTIGGSLSRSSQGTLVTVSSGGGGSGGGSEVQLLESGGGLVQPGGSLRLSCAASGRTL

SSYAMGWFRQAPGKGREFVSRISQGGTAIYYADSVKGRFTISRDNSKNTLYLQMNSL

RPEDTAVYYCAKDPSPYYRGSAYLLSGSYDSWGQGTLVTVSS

There is an indication that pyroglutamte formation increases with a higher pH environment (pH6.5 seems to show higher pyroglutamte formation than pH6 - see Figure 42).

### 3.4) Analysis of the split of the RPC main peak during storage

The RPC main peak splits up after prolonged storage

The RPC main peak observed with SEQ ID NO:1 appears to divide into several different species upon prolonged incubation at elevated temperatures. The data indicate that some earlier eluting new species are generated during prolonged storage (see Figure 3 and 16).

The analyses were found to be more straightforward with the monovalent building block SEQ ID NO:2 because of an improved resolution. The RPC profile reveals more readily the break up of the main peak while, at the same time, the complexity is reduced. Figure 16 shows that two new earlier eluting peaks can be discerned. As indicated in Figure 16, we will refer to these peaks as I1 and I2.

Splitting of the main peak results from isomerization of D105 and D62

To identify the earlier eluting I1 and I2 species, these two peaks, as well as the main peak, were collected after RP-HPLC separation and their mass determined by ESI-TOF mass spectrometry. The material used for the ESI-TOF analysis was a preparation of SEQ ID NO:2 stored during 6 weeks at 37°C. All three peaks were found to have the same mass, i.e. the mass calculated for SEQ ID NO:2. The result demonstrates that the modifications that create I1 and I2 do not affect the mass. The data led us to hypothesize that I1 and I2 may result from either the mass-neutral isomerization of an aspartic acid, or, possibly, the deamidation of an asparagine residue, which results in an increase of the weight of only 1 Da. This working hypothesis was also based on literature data which indicate that isomerization variants typically elute earlier than the non-modified form in RP chromatography. Inspection of the amino acid sequence of SEQ ID NO:1 shows that the three most plausible degradation sites are N84/S85, D105/G106, and D62/S63. In each case, the N- or D-residue is followed by a glycine or serine, residues which are, in accordance with the present invention generally accepted to be the most destabilizing. Also, the D105 is located in the CDR3 region which may be assumed to be rather flexible, another condition which is known to favor β-Asp formation (Clarke, 1987; Robinson, 2002; Xie, 2003). In this context, it is of note that the isomerization of aspartate residues which are located in the CDR regions of conventional monoclonal antibodies has been reported (Cacia *et al.,* 1996; Wakankar *et al.,* 2007).

The asparagine residue at position 84 is strictly conserved in all llama/dromedary structures. Its side chain is fairly exposed to solvent. No experimental evidence for a deamidation of N84 could be found. First, the peptide mapping data show that prolonged incubation at 37°C does not result in the appearance of a molecular species that elutes earlier than the T10-peak. Secondly, the N84D mutant of SEQ ID NO:2, one of the molecular species that would form upon deamidation of N84 was found to co-elute with the wt species and did not account for any of the RPC pre- or post-peaks.

The most convincing evidence that isomerization of the aspartic acid residues at positions 62 and 105 is taking place derives from a mutational analysis of the di-peptides D105/G106 and D62/S63. Figure 17 shows that replacement of D105 by A, E or Q eliminated the occurrence of the I1 peak, which was clearly seen in the corresponding RPC profile of the wt molecule incubated for 8 weeks at 37°C. The D105N mutant on the other hand, seems to have completely converted after 8 weeks of incubation at 37°C to a mixture of isoaspartyl and normal peptide. The ratio at equilibrium of β-D105 over D105 is 70:30 or about 2.3, a figure similar to that found by others (Geiger and Clarke, 1987, J. of Biological Chemistry 262, 785-794). The chromatograms of the D105N mutant show that the other product related variants (i.e. the oxidized and pyroglutamate form) are also divided in the same relative amounts over the D105 and β-D105 form. Additionally, substitution of an alanine residue for the G106 was found to result in a much smaller I1-peak. The linkage of the I1 species to the presence of D105 and the additional finding that the I1 peak can be modulated by the nature of the amino acid immediately downstream of the aspartic acid provide strong evidence that indeed an isomerization at position 105 is responsible for the formation of the earlier eluting species I1. Similarly, it was shown that substitution of an A or E for D62 prevents the formation of the I2 peak during incubation at 37°C (Figure 18). Also, the replacement of the S63 residue by a glycine clearly promotes the formation of the I2-peak. In the latter case, the isomerization at position 62 becomes more important than that at position 105 and the chromatogram after 4 weeks storage at 37°C clearly shows signs of the presence of the β-D105/β-D62 double isomerized variant. The same is true for the aforementioned D105N mutant where β-D105 is formed in abundance. It would appear from these data that aspartic acid isomerization is taking place at both position 105 and 62; the two modifications clearly account for the newly formed peaks upon storage of SEQ ID NO:2 at 37°C. The relative peak areas of I1 and I2 indicate that the rate of D62 isomerization is considerably lower than that at position 105. In the case of SEQ ID NO:1, multiple unresolved peaks seem to form upon 37°C incubation (see Figure 16). We believe that these are adequately explained by the two above-mentioned isomerization events considering the added complexity of a bivalent molecule (i.e. additional variants can be generated such as an SEQ ID NO:1 with β-D105 in both domains).

The first evidence for β-aspartate formation at position 62 derives from the detection of a peak in the peptide map of SEQ ID NO:1 (i.e. peak T7≠ in Figure 5) which increases linearly with the time of storage at 37°C and which elutes before T7, i.e. the peptide that contains the D62 residue. The T7≠ peak represents about 6.4% of the total amount (T7+T7≠) after an 8-week incubation period at 37°C. The mass of this peak was found to correspond to the theoretical mass of the T7 fragment (1487.5 versus 1487.7 Da) and MS/MS analysis confirmed the identicalness. A mass neutral isomerization of the D62 residue provides the best explanation for these findings. The b- and y-type series ions, generated under low-energy conditions, permitted the entire sequence determination of the T7≠ peak but did not allow for the differentiation of α- and β-aspartic acids. Isomerization of D105 cannot be studied with the peptide map because the residue is part of a small 5-mer peptide which goes unnoticed in the peptide map.

The Isoquant® Isoaspartate Detection Kit was used for quantitative detection of isoaspartic acid residues in SEQ ID NO:1 and SEQ ID NO:2 stored at 37°C. The rate of β-D formation at 37°C was about 7.6% and 3.9% (mole isoaspartate : mole protein) per month for BATCH 1 and SEQ ID NO:2, respectively. The 2-fold difference is in agreement with the valency of these Nanobodies®. The level of isoaspartate detected with the Isoquant Kit in SEQ ID NO:2 is in the same range as the amount of β-D62 deduced from the peptide map (i.e. 2x3.9% versus 6.4% after 8-weeks of storage at 37°C). This result suggested that the isoaspartate at position 105, either in the intact Nanobody® or in the trypsin fragment, was perhaps not a substrate (or at the least a very poor one) for the PIMT enzyme although this enzyme has broad isoaspartate substrate specificity *in vitro.* This inference was supported by the observation that the isolated RPC peak I1 contains only about one tenth of the expected amount of isoaspartate (in hindsight, it appears that the detected level was to be attributed to a contamination of the I1 peak with I2) - the RPC main peak was isolated in parallel and scored negative in the Isoquant assay. The isolated RPC peaks were verified by RPC re-analysis and quantified spectrophotometrically. Definitive proof that β-D105 is not a substrate for the PIMT enzyme was eventually obtained by running the Isoquant assay on the synthetic peptides A-E-IsoD-G-R and its non-modified counterpart A-E-D-G-R. This peptide, corresponding to the trypsin fragment that contains the D105 residue (refer to Figure 4), was indeed not a substrate for the PIMT enzyme under the assay conditions.

The increase of the I1 peak area during storage at 37°C has been used to derive the reaction rate of D105 isomerization at this temperature. Linear least-squares regression analysis was used to fit the data set and obtain the pseudofirst-order rate constant ($k_{obs}$). The following equation was used for the fitting:

$$\ln (I1_\infty - I1 / I1_\infty) = -k_{obs}t \qquad [\text{ eq. 1}]$$

where I1 represents the relative area of peak I1 in the SEQ ID NO:2 RPC chromatograms at time t, and $I1_\infty$ is the relative peak area at infinity (t -> ∞). $I1_\infty$ was set at 70% because the isomerization is expected to yield a 70:30 ratio (as is observed for the deamidation of the D105N mutant; supra). The rate constant was found to be 0.006 days$^{-1}$ (95% confidence interval: 0.0065 - 0.0049; equivalent to t½ ≈ 115 days), a figure which is in line with the isomerization rates found in other proteins/peptides (Stephenson and Clarke, 1989, J. of Biological Chemistry 264, 6164-6170).

The experimental findings with respect to isomerization at the positions 62 and 105 are in good agreement with the structural data. In SEQ ID NO:1, D62 is highly exposed to solvent; the relative accessible surface area is 0.906. The rather low relative accessible surface area value of 0.191 calculated for D105 can be explained by the proximity in the crystal structure of the side chains of R102 and R107. In solution, however, arginine side chains are very flexible. In each of the three SEQ ID NO:1 molecules present in the asymmetric unit, the side chain of D105 interacts with the backbone amide of G106, which can provide an explanation for the significant isomerization observed at this position. In contrast, D90, a residue where no isomerization is taking place according to the peptide mapping data (data not shown), does not seem to be accessible by the solvent. Moreover, this residue accepts a hydrogen bond from R67, rather than interacting with the T91 backbone amide, which is expected to oppose isomerization.

## Isomerization of D105 is the predominant molecular mechanism underlying loss of potency

SEQ ID NO: 1 loses its binding affinity (here also referred to as potency) during storage. Several storage studies were performed. It was found that the loss is detectable at 25°C (~50% loss during 12 months), at 40°C (~40% and ~20% residual activity after 7 weeks and 5 months, respectively) but not at 5°C. In a similar study, the monovalent building block SEQ ID NO: 2 was also found to lose affinity for the vWF A1 domain (as determined by BIAcore analysis) during storage. The loss in affinity observed with SEQ ID NO:2 (about 20% during the first 8 weeks of incubation at 37°C) is roughly in accordance with the loss of potency of SEQ ID NO:1, considering that SEQ ID NO:1 will deteriorate about twice as fast because of the bivalent nature of the molecule.

The first clues that isomerization at position 105 could be responsible for the loss of bio-activity derives from the observation that the magnitude of the loss of affinity of SEQ ID NO:2 seems to correlate with the relative peak area of RPC peak I1 (see Figure 16). This view is also supported by the fact that D105 is located in CDR3, which is generally accepted to constitute the primary antigen binding region, i.e. most of the binding free energy. The following observations led us to conclude that the D105 isomerization represents the predominant inactivation mechanism (see Table B-5):

i. The isolated RPC peak I1 has 5% of the activity of the main peak; this is the same material that was used in the Isoquant assay (supra) and it should not be excluded that the residual activity is the result of contamination.

ii. The D105A mutation results in a 10-fold drop in affinity indicating that the D105 side chain provides an important contribution to the binding to the vWF A1 domain.

iii. In the D105N mutant, isomerization is interchanged for a considerably faster deamidation process. At 37°C, the asparaginyl residue quickly modifies to a mixture of the β-D and D in a ratio of ~2.5:1; RPC analyses have indicated that this modification reaction is complete after about 2 weeks (data not shown). The β-D over D ratio is in excellent agreement with the observed residual activity of about 30%.

iv. On the whole, the stability studies on the D105 muteins, except D105N, indicate that they maintain their activity during incubation at 37°C or, in any case, inactivate at a considerably slower rate than the wild type SEQ ID NO:2 molecule.

Table **B-5**: Bio-activity of the SEQ ID NO:2 muteins, containing substitutions at either the D62/S63 or the D105/G106 isomerization site. The equilibrium dissociation constants as well as the activities after 4, 8 and 16 weeks incubation at 37°C are listed. The activity during storage was followed by determining the initial binding rate to vWF A1 domain immobilized onto a sensor-chip (5 measurements); the activity was compared to that at time zero and is expressed as a percentage ($\pm$ 95% confidence interval; n=10).

| Mutants | Affinity ($K_D$; nM) | % activity after 4 wks at 37°C | % activity after 8 wks at 37°C | % activity after 16 wks at 37°C |
|---|---|---|---|---|
| SEQ ID NO:2 wild type | 1.0 | 93.5 $\pm$3.2 | 80.3 $\pm$2.9 | 60.2 $\pm$4.8 |
| SEQ ID NO:2 D62E | 1.3 | 89.6 $\pm$2.2 | 80.2 $\pm$2.1 | --- |

(continued)

| Mutants | Affinity ($K_D$; nM) | % activity after 4 wks at 37°C | % activity after 8 wks at 37°C | % activity after 16 wks at 37°C |
|---|---|---|---|---|
| SEQ ID NO:2 D62A | 1.0 | 88.4 ±2.9 | 87.4 ±1.4 | --- |
| SEQ ID NO:2 S63G | 1.2 | 95.5 ±2.6 | 83.8 ±2.4 | --- |
| SEQ ID NO:2 D105E | 4.4 | --- | 98.8 ±1.9 | 99.6 ±3.1 |
| SEQ ID NO:2 D105A | 9.3 | --- | 98.7 ±2.5 | 104.5 ±2.1 |
| SEQ ID NO:2 D105Q | 1.1 | 97.6 ±2.2 | 90.8 ±2.1 | --- |
| SEQ ID NO:2 D105N | 0.8 | 29.5 ±4.0 | 32.7 ±2.2 | --- |
| SEQ ID NO:2 D105S | 3.7 | 93.7 ±4.8 | 94.2 ±2.6 | --- |
| SEQ ID NO:2 D105T | 7.2 | 101.0 ±2.5 | 96.0 ±2.2 | --- |
| SEQ ID NO:2 G106A | no detectable affinity | --- | --- | --- |

Available data also demonstrate that isomerization at position 62 does not adversely affect the binding affinity. This follows from the observation that (i) the D105 muteins seem to maintain their activity during 37°C storage (supra), and (ii) the S63G mutation, which renders the isomerization at position 62 more important than that at position 105, does not inactivate faster than the wild type SEQ ID NO:2 during storage at 37°C. The finding that the D62A mutant has the same affinity as the wild type molecule demonstrates that this residue does not contribute to the binding and is in line with the belief that isomerization at this position is without affect on the affinity.

The mutational analyses of the D105/G106 and D62/S63 isomerization sites have shown that replacement of the glycine residue at position 106 by an alanine eliminates all activity (Table B-5). This observation is hitherto not understood. The thought that this glycine residue is characterized by phi/psi dihedral angles that are not normally observed with other amino acids is not supported by the crystal structure data; the phi/psi angles for G106 (on average -86°/- 5°) are in a range that is not abnormal for loop regions. It can however not be excluded that the G106 residue and the CDR3 region adopt a different local conformation upon binding to the vWF a1 domain.

Based on the viewpoint that isomerization at position 105 is the predominant molecular inactivation mechanism, we utilized the loss of activity during storage at 37°C to derive the reaction rate of D105 isomerization. The $k_{obs}$ values were obtained by pseudofirst-order fits to the affinity/potency data obtained from the stability studies conducted with SEQ ID NO: 2 and SEQ ID NO: 1. The following equation was used for the fitting:

$$\ln (A - A_\infty / A_0 - A_\infty) = -k_{obs}t \quad [\text{ eq. 2}]$$

where A represents the relative activity at time t, $A_0$ is the initial relative activity, and $A_\infty$ is the relative activity at infinity (t -> ∞). $A_\infty$ was set at 30% for SEQ ID NO:2 because the loss of activity as a result of D105 isomerization is expected to reach a plateau at ~30%. In the case of SEQ ID NO: 1, the loss of potency is predicted to level off at an $A_\infty$ value of ~9% (= 30% x 30%) because of the fact that bi-functionality is required for full potency. The apparent isomerization rate constants were 0.007 days$^{-1}$ (95% confidence interval: 0.0074 - 0.0062) when measured on SEQ ID NO:2 and 0.012 days$^{-1}$ (95% confidence interval: 0.0177 - 0.0065) when determined from SEQ ID NO:1. The value derived from the SEQ ID NO:2 stability data is in good agreement with the value that was derived from the increase in I1 peak area (see above). This corroborates the idea that loss of activity correlates with D105 isomerization, at least over the first months of storage at 37°C. The loss of potency of SEQ ID NO:1 occurs about twice as fast as the inactivation of its monovalent counterpart SEQ ID NO:2. This is in accord with the presence of two equivalent sites (i.e. D105 in each domain), isomerization at each of which will inactivate the Nanobody. The apparent isomerization rate as deduced from this loss of potency data is therefore also two times higher than that observed for SEQ ID NO: 2.

Conclusions

Conclusive evidence is presented that the major product related substances of SEQ ID NO: 1 are the result of:

iv.RPC pre-peak 1: a single oxidation event (+16Da), occurring in either of the two identical domains at one specific methionine (M78) out of three methionines present,
v.RPC post-peak 1: single mis-incorporation of a norleucine residue at a methionine site during bio-synthesis (-18Da),
vi.RPC post-peak 2: cyclization of the E1 residue resulting in formation of pyroglutamate, and

In addition, it was found that the potency of all three above-mentioned variants does not differ significantly from that of the authentic product. These results, in combination with the relative abundances of these product related variants (Table B-1), lead to the conclusion that the purity of SEQ ID NO:1 DS/DP is better than 99% based on activity.

Prolonged incubation of SEQ ID NO:1 at elevated temperatures (i.e. ≥25°C) leads to considerable changes in the RPC profile. The above-mentioned oxidation and pyroglutamate variants increase during storage in parallel with incubation temperature and time. In addition, the main peak splits into several different species, indicating that one or more new molecular species are being generated. We have found that splitting of the RPC main peak is to be attributed to the isomerization of aspartic acid at position 105 and, to a lesser extent, of D62. We also show that the isomerization of the aspartic acid residue at position 105, which is located in the CDR3 region, is the predominant molecular mechanism underlying the loss of potency of SEQ ID NO: 1 at elevated temperatures. The findings are summarized in Figure 21.

**Example 2: Stabilization of a RANKL binding Nanobody® construct (SEQ ID NO: 19)**

1. Generation of RANKL binding Nanobodies

Amino acid sequences:

RANKL-1 (SEQ ID NO: 19)

EVQLVESGGGLVQPGGSLRLSCAASGFTFSSYPMGWFRQAPGKGREFVSSITGSGGST
YYADSVKGRFTISRDNAKNTLYLQMNSLRPEDTAVYYCAAYIRPDTYLSRDYRKYD
YWGQGTLVTVSS

RANKL-1_D62E (SEQ ID NO: 20)

EVQLVESGGGLVQPGGSLRLSCAASGFTFSSYPMGWFRQAPGKGREFVSSITGSGGST
YYAESVKGRFTISRDNAKNTLYLQMNSLRPEDTAVYYCAAYIRPDTYLSRDYRKYD
YWGQGTLVTVSS

Based on the amino acid sequence disclosed above, the skilled person in the art is able to generate the polypeptides based on e.g. backtranslation of the polypeptide sequence, generation of overlapping oligo primers, PCR amplification, cloning into suitable expression vector, expression in suitable host, and isolation/purification of desired polypeptide, e.g. RANKL-1 above (e.g. provided by companies such as GeneArt, DNA-2-go™, sloning BioTechnology).

Analysis of the primary sequence of RANKL-1 identified D62 as a potential site for isomerisation and hence as a potential source for chemical instability of the molecule. To test this possibility, a stability assay was performed with the RANKL-1 molecule and a mutant in which the potential isomerisation site is replaced by a glutamic acid residue (E), RANKL-1_D62E. The D62E mutation in RANKL-1 was introduced by overlap PCR using primers including the mutation:

RevRANKL-1_D62E: CCTCCCTTTGACGGATTCCGCGTAATACGT (SEQ ID NO: 21) FwRANKL-1_D62E: ACGTATTACGCGGATTCCGTCAAAGGGAGG (SEQ ID NO: 22)

Both cDNAs encoding RANKL-1 or RANKL-1_D62E were cloned as SfiI /BstEII fragments in the pAX054 vector, is a derivative of pUC119. It contains the LacZ promoter which enables a controlled induction of expression using IPTG. The vector has a resistance gene for kanamycin. The multicloning site harbours several restriction sites of which *Sfi*I and *BstE*II are frequently used for cloning Nanobodies®. In frame with the Nanobody® coding sequence, the vector codes for a C-terminal c-myc tag and a (His)6 tag. The signal peptide is the gen3 leader sequence which translocates the expressed Nanobody® to the periplasm.

For production, RANKL-1 and RANKL-1_D62E constructs were inoculated in 50 ml TB/0.1% glucose/Kanamycin and the suspension incubated overnight at 37°C. 5 x 400 ml medium was inoculated with 1/100 of the obtained o/n preculture. Cultures were further incubated at 37°C, 250 rpm until OD600>5. The cultures were induced with 1 mM IPTG and further kept incubating for 4 hours at 37°C 250 rpm. The cultures were centrifuged for 20 minutes at 4500 rpm and afterward the supernatant was discarded. The pellets were stored at - 20°C. For purification, pellets were thawed and re-suspended in 20 mL dPBS and incubated for 1 hour at 4°C. Then, suspensions were centrifuged at 8500 rpm for 20 minutes to clear the cell debris from the periplasmic extract.

Nanobodies were purified via cation exchange (Source 30S column, washbuffer: 10 mM citric acid pH 4.0; Elution buffer 10 mM citric acid/1M NaCl pH 4.0) followed by size exclusion chromatography (Superdex 75 Hiload 16/60 column; in d-PBS))

The OD 280 nm is measured and the concentration calculated. Samples are stored at -20C.

## Alphascreen

Purified samples of Nanobodies RANKL-1 and RANKL-1_D62E were analyzed in Alphascreen for their ability to inhibit the interaction between human RANKL and human RANK-Fc. In this assay, various concentrations of anti-RANKL Nanobodies ranging from 1 uM to 10 pM were incubated with 3 nM biotinylated human RANK for 15 min in a 384-wells plate. Subsequently a mixture of RANKL (1 nM) and acceptor beads (20 ug/ml) coated with anti- RANKL MAb BN-12 (Diaclone) were added and incubated for 30 min. Finally, streptavidin coated donor beads (20 ug/ml) were added. After 1 hour of incubation plates were read on the Envison Alphascreen reader (PerkinElmer). All experiments were performed in duplicate. Inhibition curves and $IC_{50}$ values are shown in Figure 22. D62E mutation in RANKL-1_D62E mutant does not interfere with binding potency.

Stability study

The protein batches were diluted to 1 mg/mL in D-PBS. Afterwards the samples were filtrated through a 0.22 micrometer filter. One part of the sample was stored at -80°C as reference samples, the other part was stored at 37°C for 4 weeks (in an incubation oven) for subsequent Reverse Phase-HPLC analysis.

Reverse Phase-HPLC analysis: The column used was a ZORBAX 300SB C3 (5 $\mu$m) column on an Agilent system. The column had a temperature of 70°C during the experiments. Buffer A used during experiments was 0.1 % trifluoroacetic acid and buffer B was 0.1 % trifluoroacetic acid / 99.9% acetonitril. The program used is described below.

*Program*

| Time (min) | %B |
|:---:|:---:|
| 2 | 10 |
| 3 | 27,5 |
| 25,5 | 35 |
| 26 | 100 |
| 28,5 | 100 |
| 29 | 10 |
| 35 | 10 |

Figure 23 shows that both RANKL-1 and RANKL-1_D62E do not show peaks suggesting any isomerisation (e.g. isomarization on D62 and/or D105) whereas control Nanobody vWF12A2h1 SEQ ID NO: 2 does. Peaks reflecting formation of N-terminal cyclic pyroglutamate were present for both RANKL Nanobodies indicating that the 2 polypeptides are instable at position 1 (under given conditions).

## Example 3: Optimizing column temperature for RP-HPLC

Material - IL6R203 (SEO ID NO: 23):

EVQLVESGGGLVQPGGSLRLSCAASGFTFSDYDIGWFRQAPGKGREGVSGISSSDGNT
YYADSVKGRFTISRDNAKNTLYLQMNSLRPEDTAVYYCAAEPPDSSWYLDGSPEFFK
YWGQGTLVTVSSGGGGSGGGSEVQLVESGGGLVQPGNSLRLSCAASGFTFSSFGMS
WVRQAPGKGLEWVSSISGSGSDTLYADSVKGRFTISRDNAKTTLYLQMNSLRPEDTA
VYYCTIGGSLSRSSQGTLVTVSSGGGGSGGGSEVQLVESGGGLVQPGGSLRLSCAAS
GFTFSDYDIGWFRQAPGKGREGVSGISSSDGNTYYADSVKGRFTISRDNAKNTLYLQ
MNSLRPEDTAVYYCAAEPPDSSWYLDGSPEFFKYWGQGTLVTVSS

IL6R203 (SEQ ID NO: 23) was generated as e.g. disclosed above (by service provider GeneArt) and analyzed on the ZORBAX C 3 column under the above described chromatographic conditions but at 4 different column temperatures (75, 70, 60 and 50°C) in order to assess the effect of column temperature on the RP-HPLC profile.

HPLC conditions:

Mobile Phase A: 99.9% H2Oq / 0.1% TFA
Mobile Phase B: 0.1% TFA / 99.9% Acetonitrile
Column: ZORBAX 300SB-C3 (Agilent, Part No. 883995-909, Serial No. USKD001612) Flow rate: 1 mL/min
Gradient: 0.33 %B/min_(5%B (0 min) - 5%B (3 min) - *30.5%B* (3.5 *min*) - *40.5%B (33.5 min)* - 95%B (34 min) - 95%B (37 min) - 5%B (37.1 min) - 5%B (40 min))
Detection: UV 214 nm (280 nm was also collected)
Injection amount: 5 μg
Column temperatures used : 50°C - 60°C - 70°C - 75°C

**Table: B-6**

| Column temperature | Peak | Area (mAU*min) |
|---|---|---|
| *75°C* | *Main peak* | 5382,81 |
| | *Anterior gradient peak* | 138,07 |
| *70°C* | *Main peak* | 5173,70 |
| | *Anterior gradient peak* | 187,26 |
| *60°C* | *Main peak* | 3492,77 |
| | *Anterior gradient peak* | 858,34 |
| : Overview of integration data of analysis of IL6R203 using different column temperatures (75°C, 70°C and 60°C). Chromatograms of lower column temperatures were not integrated. As can be seen, decreasing column temperature has a negative effect on peak recovery. Integration data are shown for the main peak and the peak denoted as the anterior gradient peak. Clearly the main peak decreases at lower column temperatures. A critical point seems to be reached between 60-70°C. While the main peak area decreases, the amount of material eluting in the anterior gradient peak increases significantly. This might indicate a higher degree of stickyness of the molecule at lower column temperatures. | | |

Evaluation:

- Column temperature has a dramatic effect on elution peak characteristics. The obtained data show an optimal temperature between 70-75°C. The lower temperatures that were tested cause a loss in peak area recovery (see table B-6), resolution and induce tailing. All ready at 60°C the peak shape is completely different from that at 70-75°C.

- The 75°C chromatogram shows an increase in area/height of several side-peaks, when compared to the 70°C chromatogram. These may be temperature induced peaks and not actual subpopulations in the IL6R203 batches.

IL6R202

IL6R202 (SEQ ID NO: 24) was generated as e.g. disclosed above (e.g. by service provider GeneArt) and the same range of column temperatures were also tested on IL6R202.

IL6R202: SEQ ID NO: 24

EVQLVESGGGLVQPGGSLRLSCAASGFTFSDYDIGWFRQAPGKGREGVSGISSSDGNT
YYADSVKGRFTISRDNAKNTLYLQMNSLRPEDTAVYYCAAEPPDSSWYLDGSPEFFK
YWGQGTLVTVSSGGGGSGGGSEVQLVESGGGLVQPGNSLRLSCAASGFTFSSFGMS
WVRQAPGKGLEWVSSISGSGSDTLYADSVKGRFTISRDNAKTTLYLQMNSLRPEDTA
VYYCTIGGSLSRSSQGTLVTVSS

HPLC conditions:

Mobile Phase A: 0.1% TFA
Mobile Phase B: 0.1% TFA/99.9% Acetonitrile
Column: ZORBAX 300SB-C3 (Agilent, Part No. 883995-909, Serial No. USKD001612) Flow rate: 1 mL/min
Gradient: 0.33 %B/min (5%B (0 min) - 5%B (3 min) - *30.5%B (3.5 min) - 40.5%B (33.5 min)* - 95%B (34 min) - 95%B (37 min) - 5%B (37.1 min) - 5%B (40 min))
Detection: UV 214 nm
Injection amount: 5 μg

Column temperatures used :
50°C - 60°C - 70°C - 75°C

Table B-7:

| Column temperature | Peak | Area (mAU*min) |
|---|---|---|
| *75°C* | *Main peak* | 18155,9 |
| | *Anterior gradient peak* | 283,95 |
| *70°C* | *Main peak* | 18265,30 |
| | *Anterior gradient peak* | 395,94 |

(continued)

| Column temperature | Peak | Area (mAU*min) |
|---|---|---|
| 60°C | Main peak | 16568,80 |
| | Anterior gradient peak | 1174,07 |
| [i]: Overview of integration data of analysis of IL6R202 using different column temperatures (75°C, 70°C and 60°C). Chromatograms of lower column temperatures are not shown. As can be seen with IL6R203, a critical point is reached between 60-70°C. At the lower temperature end of this border a considerable loss in peak recovery seems to occur. At the same time the area of the anterior gradient peak rises considerably. | | |

Evaluation:

- Column temperature has a dramatic effect on the elution peak profile of IL6R202 (as for IL6R203). Optimal temperature lies between 70-75°C.
- Practically it would be better to use the column temperature of 70°C to minimize the occurrence of artifact peaks caused by IL6R202 degradations at such high column temperature

**Example 4: Stabilization of 12A2h1 and ALX-0081 taking into consideration findings from mutant studies above**

Based on our insights in the chemical stability of SEQ ID NO: 1 and its monovalent building block SEQ ID NO: 2, we engineered a variant of SEQ ID NO: 2 which incorporates four single amino acid substitutions, namely E1D, D62E, M78T and D105Q (SEQ ID NO: 26). Thus, the stabilized monovalent building block is referred to as stabilized 12A2h1 or SEQ ID NO: 26 and the stabilized vWF compound of example 1 is referred to as VWF0001 or SEQ ID NO: 25 herein.

SEQ ID NO: 25 - Amino Acid Sequence of ALX-0081 SV1, also referred to herein as VWF0001

```
DVQLVESGGG LVQPGGSLRL SCAASGRTFS YNPMGWFRQA PGKGRELVAA ISRTGGSTYY
PESVEGRFTI SRDNAKRTVY LQMNSLRAED TAVYYCAAAG VRAEQGRVRT LPSEYTFWGQ
GTQVTVSSAA AEVQLVESGG GLVQPGGSLR LSCAASGRTF SYNPMGWFRQ APGKGRELVA
AISRTGGSTY YPESVEGRFT ISRDNAKRTV YLQMNSLRAE DTAVYYCAAA GVRAEQGRVR
TLPSEYTFWG QGTQVTVSS
```

SEQ ID NO: 26 - Amino Acid Sequence of 12A2H1 SV1

```
DVQLVESGGG LVQPGGSLRL SCAASGRTFS YNPMGWFRQA PGKGRELVAA ISRTGGSTYY
PESVEGRFTI SRDNAKRTVY LQMNSLRAED TAVYYCAAAG VRAEQGRVRT LPSEYTFWGQ
GTQVTVSS
```

The construction of the 12A2h1SV1 and ALX-0081SV1 genes was done by gene assembly. A set of overlapping oligonucleotides covering the genes of interest was ordered for assembling the gene by PCR. The rescue PCR products were purified from gel and digested with KpnI and NdeI restriction enzymes. The fragments were then ligated in pet28a/TAC/pelB vector previously cut with KpnI / NdeI enzymes.

The ligation mixture was then transformed in TOP 10 electro competent cells. After addition of SOC medium and 1hr incubation at 37°C an aliquot was plated out onto LB /Kanamycin plates and incubated at 37°C overnight. The following day individual clones were analyzed by colony PCR using the pet28a promoter and the T7 terminator primers. PCR-positive clones for 12A2SV1 and ALX-0081SV1 were subsequently grown overnight in LB / Kanamycin. From the overnight cultures, plasmid DNA was prepared by Mini-Prep (Sigma Aldrich kit) for DNA sequencing.

<u>Expression and purification of 12A2h1SV and ALX0081SV1</u>

Overnight starter cultures were prepared and were used to start the expression on larger scale in Terrific Broth medium containing Kanamycin and 0.1% g/v glucose. A flask containing 300mL of the above medium was inoculated with 10 mL of starter culture and afterwards grown at 37°C while shaking at 250 rpm. After for approximately 4 hours the temperature was lowered to 28°C. After another 3 hours induction was started by IPTG to a final concentration of 1mM and the culture was further incubated overnight at 28°C. The following day the cultures were centrifuged for 30 minutes at 4500 rpm.The pellets were shortly frozen and after thawing PBS / 1 mM EDTA was added. After having resuspended the cells, the suspension was shaken for 2 hours at room temperature. The suspensions were centrifuged at 8500 rpm for 20 minutes to clear the cell debris from the extract. The supernatant was afterwards acidified to pH 3.5 and stored overnight at 4°C. The next morning the suspension was centrifuged at 8000 rpm and the supernatant was filtrated. After filtration the solution was diluted with water to conductivity below 5 mS/cm. The solution was afterwards loaded on a Source S ion-exchange column preequilibrated with buffer A (10 mM citric acid pH 3.5). After washing with buffer A, bound material was eluted with a 10 column volumes gradient of 0-100% gradient of buffer B (10 Mm citric acid / 1 M NaCl pH 3.5). Fractions containing the proteins of interest were identified by SDS-PAGE and pooled. The pooled fractions were afterwards processed by gel filtration chromatography on Superdex75 column. The protein concentration of fractions containing the purified constructs was afterwards determined spectrophotometrically at 280nm thereby using the calculated extinction coefficient and molecular weight. The purity of the proteins was confirmed by HPLC-RPC.

<u>Binding functionality of the stable variants 12A2h1SV1 and ALX-0081SV1.</u>

The binding potency of ALX-0081SV1 was determined on a Biacore 300 instrument with the parallel line method and thereby using ALX-0081 as reference material. The method is designed in such a way that bifunctionality is observed. In this assay we observed as expected avid binding for the ALX-0081 SV1 and no potency for 12A2h1SV1. The relative potency in comparison with ALX0081 (SEQ ID NO 1) was around 80%.

The affinity of 12A2h1SV1 for the A1 domain of VWF was also determined with the Biacore instrument. The kinetic rate constants $(k_{on}$ and $k_{off})$ as well as the equilibrium dissociation constant $(K_D)$ were determined (see Table below).

|  | SEQ ID NO: 2 | SEQ ID NO: 26 |
|---|---|---|
| $k_{on}$ **(1/Ms)** | 1.93 E+07 | 2.1 E+07 |
| $k_{off}$ **(1/s)** | 0.0207 | 0.0207 |
| $K_D$ **(nM)** | 1.07 | 0.986 |

<u>Chemical stability of the stable variants</u>

Both 12A2h1 SV1 and ALX-0081SV1 were incubated at 37°C at a concentration of 1 mg/mL in D-PBS. At different time points, samples were analyzed by RP/HPLC. It appeared that only minor amounts of variants were formed after 8 weeks as shown in Figure 39 and Figure 40. In contrast with the profiles presented in Figure 16 for SEQ ID NO:1 and SEQ ID NO:2, the RP/HPLC profiles are less complex and lack the major pre- and post-peaks , which have shown to result from oxidation, puroglutamte formation and aspartate isomerization. Thus, by replacing the critical residues by appropriate well-chosen amino acid residues, we were able to produce a variant with essentially the same bio-activity and a dramatically improved chemical stability (see also Example 6 for in vivo activity measured by Folts model and RIPA).

We also determined the functionality of the samples which were stored at 37°C Biacore. In these experiments both reference and stability samples were 10,000 fold diluted and subsequently passed over a sensor chip coated with 3500RU vWF A1 domain. As only minor differences in binding sensorgrams were observed between the reference and stabilized samples, we conclude that the chemical stability is improved while the binding functionality is about the same.

Similar results as described above are also expected for the mutant binders according to SEQ ID NO: 39 (bivalent) and SEQ ID NO: 40 (monovalent building block), respectively.

**Example 5: Stabilization of an another Nanobody construct, i.e. an IL6R binding Nanobody® construct (SEQ ID NO: 27)**

DNA sequences:

**IL6R201 (SEQ ID NO: 27; wildtype: to be checked for stability and potentially** stabilized):

GAGGTACAGCTGGTGGAGTCTGGCGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
CGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGGATGGCTCT
CCTGAATTCTTTAAATATTGGGGTCAGGGTACGCTGGTCACCGTCTCCTCATAATG
A

**Mutants of IL6R201:**

**IL6R201_D55E (SEQ ID NO: 28):**

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
AGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGGATGGCTCT
CCTGAATTCTTTAAATATTGGGGTCAGGGTACGCTGGTCACCGTCTCCTCATAATG
A

**IL6R201_D55Q (SEQ ID NO: 29):**

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTCA
AGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGGATGGCTCT
CCTGAATTCTTTAAATATTGGGGTCAGGGTACGCTGGTCACCGTCTCCTCATAATG
A

IL6R201_D62E (SEQ ID NO: 30):

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
CGGCAACACTTATTACGCAGAAAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGGATGGCTCT
CCTGAATTCTTTAAATATTGGGGTCAGGGTACGCTGGTCACCGTCTCCTCATAATG
A

IL6R201_D102E (SEQ ID NO: 31):

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
CGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGAAAGCTCGTGGTATCTGGATGGCTC
TCCTGAATTCTTTAAATATTGGGGTCAGGGTACGCTGGTCACCGTCTCCTCATAAT
GA

IL6R201_D102Q (SEQ ID NO: 32):

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
CGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCACAAAGCTCGTGGTATCTGGATGGCTC
TCCTGAATTCTTTAAATATTGGGGTCAGGGTACGCTGGTCACCGTCTCCTCATAAT
GA

IL6R201_D108E (SEQ ID NO: 33):

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA

CGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGGAAGGCTC
TCCTGAATTCTTTAAATATTGGGGTCAGGGTACGCTGGTCACCGTCTCCTCATAAT
GA

IL6R201_D108Q (SEQ ID NO: 34):

GAGGTGCAGCTGGTGGAGTCTGGGGGAGGTCTGGTTCAACCGGGCGGGAGCTTG
CGTCTGAGTTGCGCTGCGAGCGGTTTCACATTTAGCGACTACGACATCGGATGGT
TTCGTCAGGCTCCGGGCAAAGGTCGCGAAGGTGTGTCTGGCATTTCAAGTTCTGA
CGGCAACACTTATTACGCAGACAGCGTTAAAGGTCGTTTCACCATTTCGCGTGAT
AACGCAAAGAATACCCTGTACCTTCAAATGAATAGCTTACGCCCAGAAGATACCG
CCGTTTACTATTGTGCCGCGGAACCGCCAGATAGCTCGTGGTATCTGCAAGGCTC
TCCTGAATTCTTTAAATATTGGGGTCAGGGTACGCTGGTCACCGTCTCCTCATAAT
GA

The nanobodies were cloned in a pet28a vector which uses Tac promoter and PelB leader sequence.

**Expression and purification**

Start a pre-culture of **the** IL6R201 constructs D55E *(clone 2),* D55Q *(clone 4),* D102E *(clone* 3), D102Q *(clone 1)* D108E *(clone 2)* and *D108Q (clone 2)* (pet28a/TAC/pelB vector) in 50 ml LB/Kanamycin and incubate overnight at 37°C. The expression is started in 3 x ± 330 mL TB I+II / Kanamycin. Each flask in inoculated with 10 mL of starter culture and afterwards grown at 37°C while shaking at 250 rpm for approximately 4 hours afterwards the temperature is lowered to 28°C. Culture is induced 6 hrs later with 1 mM IPTG (330 uL of a 1M stock) and further kept incubating overnight at 28°C 250 rpm. The cultures were centrifuged for 30 minutes at 4500 rpm and afterward the supernatant was discarded. The pellets were stored at - 20°C for 2-3 hours and afterward the pellets were re-suspended in 30 mL PBS / 1 mM EDTA and shaken for 2 hours at room temperature. The suspensions were centrifuged at 8500 rpm for 20 minutes to clear the cell debris from the extract.

The column used for the purification is a MabCaptureA (Poros). Buffer A in this experiment was D-PBS / 0,5M NaCl and buffer B was 100 Mm Glycin pH 2,5. The ±30L of periplasmic extract was pumped onto the column at a flow rate of 10 mL/min. Afterwards the column was washed with buffer A for multiple column volumes. To elute the bound protein we switched to buffer B.

Next, Size exclusion chromatography was used. A Superdex 75HR 26/60 was used for the size exclusion. The gelfiltration was done in D-PBS.

The OD 280 nm is measured and the concentration calculated. Samples are stored at -20C.

## Stability study

The original protein batches were diluted to 500 or 1 mg/mL (a total volume of 5 mL) in D-PBS. Afterwards the samples were filtrated through a 0.22 micormeter filter and 500 microliter was stored at -80°C as reference samples and approximately 4500 micorliter was stored at 37°C (in an incubation oven).

## Analysis in Biacore3000

The binding properties of IL-6R201, IL-6R201 D55E, IL-6R201 D55Q, IL-6R201 D102E, IL-6R201 D102Q, IL-6R201 D108E and IL-6R201 D108Q stored for 4 weeks at 37°C is investigated by using the initial binding rate (IBR) and slope.

It is shown that the calibration curve is fully linear in the range of 0-50 ng/mL for IL-6R201 and his mutants on a high density hIL-6R chip. Therefore a range of concentrations from 0 to 50 ng/ml will be included in this experiment (to control linearity) and functionality will be determined by injecting each sample 5 times at a concentration of 40 ng/ml.

First, a high density hIL-6R chip is preconditioned by injecting IL-6R201 5 times. Next, a range of concentrations from 0-50 ng/mL of IL-6R201 is injected followed by injecting all samples 5 times.

Evaluation is done using BIAevaluation software. Slopes are determined in the BIAevaluation software using the 'General fit' method and the linear fit model. Data to be used to determine the initial binding rate (IBR) are the slopes chosen between 5 and 25s.

## Reverse Phase-HPLC

The column used was a ZORBAX 300SB C3 (5 $\mu$m) column on an Agilent system. The column had a temperature of 70°C during the experiments. Buffer A used during experiments was 0.1 % trifluoroacetic acid and buffer B was 0.1% trifluoroacetic acid / 99.9% acetonitril. The program used is described below:

*Program*

| Time (min) | %B |
|---|---|
| 2 | 10 |
| 3 | 27,5 |
| 25,5 | 35 |
| 26 | 100 |
| 28,5 | 100 |
| 29 | 10 |
| 35 | 10 |

Evaluation: Mutagenesis has been performed to remove potential isomerization sites (2 sites in CDR2 and 2 sites in CDR3). Stability studies were performed of the different mutants (storage for 4 wks at 37C). Analysis in RPC identified D108E and D108Q as a crucial mutation to preserve activity and decrease isomerization (compare Figure 27 to figure 33: only replacement of D108 to D108E or D108Q resulted in prevention of isomerization).

## Example 6: In vivo efficacy of ALX-0081 (SEQ ID NO: 1) and VWF0001 (SEQ ID NO: 25)

Folts' model

A modified Folts' model in baboons was used to determine efficacy in preventing acute thrombosis. The Folts' model is in detail described in Example 18 of WO2004/062551. Nine healthy male baboons (*Papio ursinus),* weighing between 9.6 - 17 kg, were caught in the wild and used in this study. The baboons were fed with dry standard food only.

All procedures were approved by the 'Ethics committee for Animal Experimentation of the University of the Free State and Free State Provincial Administration' in accordance with the 'National Code for Animal Use in Research, Education, Diagnosis and Testing of Drug and Related Substanced in South Africa'.

Briefly, a shunt was placed under general anesthesia between the femoral artery and femoral vein. The shunt was used for drug administration and blood sampling as well as for monitoring the blood flow with a perivascular ultrasonic flow probe that was placed around the shunt (Transonic systems TS410, rpobe: ME3PXL10O8). The femoral artery was then injured with a forceps and a clamp was placed over the injured site which was used to produce an external stenosis of the femoral artery. As a result, high shear rates were obtained and the blood flow was reduced to 20% of the original flow rate. A platelet rich thrombus was formed and subsequently dislodged mechanically, resulting in cyclic flow reductions (CFRs). One CFR is the time between stenosis and complete occlusion of the artery (zero flow). After a 30-minute control period of reproducible CFRs, the shunt was flushed and vehicle (saline) was administered as an internal control. CFRs were followed for 30 more minutes. Subsequently, increasing doses of the Nanobodies® were injected intravenously into the shunt and the effect on CFRs was studied. A new injury was applied after inhibition of CFRs in order to confirm that the inhibition was an effect of the treatment but not of a natural healing phenomenon. After complete inhibition of CFRs after administration of the highest dose of Nanobody®, Epinephrine was injected to further test the potency of the Nanobodies®. Epinephrine activates the platelets again and can distinguish between a weak and a strong inhibition of CFRs. Indeed, it has been demonstrated before that CFRs reappear in the presence of Epinephrine when Aspirin® was used in the same model.

Ten minutes after each dosing, blood samples (6.5 ml) were taken for laboratory analysis. RIPA, FVIII:C and plasma concentrations of Nanobodies® and VWF:Ag were analyzed as described elsewhere, e.g. example 18 of WO2004/062551.

Two different bleeding analyses were performed: the skin bleeding time (using a Surgicut device; measured 10 minutes after each dose of Nanobodies®) and a second surgical bleeding test in which a gauze was inserted in an incision in the groin and the total blood loss was measured by weighing the gauzes. The gauzes were replaced every 30 minutes just before each new dose of Nanobody®. The amount of blood loss for each dose was determined by weighing the gauzes. This was expressed relative to the amount of blood loss in the control gauze (during the saline injection).

RIPA

The RIPA is a biomarker for ALX-0081 and measures the rate and degree to which dispersed platelets in a sample of platelet rich plasma (PRP) forms aggregates after non-physiological activation of the A1 domain by the antibiotic ristocetin (see again e.g. example 18 of WO2004/062551). The RIPA occurs in two steps. In the first step, platelets agglutinate with VWF in the presence of ristocetin, in the second step platelets aggregate due to the release of endogenous ADP from the platelets. The clumping of the platelets causes the PRP to become less turbid. The change in turbidity is measured in a platelet aggregometer.

The RIPA was analyzed at the University of the Free State, Bloemfontein, South Africa. Platelet aggregations were performed on a Chronolog whole blood and optical Aggregometer (Model 560CA, Chronolog, USA). PRP was prepared by centrifuging the whole blood (collected on 0.32% citrate) at 1200 rpm for 5 minutes. The upper fraction containing the PRP was carefully removed. The lower fraction was further centrifuged at 3000 rpm for 10 minutes to prepare platelet poor plasma (PPP). Platelets were counted in PRP and diluted in autologous PPP to a final concentration of 200.000 platelets per $\mu$l. Aggregation was induced by addition of 3 mg/ml ristocetin (DAKO) and was measured with the aggregometer.

Efficacy of ALX-0081 and VWF0001

The first two baboons were injected with increasing doses of ALX-0081 and VWF0001 (ALX-0081_Sv or stable variant of ALX-0081), respectively.

Blood flow measurements during these Folts' experiments are shown in Figure 35.

The mean lengths of CFRs after each dose of test compound are summarized in Table B-8. The table also indicates the total dose, which is the cumulative dose of all administrations at that time point. Doses at which full inhibition of CFRs was obtained are marked in with (>1800).

Table B-8: Length of CFRs (seconds) for animals treated with ALX-0081 and VWF0001. >1800 = complete inhibition for 30 minutes, no decrease of blood flow

| | Drugs → | | ALX-0081 | VWF0001 |
|---|---|---|---|---|
| | Baboon ID → | | B1 | B2 |
| Total dose ↓ | Dose ↓ | | | |
| 0 μg/kg | Ctrl | | 92 | 84 |
| 0 μg/kg | Saline | | 142 | 97 |
| 3 μg/kg | 3 μg/kg | | 139 | 125 |
| 13 μg/kg | 10 μg/kg | | 189 | 559 |
| 43 μg/kg | 30 μg/kg | | >1800 | >1800 |
| 133 μg/kg | 90 μg/kg | | >1800 | >1800 |
| 403 μg/kg | 270 μg/kg | | >1800 | >1800 |

Full inhibition of CFRs was obtained at a dose of 30 μg/kg (cumulative dose of 43 μg/kg) for ALX-0081 and 10 μg/kg (cumulative dose of 13 μg/kg) for VWF0001.

Inhibition was retained upon a new injury but was unexpectedly lost after infusion of Epinephrine. The latter can be explained by the fact that the plasma level of ALX-0081 was probably too low at Epinephrine injection as the experiment continues for too long (in the past we administered ALX-0081 via a bolus + continuous infusion, whereas now only a bolus was administered).

The RIPA was measured in blood samples taken 10 minutes after saline injection and after each dose of ALX-0081 or VWF0001. For both baboons, results from the RIPA test were compared to the length of CFRs (Figure 36).

An inverse relationship between the RIPA and the length of the CFRs was observed. For both baboons, the RIPA results compare very well with the efficacy results in the Folts' model. Therefore, the RIPA might be considered as an efficacy biomarker for vWF binders such as e.g. ALX-0081/VWF0001.

**Example 7: Forced oxidation experiments**

**Example** 7.1 Forced oxidation experiments with IL6R201

We previously demonstrated that the methionine present at position 78 in SEQ ID NO: 2 (vWF-12A2hI) was susceptible to oxidation. In this example we describe that we have introduced a mutation in the VHH fragment IL6R201 (SEQ ID NO: 35). In this mutant, threonine at position 78 (threonine a residue which is frequently present at this position in FR3 in VH or VHH fragments) was changed into a methionine and named IL6R201 T78M (SEQ ID NO: 36). After purification of the mutant protein, a forced oxidation experiment with $H_2O_2$ was performed with both IL6R201 andIL6R201 T78M. By subsequent analysis on RP/HPLC, we could demonstrate that the mutated VHH became vulnerable to oxidation (Figure 37 - upper trace) whereas the non-mutated was resistant to treatment with $H_2O_2$ (Figure 37 - lower trace). Amino Acid Sequence of IL6R201 (SEQ ID NO: 35):

EVQLVESGGG LVQPGGSLRL SCAASGFTFS DYDIGWFRQA PGKGREGVSG

ISSSDGNTYY ADSVKGRFTI SRDNAKNTLY LQMNSLRPED TAVYYCAAEP

PDSSWYLDGS PEFFKYWGQG TLVTVSS

Amino Acid Sequence of IL6R201-T78M (SEQ ID NO: 36):

EVQLVESGGG LVQPGGSLRL SCAASGFTFS DYDIGWFRQA PGKGREGVSG ISSSDGNTYY ADSVKGRFTI SRDNAKNMLY LQMNSLRPED TAVYYCAAEP PDSSWYLDGS PEFFKYWGQG TLVTVSS

Example 7.2 Forced oxidation experiment of 119A3 with H$_2$O$_2$

Amino Acid Sequence of 119A3 (SEQ ID NO: 37)

EVQLVESGGG LVQAGGSLRL SCAASGRIFS LPASGNIFNL LTIAWHRQAP GMQRELVATI NSGSRTNYAD SVKGRFTISR DNAQKTVYLQ MNNLKPEDTA VYYCQTSGSG SPNFWGQGTQ VTVSS

119A3 (SEQ ID NO: 37) contains two methionine residues, respectively a methionine at position 52 in framework 2 and the second methionine at position 91 in FR3. Based on earlier findings with vWF-12A2h1 (SEQ ID NO: 2), we postulate that in Nanobody 119A3, only M52 is solvent accessible and thus prone to oxidation by H$_2$O$_2$. Based on our findings with vWF-12A2h1 we postulate that under native conditions only M52 is susceptible to forced oxidations, whereas under unfolding conditions both residues should be prone to oxidation. After purification, a solution of 119A3 at 1mg/mL in D-PBS was incubated for 2 hours with 10mM H$_2$O$_2$. After removal of the excess of H$_2$O$_2$ on a desalting column the mixture was analyzed by RP-HPLC. As shown in Figure 38, it appeared that the treated material (lower trace in Figure 38) was modified, as a peak which eluted earlier from the column in comparison with the non-treated material (upper trace) was present (shift of peak). We also observed that in the non-treated samples a small peak corresponding to the oxidized material was already present.

The material was also treated with H$_2$O$_2$ in the presence of 6 M guanidine. Analysis of this material on RP-HPLC uncovered also peak which eluted earlier than the non-treated sample (middle trace in Figure 38). The difference of retention times of the oxidized variants formed with native and unfolded sample, provides evidence that under native conditions only M52 is oxidized whereas in the unfolded state both residues became susceptible to oxidation.

**Claims**

1.   A method for making a polypeptide sequence comprising a single variable domain with improved chemical stability, wherein said method comprises the step of

   a. generating a library of mutated polypeptide sequences from a parent polypeptide, wherein said parent polypeptide comprises at least one single variable domain, functional derivative or fragment thereof, and wherein said parent polypeptide has a DS, DG, NG or NS motif in a CDR region, or with a DS, DG, NG or NS motif in a surface exposed region with H-donor groups close to the labile N or D, and wherein the amino acid sequence of said mutated polypeptide sequences are changed in the following way:

      i. replacing N-terminal E (E1) or N-terminal Q (Q1) with D; and
      ii. replacing at least one M that is prone to oxidation with A or T; and
      iii. replacing D or N of said DS, DG, NG or NS motif with Q or E;

   b. and screening said generated library for polypeptide sequences with a high affinity or avidity;
   c. and optionally selecting one or several mutated polypeptides with said high affinity or avidity.

2.   Method according to claim 1, wherein said DS, DG, NG or NS motif is in a CDR region.

3.   Method according to claim 1, wherein said DS, DG, NG or NS motif is in a CDR2 or CDR3 region.

4.   Method according to claim 1, wherein said DS, DG, NG or NS motif is in a CDR3 region.

5.   Method according to any one of claims 1 to 4 , wherein said high affinity or avidity is expressed as the dissociation constant to its target molecule and said dissociation constant is equal or below 100 nM.

**6.** Method according to claim 5, wherein said dissociation constant is equal or below 10 nM.

**7.** Method according to claim 6, wherein said dissociation constant is equal or below 1 nM.

**8.** Method according to any of claims 1 to 7, wherein said polypeptide consists essentially of a variable domain present in naturally occuring heavy chain anti-bodies (VHH) or a construct thereof.

**9.** A polypeptide selected from the group consisting of (SEQ ID NO: 25 and SEQ ID NO: 26.

**10.** A nucleotide sequence selected from the group consisting of SEQ ID NO: 33 and SEQ ID NO: 34.


**Patentansprüche**

**1.** Verfahren zur Herstellung einer Polypeptidsequenz, welche eine einzelne variable Domäne mit verbesserter chemischer Stabilität umfasst, wobei das Verfahren die folgenden Schritte umfasst:

a. Generieren einer Bibliothek aus mutierten Polypeptidsequenzen von einem Ausgangspolypeptid, wobei das Ausgangspolypeptid mindestens eine einzelne variable Domäne, ein funktionales Derivat oder Fragment hiervon umfasst, und wobei das Ausgangspolypeptid ein DS-, DG-, NG- oder NS-Motiv in einer CDR-Region hat oder ein DS-, DG-, NG- oder NS-Motiv in einer oberflächenexponierten Region mit H-Donorgruppen nahe dem labilen N oder D, und wobei die Aminosäuresequenz der mutierten Polypeptidsequenzen in folgender Weise verändert werden:

i. Ersetzen von N-terminalem E (E1) oder N-terminalem Q (Q1) mit D; und
ii. Ersetzen von mindestens einem M, das zur Oxidation neigt, mit A oder T; und
iii. Ersetzen von D oder N des DS-, DG-, NG- oder NS-Motivs mit Q oder E;

b. und Screenen der generierten Bibliothek nach Polypeptidsequenzen mit einer hohen Affinität oder Avidität;
c. und, optional, Auswählen einer oder mehrerer mutierter Polypeptide mit solch einer hohen Affinität oder Avidität.

**2.** Verfahren gemäß Anspruch 1, wobei das DS-, DG-, NG- oder NS-Motiv in einer CDR-Region ist.

**3.** Verfahren gemäß Anspruch 1, wobei das DS-, DG-, NG- oder NS-Motiv in einer CDR2- oder CDR3-Region ist.

**4.** Verfahren gemäß Anspruch 1, wobei das DS-, DG-, NG- oder NS-Motiv in einer CDR3-Region ist.

**5.** Verfahren gemäß einem der Ansprüche 1 bis 4, wobei die hohe Affinität oder Avidität als Dissoziationskonstante zu dem Zielmolekül ausgedrückt wird und die Dissoziationskonstante gleich oder weniger als 100 nM ist.

**6.** Verfahren gemäß Anspruch 5, wobei die Dissoziationskonstante gleich oder weniger als 10 nM ist.

**7.** Verfahren gemäß Anspruch 6, wobei die Dissoziationskonstante gleich oder weniger als 1 nM ist.

**8.** Verfahren gemäß einem der Ansprüche 1 bis 7, wobei das Polypeptid im Wesentlichen aus einer variablen Domäne vorhanden in natürlich vorkommenden Schwere-Ketten-Antikörpern (VHH) oder einem Konstrukt hiervon besteht.

**9.** Polypeptid ausgewählt aus der Gruppe, bestehend aus SEQ ID NO: 25 und SEQ ID NO: 26.

**10.** Nukleotidsequenz ausgewählt aus der Gruppe, bestehend aus SEQ ID NO: 33 und SEQ ID NO: 34.


**Revendications**

**1.** Procédé pour la réalisation d'une séquence polypeptidique comprenant un domaine variable unique à stabilité chimique améliorée, dans lequel ledit procédé comprend l'étape de :

a. générer une banque de séquences polypeptidiques mutées à partir d'un polypeptide parent, où ledit polypeptide parent comprend au moins un domaine variable unique, un dérivé fonctionnel ou un fragment de celui-ci, et où ledit polypeptide parent présente un motif DS, DG, NG ou NS dans une région CDR, ou avec un motif DS, DG, NG ou NS dans une région exposée à la surface avec des groupes donneurs de H à proximité du N ou du D labiles, et où la séquence d'acides aminés desdites séquences polypeptidiques mutées sont modifiées de la manière suivants :

i. remplacer un E N-terminal (E1) ou un Q N-terminal (QI) par un D ; et
ii. remplacer au moins un M qui est sujet à une oxydation par un A ou T ; et
iii. remplacer un D ou N dudit motif DS, DG, NG ou NS avec un Q ou E ;

b. et cribler ladite banque générée pour des séquences polypeptidiques ayant une haute affinité ou avidité ;
c. et éventuellement choisir un ou plusieurs polypeptide(s) muté(s) ayant ladite haute affinité ou avidité.

2. Procédé selon la revendication 1, dans lequel ledit motif DS, DG, NG ou NS se trouve dans une région CDR.

3. Procédé selon la revendication 1, dans lequel ledit motif DS, DG, NG ou NS se trouve dans une région CDR2 ou une région CDR3.

4. Procédé selon la revendication 1, dans lequel ledit motif DS, DG, NG ou NS se trouve dans une région CDR3.

5. Procédé selon l'une quelconque des revendications 1 à 4, dans lequel ladite haute affinité ou avidité est exprimée comme étant la constante de dissociation envers sa molécule cible et ladite constante de dissociation est inférieure ou égale à 100 nM.

6. Procédé selon la revendication 5, dans lequel ladite constante de dissociation est inférieure ou égale à 10 nM.

7. Procédé selon la revendication 6, dans lequel ladite constante de dissociation est inférieure ou égale à 1 nM.

8. Procédé selon l'une des revendications 1 à 7, dans lequel ledit polypeptide consiste essentiellement en un domaine variable présent dans des anticorps à chaîne lourde (VHH) d'origine naturelle ou une construction de celui-ci.

9. Polypeptide choisi dans le groupe constitué de la séquence SEQ ID No : 25 et de la séquence SEQ ID NO : 26.

10. Séquence nucléotidique choisie dans le groupe constitué de la séquence SEQ ID NO : 33 et de la séquence SEQ ID NO : 34.

# Figure 1

# Figure 2

# Figure 3

mAU

Split of the RPC main peak as a result of prolonged incubation at 37°C (0, 4, 8w)

# Figure 4

```
           10          20          30          40
EVQLVESGGG LVQPGGSLRL SCAASGRTFS YNPMGWFRQA

           50          60          70          80
PGKGRELVAA ISRTGGSTYY PDSVEGRFTI SRDNAKRMVY

           90         100         110         120
LQMNSLRAED TAVYYCAAAG VRAEDGRVRT LPSEYTFWGQ

          128
GTQVTVSS
```

# Figure 5

# Figure 6

# Figure 7

# Figure 8

Figure 9

Figure 10

# Figure 11

# Figure 12

# Figure 13

# Figure 14

# Figure 15

# Figure 16

## Figure 17

# Figure 18

# Figure 19

## Figure 20

## Figure 21

| pyroglutamate formation at N-terminus | norleucine incorporation at methionine sites |

EVQLVESGGG LVQPGGSLRL SCAASGRTFS YNPMGWFRQA PGKGRELVAA ISRTGGSTYY PDSVEGRFTI

SRDNAKRMVY LQMNSLRAED TAVYCAAAG VRAEDGRVRT LPSEYTFWGQ GTQVTVSS

| M78 oxidation | D105 isomerization (= molecular mechanism of inactivation) | D62 isomerization |

## Figure 22

## Figure 23

# Figure 24

# Figure 25

DAD1 C. S19=214,8 Ref=360, 100 (2007\IL6-ALB\2007-04- 16 11-00-36 ILBR203 COLUMN TEMP\RP03X0132.D)

75°C

DAD1 C. S19=214,8 Ref=360, 100 (2007\IL6-ALB\2007-04- 16 11-00-36 ILBR203 COLUMN TEMP\RP03X0133.D)

70°C

EP 2 238 156 B1

# Figure 26

# Figure 27

## Figure 28

IL6R201 D55E 0 wks at 37°C
IL6R201 D55E 4 wks at 37°C

## Figure 29

IL6R201 D55Q 0 wks at 37°C
IL6R201 D55Q 4 wks at 37°C

# Figure 30

IL6R201 D102E 0 wks at 37°C
IL6R201 D102E 4 wks at 37°C

# Figure 31

IL6R201 D102Q 0 wks at 37°C
IL6R201 D102Q 4 wks at 37°C

## Figure 32

## Figure 33

# Figure 34

Figure 35

Figure 35

# Figure 36

# Figure 37

# Figure 38

# Figure 39

# Figure 40

# Figure 41

## Figure 42 A

## Figure 42 B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9115581 A
- WO 2009004065 A
- WO 2009004066 A
- EP 0364926 A
- EP 0368684 A
- WO 04068820 A
- WO 06030220 A
- WO 06003388 A
- WO 0518629 A
- WO 9404678 A
- WO 9504079 A
- WO 9634103 A
- WO 9425591 A
- WO 9937681 A
- WO 0040968 A
- WO 0043507 A
- WO 0065057 A
- WO 0140310 A
- WO 0144301 A
- EP 1134231 A
- WO 0248193 A
- WO 9749805 A
- WO 0121817 A
- WO 03035694 A
- WO 03054016 A
- WO 03055527 A
- WO 03050531 A
- WO 0190190 A
- WO 03025020 A
- EP 1433793 A
- WO 04041867 A
- WO 04041862 A
- WO 04041865 A
- WO 04041863 A
- WO 04062551 A
- WO 05044858 A
- WO 0640153 A
- WO 06079372 A
- WO 06122786 A
- WO 06122787 A
- WO 06122825 A, Ablynx N.V.
- WO 06040153 A
- WO 07104529 A
- WO 04003019 A
- WO 9507463 A
- WO 9623810 A
- WO 9521191 A
- WO 9711094 A
- WO 9742320 A
- WO 9806737 A
- WO 9821355 A
- US 7207410 A
- US 5693492 A
- EP 1085089 A
- WO 9429457 A
- WO 9942077 A
- WO 9429469 A
- WO 9700957 A
- US 5580859 A
- US 55895466 B
- WO 9402610 A
- WO 9522618 A
- US 7004940 A
- WO 03014960 A
- US 6741957 A
- US 6304489 A
- US 6849992 A
- US 5399346 A
- US 4608392 A, Jacquet
- US 4992478 A, Geria
- US 4559157 A, Smith
- US 4820508 A, Wortzman
- US 4938949 A
- WO 2006122825 A
- WO 2004062551 A

**Non-patent literature cited in the description**

- **A A WAKANKAR et al.** *Biochemistry,* 2007, vol. 46, 1534-1544
- **SAYERS, J. R. et al.** *Nucleic Acids Res.,* 1988, vol. 16, 791-802
- **STEFFAN, N. H. et al.** *Gene,* 1989, vol. 77, 51-59
- **SINHA et al.** *NAR,* 1984, vol. 12, 4539-4557
- PCR protocols. Academic Press, 1990
- **BETTER et al.** *J. Biol. Chem.,* 1992, vol. 267, 16712-16118
- Current protocols in Molecular Biology. John Wiley and Sons, 1989
- **ROBINSON et al.** *Hum. Antibod. Hybridomas,* 1991, vol. 2, 84-93

- Antibodies; A Laboratory Manual. Cold Spring Harbor Laboratory, 1988
- **MUNSON et al.** *Anal. Biochem.,* 1980, vol. 407, 220-239
- **WARD et al.** *Nature,* 12 October 1989, vol. 341 (6242), 544-6
- **HOLT et al.** *Trends Biotechnol.,* 2003, vol. 21 (11), 484-490
- **MUYLDERMANS.** *Reviews in Molecular Biotechnology,* 2001, vol. 74, 277-302
- **LODISH et al.** Molecular Cell Biology. 113
- **SAMBROOK et al.** Molecular Cloning: A Laboratory Manual. Cold Spring Harbor Laboratory Press, 1989, vol. 1-3
- Current protocols in molecular biology. Green Publishing and Wiley Interscience, 1987
- **LEWIN.** Genes II. John Wiley & Sons, 1985
- **OLD et al.** Principles of Gene Manipulation: An Introduction to Genetic Engineering. University of California Press, 1981
- **ROITT et al.** Immunology. Mosby/Elsevier, 2001
- **ROITT et al.** Roitt's Essential Immunology. Blackwell Publishing, 2001
- **JANEWAY et al.** Immunobiology. Garland Science Publishing, 2005
- **PRESTA.** *Adv. Drug Deliv. Rev.,* 2006, vol. 58 (5-6), 640-56
- **LEVIN ; WEISS.** *Mol. Biosyst.,* 2006, vol. 2 (1), 49-57
- **IRVING et al.** *J. Immunol. Methods,* 2001, vol. 248 (1-2), 31-45
- **SCHMITZ et al.** *Placenta,* 2000, vol. 21 (A), 106-12
- **GONZALES et al.** *Tumour Biol.,* 2005, vol. 26 (1), 31-43
- **OBER et al.** *Intern. Immunology,* 2001, vol. 13, 1551-1559
- **FRIGUET et al.** *J. Immunol. Methods,* 1985, vol. 77, 305-19
- **KENNETH, A et al.** Chemical Stability of Pharmaceuticals: A Handbook for Pharmacists
- **PETERS et al.** *Pharmacokinete analysis: A Practical Approach,* 1996
- **M GIBALDI ; D PERRON.** Pharmacokinetics. Marcel Dekker, 1982
- **KABAT et al.** Sequence of proteins of immunological interest. US Public Health Services, NIH
- **RIECHMANN ; MUYLDERMANS.** *J. Immunol. Methods,* 23 June 2000, vol. 240 (1-2), 185-195
- **CHOTHIA et al.** *Nature,* 1989, vol. 342, 877-883
- **CULVER, K. W.** Gene Therapy. Mary Ann Liebert, Inc., Publishers, 1994, xii
- **GIORDANO.** *Nature F Medicine,* 1996, vol. 2, 534-539
- **SCHAPER.** *Circ. Res.,* 1996, vol. 79, 911-919
- **ANDERSON.** *Science,* 1992, vol. 256, 808-813
- **VERMA.** *Nature,* 1994, vol. 389, 239
- **ISNER.** *Lancet,* 1996, vol. 348, 370-374
- **MUHLHAUSER.** *Circ. Res.,* 1995, vol. 77, 1077-1086
- **ONODERA.** *Blood,* 1998, vol. 91, 30-36
- **VERMA.** *Gene Ther.,* 1998, vol. 5, 692-699
- **NABEL.** *Ann. N.Y. Acad. Sci.,* 1997, vol. 811, 289-292
- **VERZELETTI.** *Hum. Gene Ther.,* 1998, vol. 9, 2243-51
- **WANG.** *Nature Medicine,* 1996, vol. 2, 714-716
- **SCHAPER.** *Current Opinion in Biotechnology,* 1996, vol. 7, 635-640
- **AFANASIEVA et al.** *Gene Ther.,* 2003, vol. 10, 1850-1859
- **BLANCO et al.** *J. Immunol,* 2003, vol. 171, 1070-1077
- **CATTANEO, A. ; BIOCCA, S.** Intracellular Antibodies: Development and Applications. Landes and Springer-Verlag, 1997
- **KONTERMANN.** Methods. 2004, vol. 34, 163-170
- Remington's Pharmaceutical Sciences. Mack Publishing Company, 1990
- **REMINGTON.** the Science and Practice of Pharmacy. Lippincott Williams and Wilkins, 2005
- **A.A. WAKANKAR et al.** *Biochemistry,* 2007, vol. 46, 1534-1544
- **CIRINO et al.** *Biotechnol. Bioeng.,* 2003, vol. 83, 729-734
- **GADGIL et al.** *J. of the American Society of Mass Spectrometry,* 2006, vol. 17, 867-872
- **GEIGER ; CLARKE.** *J. of Biological Chemistry,* 1987, vol. 262, 785-794
- **STEPHENSON ; CLARKE.** *J. of Biological Chemistry,* 1989, vol. 264, 6164-6170